# EUROPEAN PATENT APPLICATION

(11) **EP 3 693 465 A1**
(43) Date of publication of application: **12.08.2020**
(21) Application number: 19155627.3
(22) Date of filing: 05.02.2019
(51) Int. Cl.: C12N 15/50, C12N 7/00, C12Q 1/68, C07K 14/165, C07K 16/08, A61K 39/215, C12N 15/113

(54) **NOVEL FISH CORONAVIRUS**

(71) Applicant: Pharmaq AS, 7863 Overhalla (NO)
(72) Inventor: Nylund, Stian, 5152 Strusshamn (NO); Sandlund, Liv, 5172 Loddefjord (NO); Økland, Arnfinn L., 5306 Erdal (NO)
(74) Representative: Dörries, Hans Ulrich

(57) **Abstract**

The invention relates to a novel fish virus, indicated to be a Coronavirus, which causes mortality in fish, and to methods of detecting said virus in fish, and related uses.

## Description

### Field of the invention

The invention *inter alia* relates to a novel fish virus, indicated to be a Coronavirus, which causes mortality in fish, and to methods of detecting said virus in fish and protecting fish from infection by said virus, and related (re)agents and uses.

### Background of the Invention

Fish are a major source of food and fish farming has become an important industry, particularly as rates of wild fish capture are flat or declining due to overfishing and loss of habitat. Examples of fish that are farmed include Atlantic salmon (Salmo salar) and lumpsucker (Cyclopterus lumpus).

However, infectious diseases in aquaculture threaten fish production and may also impact wild fish populations. For example, heart and skeletal muscle inflammation (HSMI) is known to be a frequently fatal disease of farmed Atlantic salmon. Affected fish often show reduced appetite prior to abnormal swimming behaviour and in some cases, sudden death. Usually, no external lesions are recognized. At autopsy, the heart often appears pale and somewhat loose. In some cases, the pericardial sac is filled with blood. Histological examinations indicate that most fish in affected net cages show severe lesions, although they seemingly appear to be healthy. First recognized in Norway in 1999 (Kongtorp et al., J Fish Dis 27, 2004), HSMI was subsequently implicated in several outbreaks in other farms in Norway and the United Kingdom. It is believed that piscine reovirus (PRV), a fish reovirus belongs to the family Reoviridae, subfamily Spinareovirinae, is the likely causative agent of HSMI (Kibenge et al., Virol J. 10, 2013). Since 1999 there has been an increasing number of outbreaks and the disease is considered to have a detrimental economic impact on the salmon farming industry.

Cardiomyopathy syndrome (CMS) is a severe cardiac disease affecting primarily large Atlantic salmon in the second year in seawater close to harvest. Affected fish may suddenly die without showing outward signs of disease, or may show symptoms such as abnormal swimming behaviour and anorexia. The disease was first recognized in farmed Atlantic salmon in Norway in 1985 and subsequently in farmed salmon in the Faroe Islands, the United Kingdom and Ireland. CMS has also been described in wild Atlantic salmon in Norway. In 2010, a double-stranded RNA virus of the Totiviridae family, named piscine myocarditis virus (PMCV), was described as the causative agent of CMS (Haugland et al, J. Virol, 85, 2011). PMCV is considered one of the largest problems in Atlantic salmon production, leading to major financial losses for salmon producing companies.

Disease challenges in the production of lumpfish *(Cyclopterus lumpus)* have to a certain degree been dominated by bacterial infections. Among these, *Aeromonas salmonicida subsp.* (atypical furunculosis), *Pasteurella sp., Vibrio anguillarum* and *Tenacibaculum sp.* have been the most significant species. The application of targeted vaccination programs, systematic monitoring of disease and improvements in production has led to a gradual decrease of the number of cases of atypical furuncolosis, vibriosis and pasteurellosis. However, several species of viruses have been detected in wild lumpfish, including viral hemorrhagic septicemia (VHSV) (Guðmundsdóttir et al, J. Fish Dis, 42, 2019), viral nervous necrosis (VNN) and a novel ranavirus. Recently, a virus affecting farmed lumpfish has been identified: lumpfish flavivirus (LFV/CLuV) (Skoge et al, Arch Virol, 163, 2018). LFV/CLuV shows low but distinct similarity to the unassigned Tamana bat virus (TABV). LFV/CLuV was found to be present in all kinds of lumpfish tissues of affected fish, but pathology was primarily observed in the liver and kidneys. The virus is associated with serious disease in lumpfish. After LFV/CLuV was characterized, mapping the distribution of the virus and its association with disease has shown that it is widespread, with a relatively high associated prevalence.

Certain fish farms currently experience high mortality - for example up to 80% in some lumpsucker populations - despite real-time RT-PCR and histology failing to find any known pathogens in the fish.

Thus, there is an ongoing need to identify further pathogens which infect and which kill fish, particularly farmed lumpsucker fish. Further, there is an ongoing need for methods of monitoring the production of farmed fish for the presence of infection by pathogens, to avoid outbreaks of infection and potentially to treat infected fish.

### Summary of the Invention

The present invention has surprisingly found a novel virus in lumpsucker fish, herein termed Cyclopterus lumpus Coronavirus (CLuCV). The length and organization of the genome, together with sequence analyses, indicate that CLuCV is a Torovirus, in the Coronaviridae family, with the Berne virus as its closest relative. Viruses in the Torovirus genus typically affect the intestine, and can lead to severe diarrhea in affected animals. The virus disclosed herein is the first Torovirus identified in fish.

Accordingly, one aspect of the invention provides a nucleic acid comprising at least one open reading frame (ORF) sequence selected from the group consisting of ORF-1, ORF-2, ORF-3, ORF-4 and ORF-5; wherein
ORF-1 is at least 80% identical to the nucleic acid sequence of SEQ ID NO:1,
ORF-2 is at least 80% identical to the nucleic acid sequence of SEQ ID NO:2,
ORF-3 is at least 80% identical to the nucleic acid sequence of SEQ ID NO:3,
ORF-4 is at least 80% identical to the nucleic acid sequence of SEQ ID NO:4, and
ORF-5 is at least 80% identical to the nucleic acid sequence of SEQ ID NO:5.

Another aspect of the invention provides a virus that infects and is capable of killing lumpsucker fish *(Cyclopterus lumpus),* wherein the virus genome comprises a nucleic acid sequence disclosed herein, wherein said nucleic acid sequence contains the base uracil (U) instead of the base thymine (T).

Another aspect of the invention provides an oligonucleotide primer which comprises a sequence of at least 9 nucleotides, wherein said sequence is complementary to a nucleic acid sequence which is comprised within the genome of the virus disclosed herein.

Another aspect of the invention provides a method for detecting a virus that infects and is capable of killing fish, comprising the steps of:
(a) contacting a nucleic acid extracted from a biological sample of a fish with at least one oligonucleotide primer to form a mixture, wherein the at least one oligonucleotide primer is complementary to a nucleic acid sequence which is comprised within the genome of the virus disclosed herein, and
(b) determining whether upon subjecting the mixture of a) to amplification an amplification product is present, wherein the presence of amplification product indicates the presence of RNA associated with the virus, and hence the presence of the virus in the biological sample.

Another aspect of the invention provides a method for detecting a virus that infects and is capable of killing fish, comprising the steps of:
(a) sequencing a nucleic acid extracted from a biological sample of a fish, and
(b) comparing the resulting nucleic acid sequence with a nucleic acid sequence which is, or which is complementary to, a reference sequence selected from the group consisting of SEQ ID NO:1, SEQ ID NO:2, SEQ ID NO:3, SEQ ID NO:4, SEQ ID NO:5 and SEQ ID NO:6, wherein an at least 80% sequence identity between the two sequences indicates the presence of the virus in the biological sample.

Another aspect of the invention provides an antibody that binds a polypeptide, wherein the polypeptide is encoded by a nucleic acid sequence which is comprised within the genome of the virus disclosed herein.

Another aspect of the invention provides a kit for detecting a virus in a biological sample from fish, wherein the kit comprises an oligonucleotide primer disclosed herein and/or an antibody disclosed herein.

Another aspect of the invention provides an antibody for use in treating fish infected with a virus disclosed herein.

Another aspect of the invention provides a use of the virus disclosed herein for producing a vaccine.

Another aspect of the invention provides a vaccine comprising:
(i) a nucleic acid of a sequence which is comprised within the genome of the virus disclosed herein;
(ii) a polypeptide encoded by a nucleic acid sequence which is comprised within the genome of the virus disclosed herein; or
(ii) a virus disclosed herein.

Yet another aspect of the invention provides an interfering RNA (iRNA) molecule for use in treating fish infected with a virus, wherein the iRNA molecule comprises at least 12 nucleotides of, or complimentary to, a nucleic acid sequence comprised within the genome of the virus disclosed herein.

### Brief Description of the Figures

Figure 1: A schematic of the sequence of CLuCV identified herein, which is 24,613 nucleotides long and contains five possible open reading frames (ORFs).
Figure 2: CLuCV genome nucleotide sequence.
Figure 3: CLuCV ORF-1 nucleotide sequence.
Figure 4: CLuCV ORF-2 nucleotide sequence.
Figure 5: CLuCV ORF-3, ORF-4 and ORF-5 nucleotide sequences.

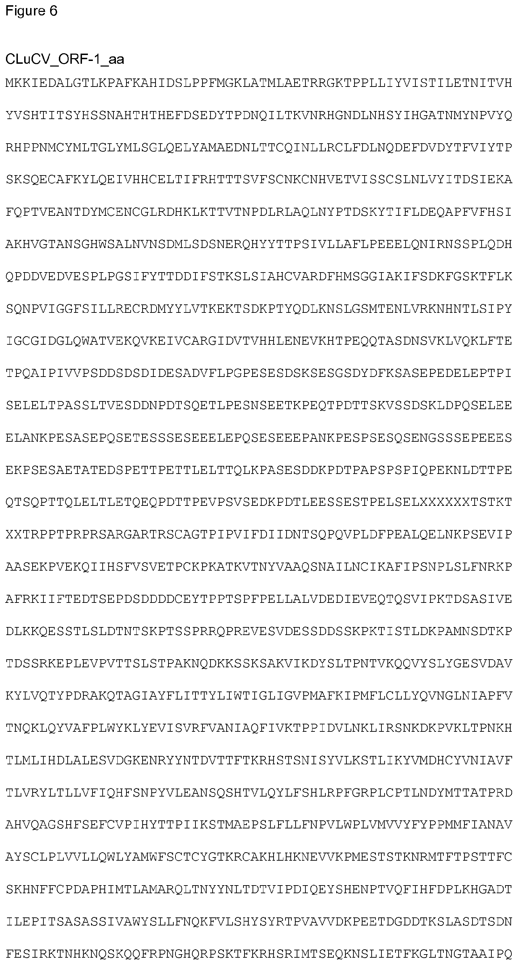

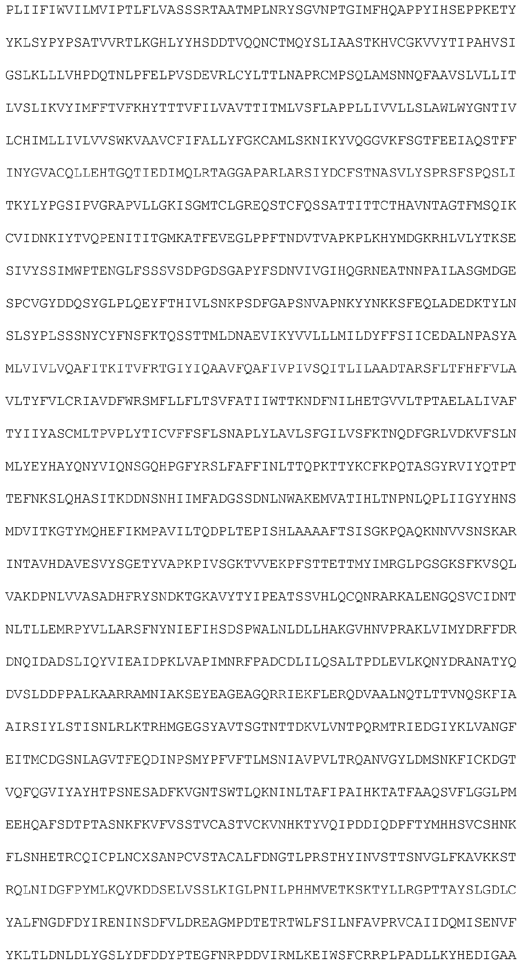

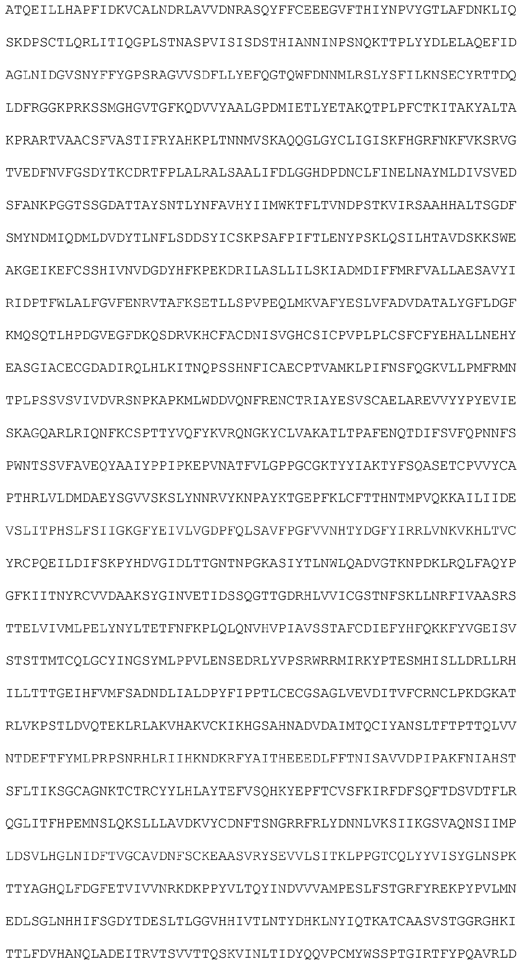

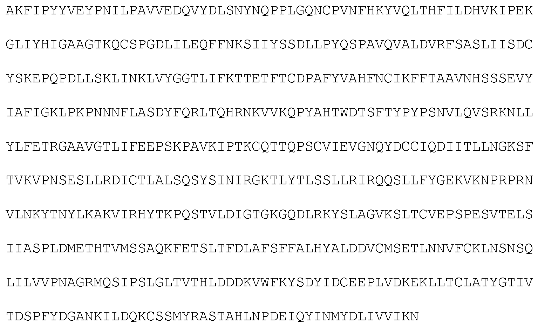

Figure 6: CLuCV ORF-1 amino acid sequence.

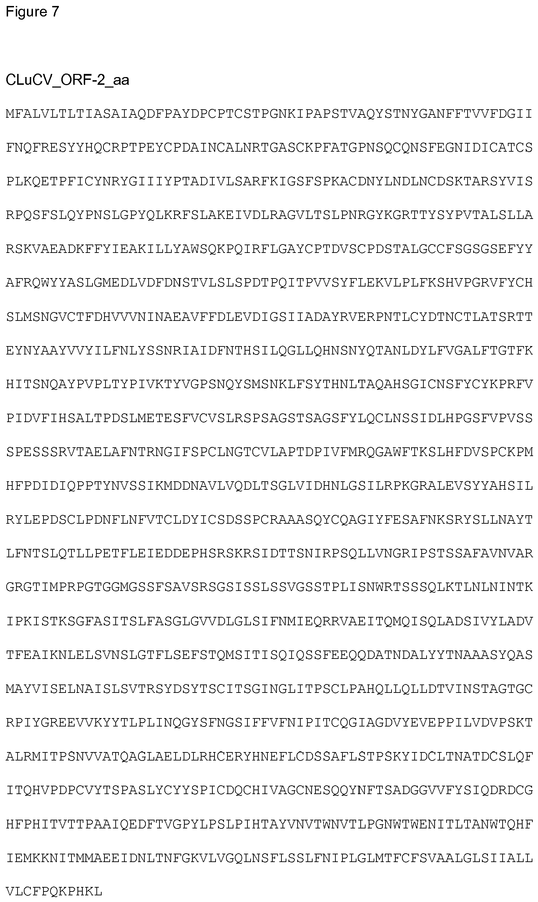

Figure 7: CLuCV ORF-2 amino acid sequence.

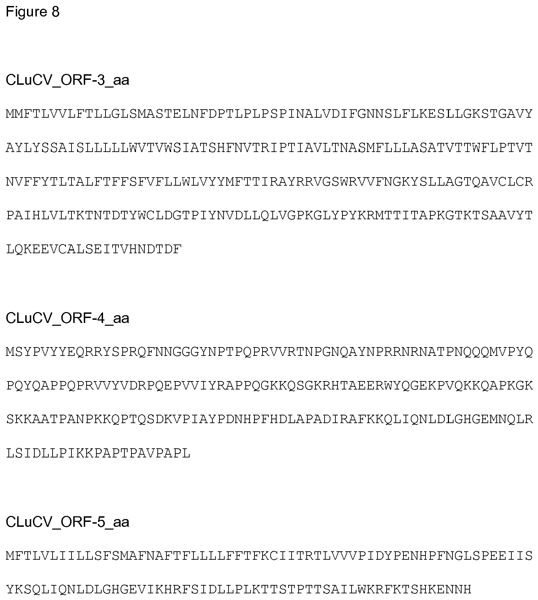

Figure 8: CLuCV ORF-3, ORF-4 and ORF-5 amino acid sequences.
Figure 9: Haematoxylin and Eosin stain of a whole section of affected lumpsucker, showing accumulation of fluid in the stomach (arrow).
Figure 10: Haematoxylin and Eosin stain of a section of lumpsucker intestine showing accumulation of mucus, and cellular discharge (arrows).
Figure 11: Haematoxylin and Eosin stain of a section of lumpsucker intestine showing accumulation of mucus (arrow), and cellular discharge.
Figure 12: Haematoxylin and Eosin stain of a section of lumpsucker intestine showing accumulation of mucus (arrow).

### Detailed Description of the Invention

### Definitions

In order for the present invention to be readily understood, several definitions of terms used in the course of the invention are set forth below.

As used herein, the term "lumpsucker" or "lumpfish" is intended to mean any species selected from the whole family of Cyclopteridae. The most preferred species according to the invention is Cyclopterus lumpus.

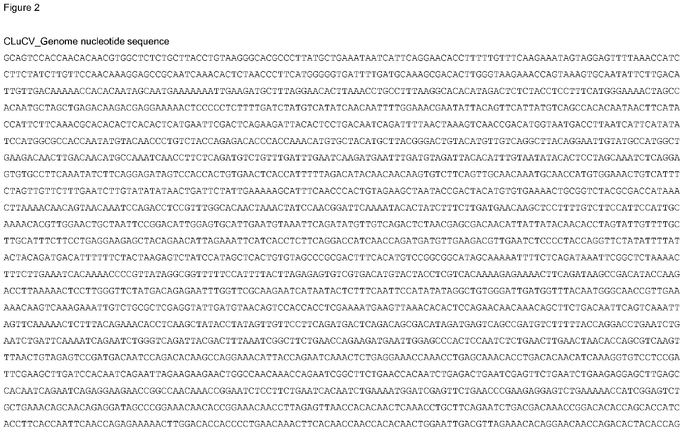

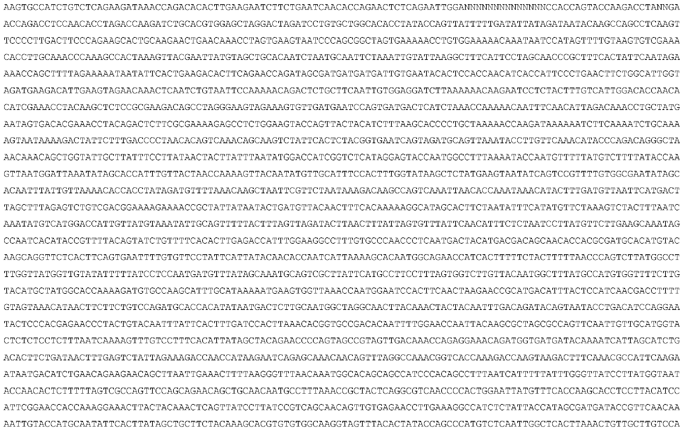

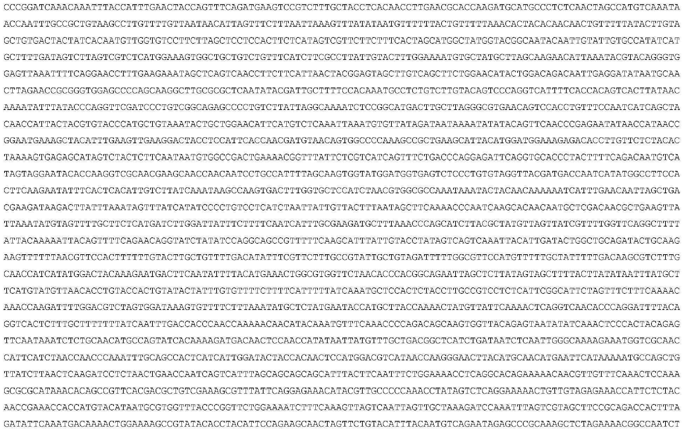

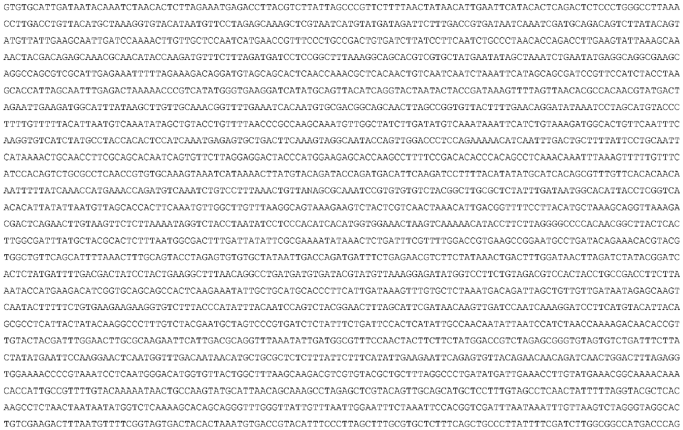

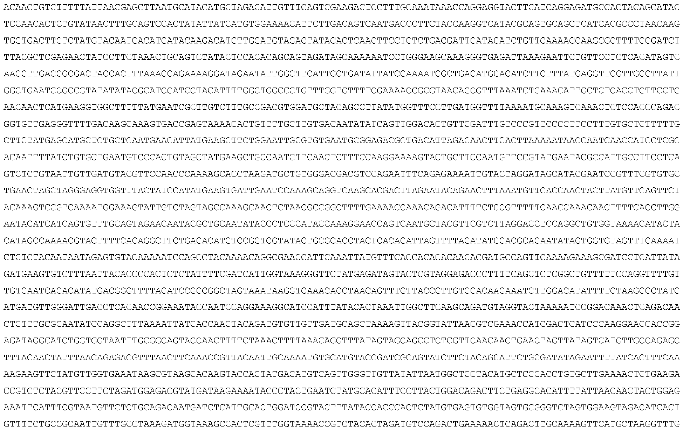

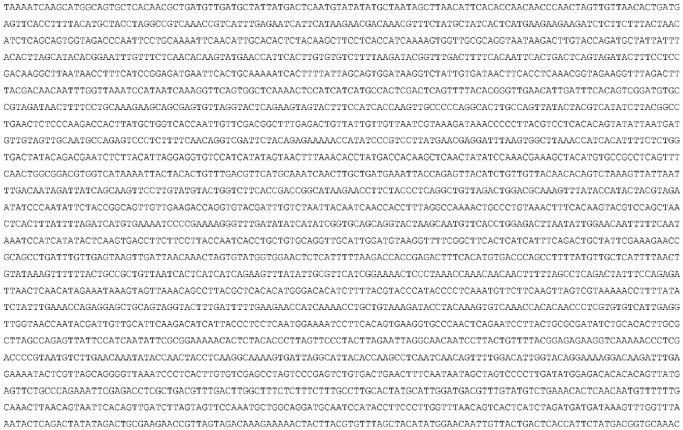

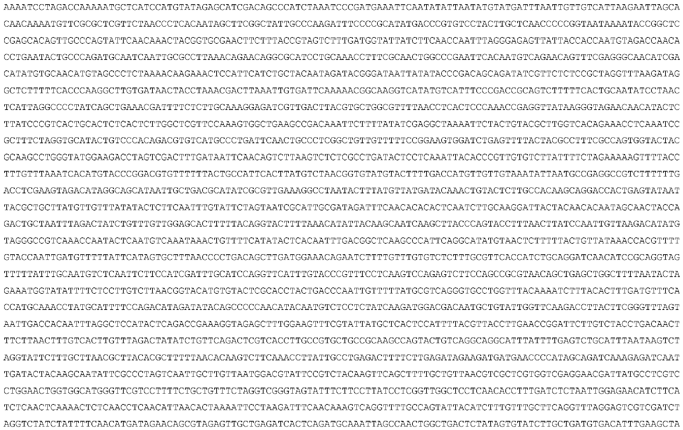

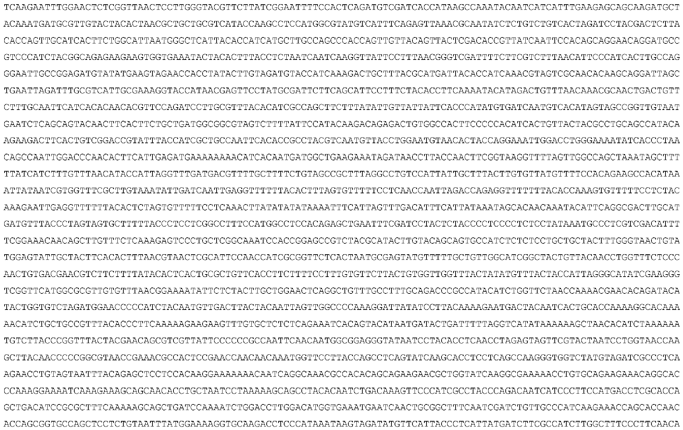

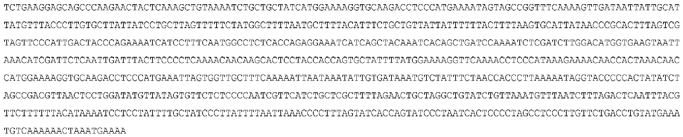

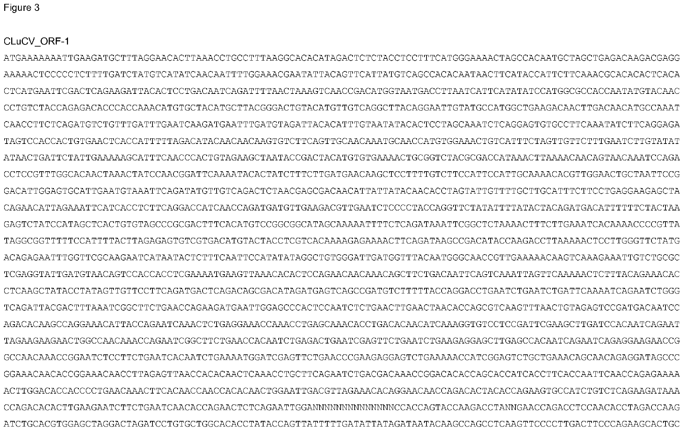

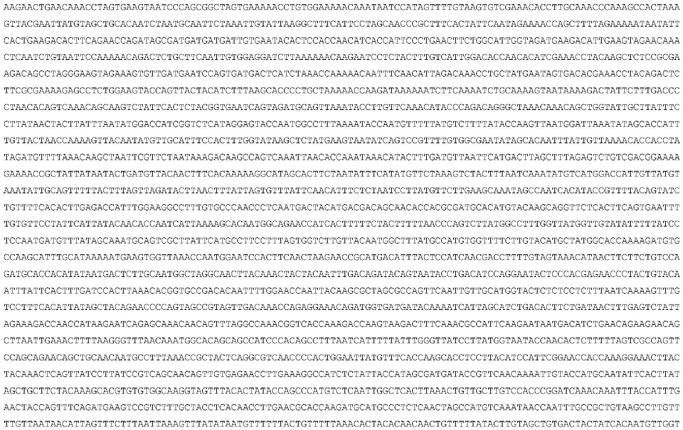

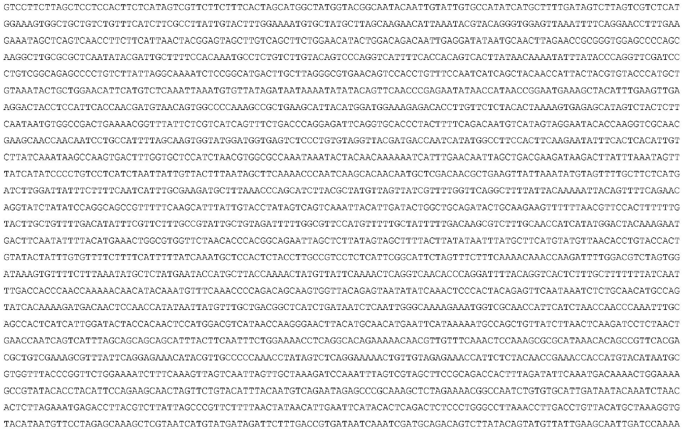

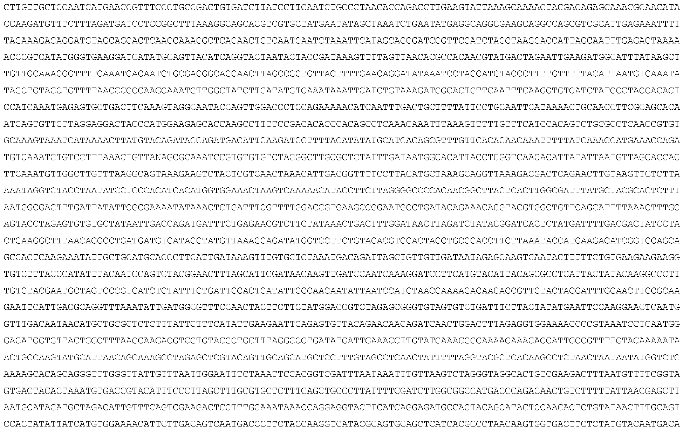

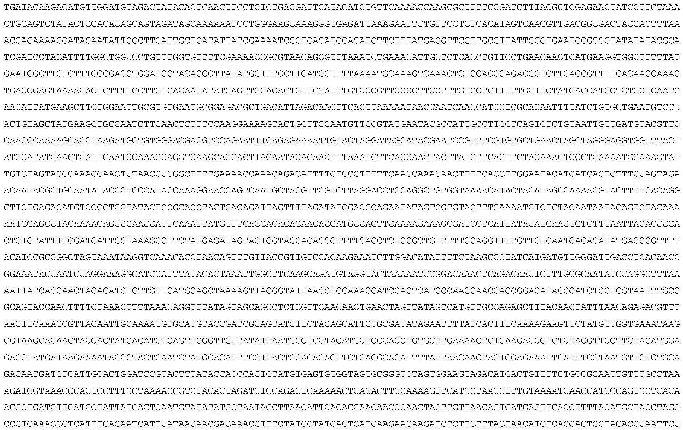

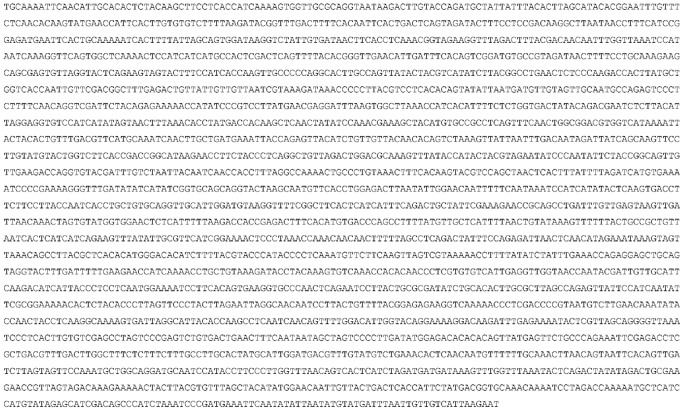

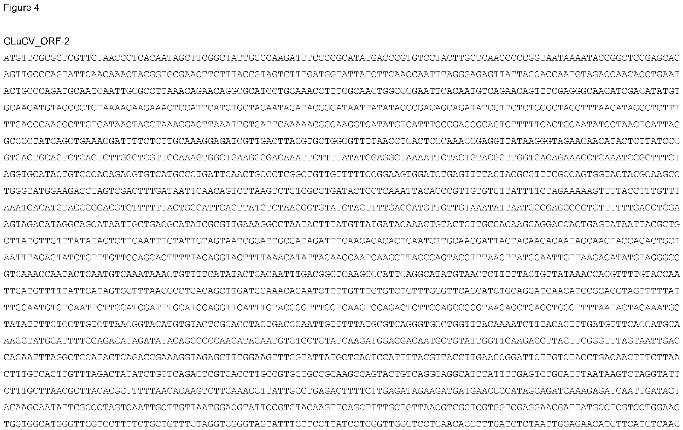

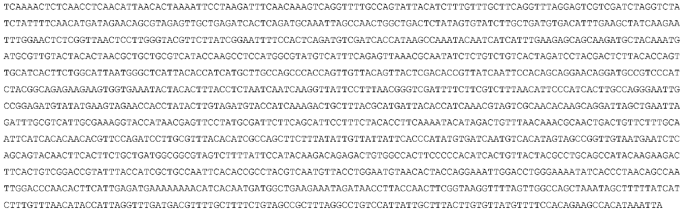

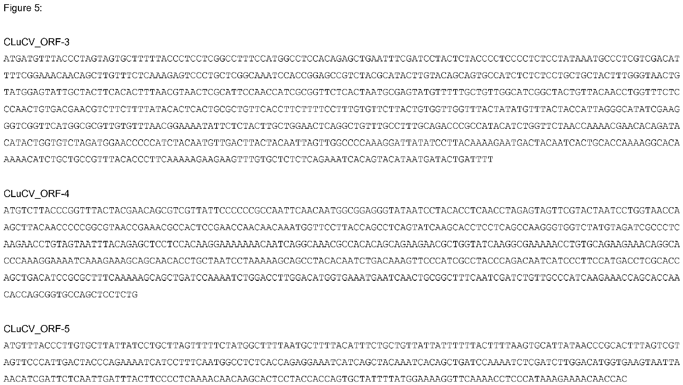

The term "nucleic acid" includes DNA molecules (e.g., cDNA or genomic DNA), RNA molecules (e.g., mRNA), analogues of DNA or RNA generated using nucleotide analogues (e.g., peptide nucleic acids and non-naturally occurring nucleotide analogues), and hybrids thereof. Thus, whereas the nucleic acid sequences provided in Figures 2 to 5 and SEQ ID NOs: 1 to 6 use the bases guanine, cytosine, adenine and thymine, embodiments of the invention relate to their corresponding RNA sequences which use the bases guanine, cytosine, adenine and uracil (i.e., with uracil instead of thymine), which RNA sequences are therefore also provided herein. The nucleic acid molecule can be single-stranded or double-stranded. Unless specified otherwise, the left-hand end of any single-stranded nucleic acid sequence discussed herein is the 5' end. The direction of 5' to 3' addition of nascent RNA transcripts is the transcription direction.

The term "oligonucleotide" means a nucleic acid comprising 200 or fewer nucleotides. Oligonucleotides can be single stranded, e.g., for use as primers, cloning primers or hybridization probes, or they can be double stranded, e.g., for use in the construction of a mutant gene. Oligonucleotides can be sense or antisense oligonucleotides. An oligonucleotide can include a label, including a radiolabel, a fluorescent label, a hapten or an antigenic label, for detection assays.

As used herein, the term "oligonucleotide primer" or "primer" is to be understood to refer to a nucleic acid (e.g., of at least 9 nucleotides in length, and less than 60 nucleotides in length) suitable for directing an activity to a region of a nucleic acid, e.g. for amplification of a target nucleic acid sequence by the polymerase chain reaction (PCR), or for in situ hybridization.

As used herein, the term "complementary" in the context of nucleic acid sequences means nucleic acid sequences that form a double-stranded structure by matching base pairs (A to T (or U); and G to C). For example, the complementary nucleic acid sequence to G-T-A-C is C-A-T-G. Other examples of complementary nucleic acid sequences are the following:
Complementary nucleic acid sequence (e.g., in case the nucleic acid is DNA):
5'-ATTCGCTTAACGCAA-3'
3'-TAAGCGAATTGCGTT-5'

The corresponding complementary sequences wherein uracil is substituted for thymine (e.g., in case the nucleic acid is RNA):
5'-AUUCGCUUAACGCAA-3'
3'-UAAGCGAAUUGCGUU-5'

As used herein, the term "amino acid" refers to one of the 20 naturally occurring amino acids or any non-natural analogues. Preferably, the term "amino acid" refers to one of the 20 naturally occurring amino acids.

The terms "polypeptide" or "protein" mean a macromolecule composed of a sequence of amino acids. A protein can be a native protein, that is, a protein produced by a naturally-occurring and non-recombinant cell; or it can be produced by a genetically-engineered or recombinant cell, and comprise molecules having the amino acid sequence of the native protein, or molecules having deletions from, additions to, and/or substitutions of one or more amino acids of the native sequence. The terms also include amino acid polymers in which one or more amino acids are chemical analogues of a corresponding naturally-occurring amino acid polymer.

The term "sequence identity" indicates a quantitative measure of the degree of homology between two sequences, which can be nucleic acid (also termed nucleotide) sequences or amino acid sequences. If the two sequences to be compared are not of equal length, they must be aligned to give the best possible fit, allowing the insertion of gaps or alternatively, truncation at the ends of the nucleic acid sequences or amino acid sequences.

In the case of a nucleotide sequence, for example, the term "at least 80% identical" thus means that at least 80% of the nucleotides over the entire sequence can be aligned with identical nucleotides from another sequence. A specified percentage of nucleotides can be referred to as e.g. 80% identical, 85% identical, 90% identical, 95% identical, 99% identical or more over a specified region when compared and aligned for maximum correspondence. For example, a sequence which is 10 nucleotides in length, say GGGAAACCTT, can be 80% identical with a continuous sequence (e.g., GGGAAACCGG), or with a non-continuous sequence (e.g., GGGACCCCTT):
100% identity example: 
GGGAAACCTT
| | | | | | | | | |
GGGAAACCTT
80% identity example: 
GGGAAACCTT
||||||||
GGGAAACCGG
80% identity example:

GGGAAACCTT
||||  ||||
GGGACCCCTT

In case a base designated "N" is found on a particular position within the reference nucleotide sequence, sequence identity is given for any of the bases adenine (A), cytosine (C), guanine (G) and thymine (T) or uracil (U), at the corresponding position on the compared sequence. See the following identity example. The same applies to amino acid sequence comparisons, i.e., in case an amino acid designated "X" is found on a particular position within the reference amino acid sequence, sequence identity is given for any amino acid at the corresponding position on the compared sequence.

80% identity example:

GGGAAANCCT
||||  ||||
GGGACCCCCT

The skilled person will acknowledge that various means for comparing sequences are available (see below).

As used herein, the term "antibody" refers to a glycoprotein comprising at least two heavy (H) chains and two light (L) chains inter-connected by disulfide bonds, or an antibody fragment (antigen binding portion) thereof. Each heavy chain is comprised of a heavy chain variable region (VH) and a heavy chain constant region (CH). The heavy chain constant region is comprised of three domains, CH1, CH2 and CH3. Each light chain is comprised of a light chain variable region (VL) and a light chain constant region (CL). The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host cells or factors, including various cells of the immune system (e.g., effector cells) and the first component (C1q) of the classical complement system. Antibodies of the invention include monoclonal antibodies (including full length monoclonal antibodies) and polyclonal antibodies, whole antibodies, chimeric antibodies, humanized antibodies, human antibodies or hybrid antibodies with dual or multiple antigen or epitope specificities, antibody fragments and antibody sub-fragments, e.g., Fab, Fab', F(ab')₂, fragments and the like, including hybrid fragments of any immunoglobulin or any natural, synthetic or genetically engineered protein that acts like an antibody by binding to a specific antigen to form a complex. Antibodies of the invention can also be Fc fusion proteins.

A "vector" is a nucleic acid that can be used to introduce another nucleic acid (or "construct") linked to it into a cell. One type of vector is a "plasmid", which refers to a linear or circular double stranded DNA molecule into which additional nucleic acid segments can be ligated. Another type of vector is a viral vector (e.g., replication defective retroviruses, adenoviruses and adeno-associated viruses), wherein additional DNA segments can be introduced into the viral genome. Certain vectors are capable of autonomous replication in a host cell into which they are introduced (e.g., bacterial vectors comprising a bacterial origin of replication and episomal mammalian vectors). Other vectors (e.g., non-episomal mammalian vectors) integrate into the genome of a host cell upon introduction into the host cell and culturing under selective pressure, and thereby are replicated along with the host genome. A vector can be used to direct the expression of a chosen nucleic acid in a cell.

A "host cell" is a cell that can be used to express a nucleic acid, e.g., a nucleic acid disclosed herein. A host cell can be a prokaryote, for example, *E. coli*, or it can be a eukaryote, for example, a single-celled eukaryote (e.g., a yeast or other fungus), a plant cell (e.g., a tobacco or tomato plant cell), an animal cell (e.g., a human cell, a monkey cell, a hamster cell, a rat cell, a mouse cell, or an insect cell) or a hybridoma. Exemplary host cells include Chinese hamster ovary (CHO) cell lines or their derivatives. Typically, a host cell is a cultured cell that can be transformed or transfected with a polypeptide-encoding nucleic acid, which can then be expressed in the host cell. It is understood that the term host cell refers not only to the particular subject cell but to the progeny or potential progeny of such a cell. Because certain modifications may occur in succeeding generations due to, e.g., mutation or environmental influence, such progeny may not, in fact, be identical to the parent cell, but are still included within the scope of the term as used herein.

The terms "treat" and "treatment" include therapeutic treatments, prophylactic treatments, and applications which reduce symptoms of a disorder or reduce the risk that a subject (e.g., a fish) will develop a disorder (e.g., symptoms of virus infection).

The term "vaccine" as used herein refers to a material that can produce an immune response that blocks the infectivity, either partially or fully, of an infectious agent, which in respect of the present invention is the virus affecting fish, e.g., lumpsuckers. Thus, when administering to a fish, the vaccine of the invention immunises the fish against the disease caused by the virus. The immunising component of the vaccine may be, e.g., DNA as in a DNA vaccine, RNA as in a RNA vaccine, a recombinant protein or fragment thereof according to the present invention, or a live or attenuated recombinant virus.

An "iRNA agent" (abbreviation for "interfering RNA agent") as used herein, is an RNA agent which can down-regulate (reduce) the expression of a target gene, e.g., a protein encoded by ORF-1, ORF-2, ORF-3, ORF-4 or ORF-5. An iRNA agent may act by one or more mechanisms, including post-transcriptional cleavage of a target mRNA sometimes referred to in the art as "RNAi", or pre-transcriptional or pre-translational mechanisms. An iRNA agent can be a double-stranded (ds) iRNA agent. An iRNA agent can also be a "small interfering RNA" (siRNA).

The terms "of the invention" or "according to the invention" as used herein are intended to refer to all aspects and embodiments of the invention disclosed and/or claimed herein. Conversely, any aspects, items or embodiments referred to herein as being "disclosed herein" or "described herein" are to be understood as being aspects, items or embodiments "of the invention" or "according to the invention".

As used herein, the term "comprising" is to be construed as encompassing both "including" and "consisting of", both meanings being specifically intended, and hence individually disclosed, embodiments according to the present invention.

As used herein, the articles "a" and "an" preceding an element or component are intended to be nonrestrictive regarding the number of instances (i.e. occurrences) of the element or component. Therefore, "a" or "an" is to be read to include one or at least one, and the singular word form of the element or component also includes the plural unless the number is obviously meant to be singular.

As used herein, the term "about" modifying the quantity of a substance, ingredient, component, or parameter employed refers to variation in the numerical quantity that can occur, for example, through typical measuring and handling procedures, e.g., liquid handling procedures used for making concentrates or solutions. Furthermore, variation can occur from inadvertent error in measuring procedures, differences in the manufacture, source, or purity of the ingredients employed to carry out the methods, and the like. In one embodiment, the term "about" means within 10% of the reported numerical value. In a more specific embodiment, the term "about" means within 5% of the reported numerical value.

### Virus nucleic acid sequences

One aspect of the invention provides a nucleic acid comprising at least one open reading frame (ORF) sequence selected from the group consisting of ORF-1, ORF-2, ORF-3, ORF-4 and ORF-5; wherein
ORF-1 is at least 80% identical to the nucleic acid sequence of SEQ ID NO:1,
ORF-2 is at least 80% identical to the nucleic acid sequence of SEQ ID NO:2,
ORF-3 is at least 80% identical to the nucleic acid sequence of SEQ ID NO:3,
ORF-4 is at least 80% identical to the nucleic acid sequence of SEQ ID NO:4, and
ORF-5 is at least 80% identical to the nucleic acid sequence of SEQ ID NO:5.

In some embodiments,
ORF-1 is at least 85% identical to the nucleic acid sequence of SEQ ID NO:1,
ORF-2 is at least 85% identical to the nucleic acid sequence of SEQ ID NO:2,
ORF-3 is at least 85% identical to the nucleic acid sequence of SEQ ID NO:3,
ORF-4 is at least 85% identical to the nucleic acid sequence of SEQ ID NO:4, and
ORF-5 is at least 85% identical to the nucleic acid sequence of SEQ ID NO:5.

In preferred embodiments,
ORF-1 is at least 90% identical to the nucleic acid sequence of SEQ ID NO:1,
ORF-2 is at least 90% identical to the nucleic acid sequence of SEQ ID NO:2,
ORF-3 is at least 90% identical to the nucleic acid sequence of SEQ ID NO:3,
ORF-4 is at least 90% identical to the nucleic acid sequence of SEQ ID NO:4, and
ORF-5 is at least 90% identical to the nucleic acid sequence of SEQ ID NO:5.

In more preferred embodiments,
ORF-1 is at least 95% identical to the nucleic acid sequence of SEQ ID NO:1,
ORF-2 is at least 95% identical to the nucleic acid sequence of SEQ ID NO:2,
ORF-3 is at least 95% identical to the nucleic acid sequence of SEQ ID NO:3,
ORF-4 is at least 95% identical to the nucleic acid sequence of SEQ ID NO:4, and
ORF-5 is at least 95% identical to the nucleic acid sequence of SEQ ID NO:5.

In yet more preferred embodiments,
ORF-1 is at least 98% identical to the nucleic acid sequence of SEQ ID NO:1,
ORF-2 is at least 98% identical to the nucleic acid sequence of SEQ ID NO:2,
ORF-3 is at least 98% identical to the nucleic acid sequence of SEQ ID NO:3,
ORF-4 is at least 98% identical to the nucleic acid sequence of SEQ ID NO:4, and
ORF-5 is at least 98% identical to the nucleic acid sequence of SEQ ID NO:5.

In yet even more preferred embodiments,
ORF-1 is at least 99% identical to the nucleic acid sequence of SEQ ID NO:1,
ORF-2 is at least 99% identical to the nucleic acid sequence of SEQ ID NO:2,
ORF-3 is at least 99% identical to the nucleic acid sequence of SEQ ID NO:3,
ORF-4 is at least 99% identical to the nucleic acid sequence of SEQ ID NO:4, and
ORF-5 is at least 99% identical to the nucleic acid sequence of SEQ ID NO:5.

In particularly preferred embodiments,
ORF-1 is the nucleic acid sequence of SEQ ID NO:1,
ORF-2 is the nucleic acid sequence of SEQ ID NO:2,
ORF-3 is the nucleic acid sequence of SEQ ID NO:3,
ORF-4 is the nucleic acid sequence of SEQ ID NO:4, and
ORF-5 is the nucleic acid sequence of SEQ ID NO:5.

In particular embodiments, the nucleic acid disclosed herein comprises at least ORF-1, ORF-2, ORF-3 and/or ORF-4, according to any of their embodiments disclosed herein.

The sequence of the nucleic acid disclosed herein can be at least 80% identical to the virus genome according to SEQ ID NO:6. In some embodiments, the sequence of the nucleic acid disclosed herein is at least 85% identical to the virus genome according to SEQ ID NO:6. In preferred embodiments, the sequence of the nucleic acid disclosed herein is at least 90% identical to the virus genome according to SEQ ID NO:6. In more preferred embodiments, the sequence of the nucleic acid disclosed herein is at least 95% identical to the virus genome according to SEQ ID NO:6. In yet more preferred embodiments, the sequence of the nucleic acid disclosed herein is at least 98% identical to the virus genome according to SEQ ID NO:6. In yet even more preferred embodiments, the sequence of the nucleic acid disclosed herein is at least 99% identical to the virus genome according to SEQ ID NO:6. In particularly preferred embodiments, the sequence of the nucleic acid disclosed herein has 100% identity to the sequence of the virus genome according to SEQ ID NO:6 (CLuCV).

Also provided herein is a nucleic acid the sequence of which is complementary to the sequence of any of the nucleic acids disclosed herein.

The sequence of said nucleic acid can be complementary to ORF-1, ORF-2, ORF-3, ORF-4 or ORF-5, according to any of their embodiments disclosed herein. The sequence of the nucleic acid can also be complementary to a nucleic acid sequence which is at least 80% identical to, in some embodiments at least 85% identical to, in preferred embodiments at least 90% identical to, in more preferred embodiments at least 95% identical to, in yet more preferred embodiments at least 98% identical to, in yet even more preferred embodiments at least 99% identical to, and in particularly preferred embodiments 100% identical to the sequence of the virus genome according to SEQ ID NO:6 (CLuCV).

In preferred embodiments, the nucleic acid sequences disclosed herein are RNA nucleic acid sequences, i.e., they contain the base uracil (U) instead of the base thymine (T). Accordingly, the viruses disclosed herein contain their genetic information in the form of such RNA nucleic acid sequences.

The skilled person will further acknowledge that alterations of the nucleic acid sequence resulting in modifications of the amino acid sequence of the protein it codes may have little, if any, effect on the resulting three dimensional structure of the protein. For example, a codon for the amino acid alanine, a hydrophobic amino acid, may be substituted by a codon encoding another less hydrophobic residue, such as glycine, or a more hydrophobic residue, such as valine, leucine, or isoleucine. Similarly, changes which result in the substitution of one negatively charged residue for another, such as aspartic acid for glutamic acid, or one positively charged residue for another, such as lysine for arginine, can also be expected to produce a protein with substantially the same functional activity.

Protein and/or nucleic acid sequence identities (homologies) can be evaluated using any of the variety of sequence comparison algorithms and programs known in the art. For sequence comparison, typically one sequence acts as a reference sequence (e.g., a sequence disclosed herein), to which test sequences are compared. A sequence comparison algorithm then calculates the percent sequence identities for the test sequences relative to the reference sequence, based on the program parameters.

The percent identity of two amino acid or two nucleic acid sequences can be determined for example by comparing sequence information using the computer program GAP, i.e., Genetics Computer Group (GCG; Madison, WI) Wisconsin package version 10.0 program, GAP (Devereux et al. (1984), Nucleic Acids Res. 12: 387-95). In calculating percent identity, the sequences being compared are typically aligned in a way that gives the largest match between the sequences. The preferred default parameters for the GAP program include: (1) The GCG implementation of a unary comparison matrix (containing a value of 1 for identities and 0 for non-identities) for nucleotides, and the weighted amino acid comparison matrix of Gribskov and Burgess, ((1986) Nucleic Acids Res. 14: 6745) as described in Atlas of Polypeptide Sequence and Structure, Schwartz and Dayhoff, eds., National Biomedical Research Foundation, pp. 353-358 (1979) or other comparable comparison matrices; (2) a penalty of 8 for each gap and an additional penalty of 2 for each symbol in each gap for amino acid sequences, or a penalty of 50 for each gap and an additional penalty of 3 for each symbol in each gap for nucleotide sequences; (3) no penalty for end gaps; and (4) no maximum penalty for long gaps.

Sequence identity and/or similarity can also be determined by using the local sequence identity algorithm of Smith and Waterman, 1981, Adv. Appl. Math. 2:482, the sequence identity alignment algorithm of Needleman and Wunsch, 1970, J. Mol. Biol. 48:443, the search for similarity method of Pearson and Lipman, 1988, Proc. Nat. Acad. Sci. U.S.A. 85:2444, computerized implementations of these algorithms (BESTFIT, FASTA, and TFASTA in the Wisconsin Genetics Software Package, Genetics Computer Group, 575 Science Drive, Madison, Wis.).

Another example of a useful algorithm is PILEUP. PILEUP creates a multiple sequence alignment from a group of related sequences using progressive, pairwise alignments. It can also plot a tree showing the clustering relationships used to create the alignment. PILEUP uses a simplification of the progressive alignment method of Feng & Doolittle, 1987, J. Mol. Evol. 35:351-360; the method is similar to that described by Higgins and Sharp, 1989, CABIOS 5:151-153. Useful PILEUP parameters including a default gap weight of 3.00, a default gap length weight of 0.10, and weighted end gaps.

Another example of a useful algorithm is the BLAST algorithm, described in: Altschul et al., 1990, J. Mol. Biol. 215:403-410; Altschul et al., 1997, Nucleic Acids Res. 25:3389-3402; and Karin et al., 1993, Proc. Natl. Acad. Sci. U.S.A. 90:5873-5787. A particularly useful BLAST program is the WU-BLAST-2 program obtained from Altschul et al., 1996, Methods in Enzymology 266:460-480. WU-BLAST-2 uses several search parameters, most of which are set to the default values. The adjustable parameters are set with the following values: overlap span=1, overlap fraction=0.125, word threshold (T)=II. The HSP S and HSP S2 parameters are dynamic values and are established by the program itself depending upon the composition of the particular sequence and composition of the particular database against which the sequence of interest is being searched; however, the values may be adjusted to increase sensitivity.

An additional useful algorithm is gapped BLAST as reported by Altschul et al., 1993, Nucl. Acids Res. 25:3389-3402. Gapped BLAST uses BLOSUM-62 substitution scores; threshold T parameter set to 9; the two-hit method to trigger ungapped extensions, charges gap lengths of k a cost of 10+k; Xᵤ set to 16, and X_{g} set to 40 for database search stage and to 67 for the output stage of the algorithms. Gapped alignments are triggered by a score corresponding to about 22 bits.

### Viruses

Another aspect of the invention provides a virus that infects and is capable of killing lumpsucker fish (*Cyclopterus lumpus*), wherein the virus genome comprises a nucleic acid sequence disclosed herein, wherein said nucleic acid sequence contains the base uracil (U) instead of the base thymine (T).

In preferred embodiments, the nucleic acid comprised within the virus is in the form of single-stranded RNA (ssRNA).

In some embodiments, the virus genome comprises a nucleic acid sequence comprising at least one of ORF-1, ORF-2, ORF-3, ORF-4 or ORF-5, according to any of their embodiments disclosed herein. In preferred embodiments, the sequence of the virus genome comprises at least ORF-1, ORF-2, ORF-3 and ORF-4, according to any of their embodiments disclosed herein. In some embodiments, the virus genome comprises a nucleic acid sequence which is, or which is complementary to, a nucleic acid sequence which is at least 80%, preferably at least 85%, more preferably at least 90%, even more preferably at least 95%, yet even more preferably 98%, particularly preferably 99%, or even 100% identical to the sequence of the virus genome according to SEQ ID NO:6 (CLuCV).

In some embodiments, the infection of lumpsucker fish by the virus disclosed herein causes the following symptoms in the fish:
(i) tissue damage in the intestine, and/or
(ii) diarrhoea.

In further embodiments, the infection of lumpsucker fish by the virus disclosed herein additionally causes anorexia.

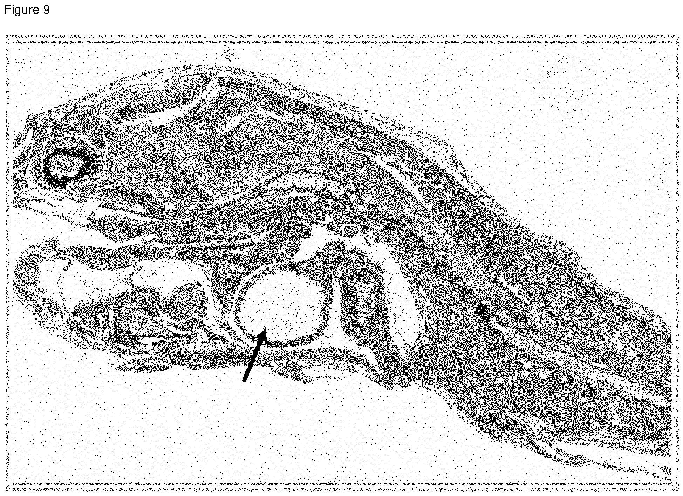

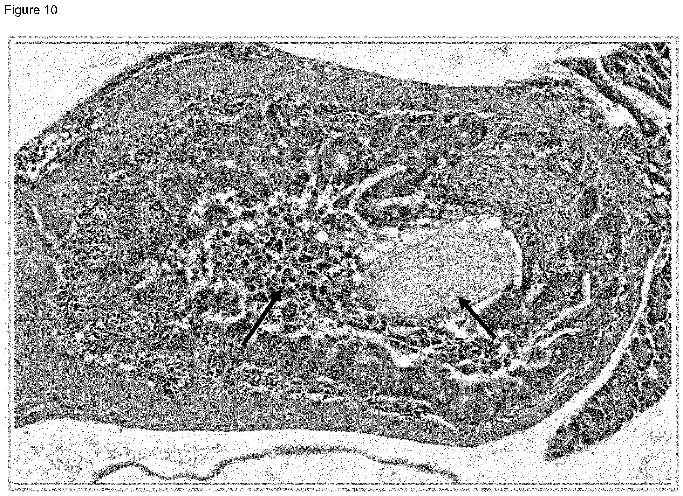

Tissue damage in the intestine can be diagnosed by the use of histology or electron microscopy to observe destruction of intestinal tissue, for example, destruction of the structure of villi, increased thickness of other layers. Preferably, tissue sections are stained using a Haematoxylin and Eosin (H&E) histological stain. In employing such a stain, infected individuals can be observed with accumulation of fluids and undigested feed particles in both the stomach and the intestine (see, e.g., Figures 9 to 12), leading to a diarrhoea-like condition in these fish. In addition, damage to the intestinal wall, with cellular discharge and increased mucus production can be observed (see, e.g., Figures 10 to 12). Fish diarrhoea can be observed in the water tanks of the farmed fish.

Changes in bio-macromolecule components (proteins in general, siderophile proteins, neutral mucopolysaccharides, glycogen and acid mucopolysaccharides) can also be observed in intestinal tissue samples by conventional methods, e.g., Western blotting, ELISA, staining of tissue sections, etc.

Anorexia in fish can be observed by the fish not feeding.

In some embodiments, the virus is an enveloped virus. Enveloped viruses have a protective layer (envelope) covering their protein capsids. The envelopes are typically derived from portions of the host cell membranes (phospholipids and proteins), but include some viral glycoproteins. They may help viruses avoid the host immune system.

In some embodiments, the virus is a Coronavirus. In preferred embodiments, the virus is a Torovirus. Such viruses are enveloped, round-shaped but pleomorphic, and around 100 to 150 nm in diameter. The virus particle typically has surface spikes proteins that are club-shaped and are evenly dispersed over the surface.

In some embodiments, the virus disclosed herein comprises a 5' untranslated region (5' UTR) which functions as an internal ribosome entry site (IRES).

In preferred embodiments, ORF-1 codes for a polyprotein, ORF-2 codes for a spike glycoprotein, ORF-3 codes for a membrane protein and ORF-4 codes for a nucleocapsid protein.

Also provided herein is a vector comprising a nucleic acid that encodes at least one ORF, as disclosed herein with all their embodiments. In some embodiments, the vector comprises a nucleic acid that encodes the complete virus disclosed herein. The vector can be used to introduce said nucleic acid(s) into a cell, such as a host cell.

Also provided herein is a host cell comprising the virus disclosed herein. The host cell may be a bacterial cell, a fish cell or a mammalian cell.

### Oligonucleotide primers

Another aspect of the invention provides an oligonucleotide primer which comprises a sequence of at least 9 nucleotides, wherein said sequence is complementary to a nucleic acid sequence which is comprised within the genome of the virus disclosed herein.

In some embodiments, the oligonucleotide primer is 9 to 60 nucleotides in length. In preferred embodiments, the oligonucleotide primer is 12 to 40 nucleotides in length. In more preferred embodiments, the oligonucleotide primer is 15 to 30 nucleotides in length. In even more preferred embodiments, the oligonucleotide primer is 18 to 25 nucleotides in length.

As the skilled person will readily appreciate, an oligonucleotide primer which is complementary to a nucleic acid sequence will hybridize to that sequence under stringent conditions. The "stringent conditions" refer to conditions of temperature, ionic strength, and the presence of other compounds such as organic solvents, under which nucleic acid hybridizations are conducted. Under stringent conditions, nucleic acid base pairing will occur only between nucleic acid seqeunces having a high frequency of complementary bases. Stringent hybridization conditions are known to the skilled person (see e.g. Green M. R., Sambrook, J., Molecular Cloning: A Laboratory Manual, Cold Spring Harbor Laboratory Press; 4th edition, 2012). The precise conditions for stringent hybridization are typically sequence-dependent and will be different in different circumstances, as the skilled person will readily appreciate. Longer sequences hybridize at higher temperatures compared to shorter sequences. Generally, stringent conditions are selected to be about 5°C lower than the thermal melting point (Tm) for the specific sequence. The Tm is defined as the temperature when 50% of the duplex molecules have dissociated into their constituent single strands. Since the target sequences are generally present at excess, at Tm, 50% of nucleic acid primers would generally be occupied at equilibrium. Typically, stringent conditions will be those in which the salt concentration is less than about 1.0 M sodium ions, typically about 0.01 to 1.0 M sodium ions (or other salts) at pH 6.8 to 8.3 and the temperature is at least about 30°C for short primers (e.g., 10 nucleotides to 50 nucleotides) and at least about 60°C for longer primers. Stringent conditions may also be achieved with the addition of destabilizing agents, such as formamide. As the skilled person will readily appreciate, due to sequence complementarity of the sequences, the oligonucleotide primer according to the invention therefore hybridizes to a nucleic acid sequence which is comprised within the genome of the virus disclosed herein.

An oligonucleotide primer according to the present invention may be labelled with a molecular marker in order to enable visualization of hybridization to target sequence or quantification of amplification of target sequence. Various molecular markers or labels are known to the skilled person.

In a particular embodiment, herein provided is an oligonucleotide primer which comprises at least 9 consecutive nucleotides of a sequence which is, or which is complementary to, a portion (e.g., 9 to 60 nucleotides in length, preferably 12 to 40 nucleotides in length, more preferably 15 to 30 nucleotides in length, even more preferably 18 to 25 nucleotides in length) of a reference nucleic acid sequence selected from the group consisting of SEQ ID NO:1, SEQ ID NO:2, SEQ ID NO:3, SEQ ID NO:4, SEQ ID NO:5 or SEQ ID NO:6. In a particularly preferred embodiment, the sequence of said oligonucleotide primer consists of said sequence of consecutive nucleotides.

In another particular embodiment, herein provided is an oligonucleotide primer which comprises at least 9 (preferably at least 12, more preferably at least 15, even more preferably at least 18) nucleotides of a sequence which is at least 80% identical to a sequence which is, or which is complementary to, a sequence selected from the group consisting of SEQ ID NO:12, SEQ ID NO:13, SEQ ID NO:14, SEQ ID NO:15, SEQ ID NO:16, SEQ ID NO:17, SEQ ID NO:18, SEQ ID NO:19, and SEQ ID NO: 20.

Another aspect of the invention provides a use of at least one oligonucleotide primer in a method of detecting the virus disclosed herein, wherein the at least one primer comprises a sequence of at least 9 nucleotides (preferably at least 12, more preferably at least 15, even more preferably at least 18) and wherein said sequence is complementary to a nucleic acid sequence which is comprised within the genome of said virus.

In some embodiments, the at least one oligonucleotide primer is a primer pair, i.e., two primers, one forward primer and one reverse primer, which are complementary to two regions on a nucleic acid sequence, and which can be used to amplify a sequence between said two regions. This is well known to a skilled person, and it is within his/her skills to find oligonucleotide primers suitable to constitute a pair. According to the use of the invention, a "primer pair" can be used to amplify a nucleic acid sequence which is comprised within the genome of the virus disclosed herein.

In some embodiments, the use employs cDNA synthesis in a method of detecting the virus disclosed herein. For example, random oligonucleotide primers (e.g., hexanucleotides) are used to synthesize cDNAs from a nucleic acid sequence which is comprised within the genome of said virus.

In some embodiments, the use employs PCR in a method of detecting the virus disclosed herein. In some embodiments, the use employs RT-PCR in a method of detecting the virus disclosed herein. In some embodiments, the use employs RT-qPCR in a method of detecting the virus disclosed herein. In some embodiments, the use employs Random Multiplex RT-PCR in a method of detecting the virus disclosed herein; this method uses a mixture of primers designed to be resistant to primer-dimer amplification (see Clem et al, Virol J, 4, 2007). In some embodiments, the use employs transcription mediated amplification (TMA) in a method of detecting the virus disclosed herein. In some embodiments, the use employs strand displacement amplification (SDA) in a method of detecting the virus disclosed herein.

In some embodiments, the use employs *in situ* detection, also termed *in situ* hybridization (ISH) in a method of detecting the virus disclosed herein, for example fluorescence *in situ* hybridization (FISH). ISH uses a labelled complementary DNA, RNA or modified oligonucleotide primer sequence (probe) to enable visualization of specific nucleic acids in morphologically preserved cells and tissue sections. The probe can be labelled with radio-, fluorescent- or antigen-labels (e.g., digoxigenin), which can then be localized and quantified in the tissue using either autoradiography, fluorescence microscopy, or immunohistochemistry, respectively.

### Diagnostic methods

Another aspect of the invention provides a method for detecting a virus that infects and is capable of killing fish, comprising the steps of:
(a) contacting a nucleic acid extracted from a biological sample of a fish with at least one oligonucleotide primer to form a mixture, wherein the at least one oligonucleotide primer is complementary to a nucleic acid sequence which is comprised within the genome of the virus disclosed herein, and
(b) determining whether upon subjecting the mixture of a) to amplification an amplification product is present, wherein the presence of amplification product indicates the presence of RNA associated with the virus, and hence the presence of the virus in the biological sample.

In some embodiments, nucleic acid in step (a) of the method, for example RNA, is extracted from biological samples by using solid-phase extraction, e.g., on-column purification using a solid phase of silica gel membrane. In some embodiments, nucleic acid in step (a) of the method, for example RNA, is extracted from biological samples by using phenol/chloroform extraction.

The method may use any suitable oligonucleotide primer disclosed herein. Generally, the at least one oligonucleotide primer of step (a) of the method is chosen to produce an amplification product according to step (b) which has a length of 45 nucleotides to 3000 nucleotides. However, amplification products even smaller or greater in length may suitably be produced by the methods and oligonucleotide primer disclosed herein.

In some embodiments, the at least one oligonucleotide primer of step (a) of the method is chosen to produce an amplification product according to step (b) for a PCR or RT-PCR assay which has a length of 100 nucleotides to 2500 nucleotides. In preferred embodiments, the at least one oligonucleotide primer of step (a) of the method is chosen to produce an amplification product according to step (b) for a PCR or RT-PCR assay which has a length of 200 nucleotides to 1500 nucleotides. In more preferred embodiments, the at least one oligonucleotide primer of step (a) of the method is chosen to produce an amplification product according to step (b) for a PCR or RT-PCR assay which has a length of 300 nucleotides to 1000 nucleotides.

In some embodiments, the at least one oligonucleotide primer of step (a) of the method is chosen to produce an amplification product according to step (b) for a real-time RT-PCR assay which has a length of 45 nucleotides to 500 nucleotides. In preferred embodiments, the at least one oligonucleotide primer of step (a) of the method is chosen to produce an amplification product according to step (b) for a real-time RT-PCR assay which has a length of 50 nucleotides to 350 nucleotides. In more preferred embodiments, the at least one oligonucleotide primer of step (a) of the method is chosen to produce an amplification product according to step (b) for a real-time RT-PCR assay which has a length of 55 nucleotides to 250 nucleotides.

In some embodiments, the amplification of step (b) of the method employs PCR. In some embodiments, the amplification of step (b) of the method employs RT-PCR. In some embodiments, the amplification of step (b) of the method employs RT-qPCR.

In some embodiments, the amplification product of step (b) of the method is determined by Southern blot. In some embodiments, the amplification product of step (b) of the method is determined by Northern blot. In some embodiments, the amplification product of step (b) of the method is determined by spectrophotometry. In some embodiments, the amplification product of step (b) of the method is determined by use of a DNA dye. In some embodiments, the amplification product of step (b) of the method is determined by quantifying the presence of a labelled oligonucleotide primer, for example, quantifying the presence of a fluorescently-labelled oligonucleotide primer.

Another aspect of the invention provides a method for detecting a virus that infects and is capable of killing fish, comprising the steps of:
(a) sequencing a nucleic acid extracted from a biological sample of a fish, and
(b) comparing the resulting nucleic acid sequence with a nucleic acid sequence which is, or which is complementary to, a reference sequence selected from the group consisting of SEQ ID NO:1, SEQ ID NO:2, SEQ ID NO:3, SEQ ID NO:4, SEQ ID NO:5 and SEQ ID NO:6, wherein an at least 80% sequence identity between the two sequences indicates the presence of the virus in the biological sample.

In some embodiments, nucleic acid in step (a) of the method, for example RNA, is extracted from biological samples by using solid-phase extraction, e.g., on-column purification using a solid phase of silica gel membrane. In some embodiments, nucleic acid in step (a) of the method, for example RNA, is extracted from biological samples by using phenol/chloroform extraction.

In preferred embodiments, the sequencing in step (a) of the method provides a DNA sequence, which can be directly compared to the reference DNA sequences selected from the group consisting of SEQ ID NO:1, SEQ ID NO:2, SEQ ID NO:3, SEQ ID NO:4, SEQ ID NO:5 and SEQ ID NO:6.

In some embodiments, the sequencing in step (a) of the method is performed by Sanger sequencing (chain termination method). In preferred embodiments, the sequencing in step (a) of the method is performed by Next Generation Sequencing (NGS), preferably Illumina (Solexa) sequencing, Roche 454 sequencing, Ion Torrent or SOLiD sequencing (Goodwin S, et al., (2016) Coming of age: Ten years of next-generation sequencing technologies. Nature reviews, Genetics, 17, 333-351).

In some embodiments, an at least 85% sequence identity between the two sequences in step (b) of the method indicates the presence of the virus in the biological sample.

In preferred embodiments, an at least 90% sequence identity between the two sequences in step (b) of the method indicates the presence of the virus in the biological sample.

In more preferred embodiments, an at least 95% sequence identity between the two sequences in step (b) of the method indicates the presence of the virus in the biological sample.

In yet more preferred embodiments, an at least 98% sequence identity between the two sequences in step (b) of the method indicates the presence of the virus in the biological sample.

In yet even more preferred embodiments, an at least 99% sequence identity between the two sequences in step (b) of the method indicates the presence of the virus in the biological sample.

In a particularly preferred embodiment, a 100% sequence identity between the two sequences in step (b) of the method indicates the presence of the virus in the biological sample.

### Antibodies

Another aspect of the invention provides an antibody that binds a polypeptide, wherein the polypeptide is encoded by a nucleic acid sequence which is comprised within the genome of the virus disclosed herein.

In some embodiments, the polypeptide is selected from the group consisting of:
(i) a polypeptide comprising an amino acid sequence which is at least 80%, preferably at least 85%, more preferably at least 90%, yet more preferably at least 95%, yet even more preferably at least 98%, particularly preferably at least 99%, or even 100% identical to, or which is, SEQ ID NO:7;
(ii) a polypeptide comprising an amino acid sequence which is at least 80%, preferably at least 85%, more preferably at least 90%, yet more preferably at least 95%, yet even more preferably at least 98%, particularly preferably at least 99%, or even 100% identical to, or which is, SEQ ID NO:8;
(iii) a polypeptide comprising an amino acid sequence which is at least 80%, preferably at least 85%, more preferably at least 90%, yet more preferably at least 95%, yet even more preferably at least 98%, particularly preferably at least 99%, or even 100% identical to, or which is, SEQ ID NO:9;
(iv) a polypeptide comprising an amino acid sequence which is at least 80%, preferably at least 85%, more preferably at least 90%, yet more preferably at least 95%, yet even more preferably at least 98%, particularly preferably at least 99%, or even 100% identical to, or which is, SEQ ID NO:10; and
(v) a polypeptide comprising an amino acid sequence which is at least 80%, preferably at least 85%, more preferably at least 90%, yet more preferably at least 95%, yet even more preferably at least 98%, particularly preferably at least 99%, or even 100% identical to, or which is, SEQ ID NO:11.

In some embodiments, the antibody is a polyclonal antibody. In some embodiments, the antibody is a monoclonal antibody.

The antibodies disclosed herein may be prepared by genetic immunization methods in which native proteins are expressed in vivo with normal post-transcriptional modifications, avoiding antigen isolation or synthesis. For example, hydrodynamic tail or limb vein delivery of naked plasmid DNA expression vectors can be used to produce the antigen of interest in vivo in mice, rats, and rabbits and thereby induce antigen-specific antibodies (Tang et al, Nature 356(6365): 152-4 (1992); Tighe et al, Immunol. Today 19(2) 89-97 (1998); Bates et al, Biotechniques, 40(2) 199-208 (2006)). This allows the efficient generation of high-titre, antigen-specific antibodies. Antibodies can also be derived by *in vitro* methods. Suitable examples include but are not limited to hybridoma technologies, phage display, yeast display and the like.

### Kits

Another aspect of the invention provides a kit for detecting a virus in a biological sample from fish, wherein the kit comprises an oligonucleotide primer disclosed herein and/or an antibody disclosed herein.

In some embodiments, the kit is a real-time RT-PCR assay, for example the kit is a real-time RT-qPCR assay.

In some embodiments, the kit is for detecting a Coronavirus in a biological sample from fish. In preferred embodiments, the kit is for detecting a Torovirus in a biological sample from fish. In more preferred embodiments, the kit is for detecting a Torovirus in a biological sample from lumpsucker fish.

### Medical Uses and Vaccines

Another aspect of the invention provides an antibody for use in treating fish, particularly lumpsucker fish, against disease caused by Coronavirus, specifically Torovirus, infection. In a preferred embodiment, the antibody is for use in treating fish, particularly lumpsucker fish, against disease caused by the virus disclosed herein (CLuCV). The antibody binds a polypeptide which is encoded by a nucleic acid sequence comprised within the genome of the virus disclosed herein.

In preferred embodiments, the fish is a lumpsucker fish.

In some embodiments, the fish show the following symptoms:
(i) tissue damage in the intestine, and/or
(ii) diarrhoea.

In further embodiments, the fish additionally show symptoms of anorexia.

The symptoms can be determined as described above.

Another aspect of the invention provides a use of virus disclosed herein for producing a vaccine against disease caused by said virus.

Another aspect of the invention provides a vaccine comprising:
(i) a nucleic acid of a sequence which is comprised within the genome of the virus disclosed herein;
(ii) a polypeptide encoded by a nucleic acid sequence which is comprised within the genome of the virus disclosed herein; or
(ii) a virus disclosed herein.

The vaccine is for protecting fish, particularly lumpsucker fish, against disease caused by Coronavirus, specifically Torovirus, infection. In a preferred embodiment, the vaccine is for protecting fish, particularly lumpsucker fish, against disease caused by infection with the virus disclosed herein (CLuCV).

In some embodiments where the vaccine comprises a nucleic acid of a sequence comprised within the genome of the virus disclosed herein, said nucleic acid sequence comprises at least one of ORF-1, ORF-2, ORF-3, ORF-4 or ORF-5, according to any of their embodiments disclosed herein. In preferred embodiments where the vaccine comprises a nucleic acid of a sequence comprised within the genome of the virus disclosed herein, said nucleic acid sequence comprises at least ORF-1, ORF-2, ORF-3 and ORF-4, according to any of their embodiments disclosed herein. In some embodiments where the vaccine comprises a nucleic acid of a sequence comprised within the genome of the virus disclosed herein, said nucleic acid sequence is, or is complementary to, a nucleic acid sequence which is at least 80%, preferably at least 85%, more preferably at least 90%, yet more preferably at least 95%, even more preferably at least 98%, particularly preferably at least 99%, or even 100% identical to, the sequence of the virus genome according to SEQ ID NO:6 (CLuCV).

The nucleic acid may be DNA or RNA.

In some embodiments where the vaccine comprises a polypeptide encoded by a nucleic acid sequence which is comprised within the genome of the virus disclosed herein, said polypeptide is selected from the group consisting of:
(i) a polypeptide comprising an amino acid sequence which is at least 80%, preferably at least 85%, more preferably at least 90%, yet more preferably at least 95%, yet even more preferably at least 98%, particularly preferably at least 99%, or even 100% identical to, or which is, SEQ ID NO:7;
(ii) a polypeptide comprising an amino acid sequence which is at least 80%, preferably at least 85%, more preferably at least 90%, yet more preferably at least 95%, yet even more preferably at least 98%, particularly preferably at least 99%, or even 100% identical to, or which is, SEQ ID NO:8;
(iii) a polypeptide comprising an amino acid sequence which is at least 80%, preferably at least 85%, more preferably at least 90%, yet more preferably at least 95%, yet even more preferably at least 98%, particularly preferably at least 99%, or even 100% identical to, or which is, SEQ ID NO:9;
(iv) a polypeptide comprising an amino acid sequence which is at least 80%, preferably at least 85%, more preferably at least 90%, yet more preferably at least 95%, yet even more preferably at least 98%, particularly preferably at least 99%, or even 100% identical to, or which is, SEQ ID NO:10; and
(v) a polypeptide comprising an amino acid sequence which is at least 80%, preferably at least 85%, more preferably at least 90%, yet more preferably at least 95%, yet even more preferably at least 98%, particularly preferably at least 99%, or even 100% identical to, or which is, SEQ ID NO:11.

In some embodiments where the vaccine comprises a virus disclosed herein, the genome of said virus comprises a nucleic acid sequence which is an RNA nucleic acid sequence comprising at least one of ORF-1, ORF-2, ORF-3, ORF-4 or ORF-5, according to any of their embodiments disclosed herein. In some embodiments where the vaccine comprises a virus disclosed herein, the genome of said virus comprises a nucleic acid sequence which is an RNA nucleic acid sequence which is, or which is complementary to, a nucleic acid sequence which is at least 80%, preferably at least 85%, more preferably at least 90%, even more preferably at least 95%, yet even more preferably 98%, particularly preferably 99%, or even 100% identical to the sequence of the virus genome according to SEQ ID NO:6 (CLuCV).

In some embodiments, the vaccine comprises an amount of antigen which is in the range of 0.05 to 1.0 mg/ml, such as from 0.1 to 0.5 mg/ml, from 0.15 to 0.4 mg/ml, or from 0.2 to 0.3 mg/ml. The vaccine may be for administration in dosages of 0.005 to 0.5 mg/individual, preferably from 0.01 to 0.05 mg/individual, more preferably from 0.01 to 0.02 mg/individual.

In some embodiments, the vaccine comprises an amount of antigen corresponding to a TCID₅₀ of 10⁵ to 10¹⁰ per dosage, preferably a TCID₅₀ of 10⁶ to 10⁹ per dosage.

The vaccine may be in the form of a suspension of the virus or it may be lyophilized. In a lyophilized vaccine it may be useful to add one or more stabilizers. Suitable stabilizers are for example carbohydrates such as sorbitol, mannitol, starch, sucrose, dextran; protein containing agents such as bovine serum or skimmed milk; and buffers such as alkali metal phosphates.

The vaccine according to the invention may further be in a formulation comprising an adjuvant. Examples of adjuvants frequently used in fish and shellfish farming are muramyldipeptides, lipopolysaccharides, several glucans and glycans, mineral oil, Montanide™ and Carbopol®. An overview of adjuvants suitable for fish vaccines is given in the review paper of Sommerset (Expert Rev. Vaccines 4(1), 89-101 (2005)).

The vaccine of the invention may further comprise a suitable pharmaceutical carrier. In some embodiments the vaccine is formulated as an emulsion of water in oil. The vaccine may also comprise a so-called "vehicle". A vehicle is a device to which the antigen adheres, without being covalently bound to it. Such vehicles are inter alia biodegradable nano/micro-particles or -capsules of PLGA (poly-lactide-co-glycolic acid), alginate or chitosan, liposomes, niosomes, micelles, multiple emulsions and macrosols, all known in the art. A special form of such a vehicle, in which the antigen is partially embedded in the vehicle, is the so-called ISCOM.

In addition, the vaccine may comprise one or more suitable surface-active compounds or emulsifiers, e.g., Cremophore®, Tween® and Span®. Also, adjuvants such as interleukin, CpG and glycoproteins may be used.

In some embodiments, the vaccine is provided in fish feed, said feed may for example be pelleted or extruded feed.

Another aspect of the invention provides an interfering RNA (iRNA) molecule for use in treating fish, particularly lumpsucker fish, against disease caused by Coronavirus, specifically Torovirus, infection. In a preferred embodiment, the iRNA is for use in treating fish, particularly lumpsucker fish, against disease caused by the virus disclosed herein (CLuCV). In a particular embodiment, the iRNA molecule comprises at least 12 (preferably contiguous) nucleotides of, or complementary to, a nucleic acid sequence comprised within the genome of the virus disclosed herein.

In preferred embodiments, the fish is a lumpsucker fish.

In some embodiments, the fish show the following symptoms:
(i) tissue damage in the intestine, and/or
(ii) diarrhoea.

In further embodiments, the fish additionally show symptoms of anorexia.

The symptoms can be determined as described above.

In some embodiments, the iRNA molecule comprises at least 12 (preferably contiguous) nucleotides of, or complimentary to, a nucleic acid sequence comprising ORF-1, ORF-2, ORF-3, ORF-4 or ORF-5, according to any of their embodiments disclosed herein. In some embodiments, the iRNA molecule comprises at least 12 (preferably contiguous) nucleotides of, or complimentary to, a nucleic acid sequence which is at least 80%, preferably at least 85%, more preferably at least 90%, even more preferably at least 95%, yet even more preferably 98%, particularly preferably 99%, or even 100% identical to the sequence of the virus genome according to SEQ ID NO:6 (CLuCV).

### Sequences

The sequences referred to in the present disclosure are the following (see also the Figures and the sequence listing):

**Table 1**

| **Sequence** | **Description** |
|---|---|
| SEQ ID NO: 1 | CLuCV_ORF-1 nucleotide sequence (Polyprotein) |
| SEQ ID NO: 2 | CLuCV_ORF-2 nucleotide sequence (Spike Glycoprotein) |
| SEQ ID NO: 3 | CLuCV_ORF-3 nucleotide sequence (Membrane protein) |
| SEQ ID NO: 4 | CLuCV_ORF-4 nucleotide sequence (Nucleocapsid) |
| SEQ ID NO: 5 | CLuCV_ORF-5 nucleotide sequence (Unknown) |
| SEQ ID NO: 6 | CLuCV_Genome nucleotide sequence |
| SEQ ID NO: 7 | CLuCV_ORF-1 amino acid sequence (Polyprotein) |
| SEQ ID NO: 8 | CLuCV_ORF-2 amino acid sequence (Spike Glycoprotein) |
| SEQ ID NO: 9 | CLuCV_ORF-3 amino acid sequence (Membrane protein) |
| SEQ ID NO: 10 | CLuCV_ORF-4 amino acid sequence (Nucleocapsid) |
| SEQ ID NO: 11 | CLuCV_ORF-5 amino acid sequence (Unknown) |
| SEQ ID NO: 12 | TCL-ORF-1 Forward primer |
| SEQ ID NO: 13 | TCL-ORF-1 Taqman Probe |
| SEQ ID NO: 14 | TCL-ORF-1 Reverse primer |
| SEQ ID NO: 15 | TCL-M Forward primer |
| SEQ ID NO: 16 | TCL-M Taqman Probe |
| SEQ ID NO: 17 | TCL-M Reverse primer |
| SEQ ID NO: 18 | TCL-S Forward primer |
| SEQ ID NO: 19 | TCL-S Taqman Probe |
| SEQ ID NO: 20 | TCL-S Reverse primer |

### Examples

The following examples illustrate the present invention. They are intended to aid in the understanding of the invention, and they should not be construed to in any way limit the scope of the invention.

### Example 1: Identification of CLuCV

Fish samples were obtained from a lumpsucker (Cyclopterus lumpus) farming site experiencing high mortality (60-80%). The samples were analyzed using standard real-time RT-PCR and histology. No known pathogens were identified using real-time RT-PCR. Histological analysis indicated signs of tissue damage in the intestine in the affected individuals, but no potentially pathogenic bacteria and no microparasites could be observed.

Total RNA was extracted from moribund fish in a phenol/chloroform extraction protocol (Qiagen RNeasy® 96 Universal Tissue Kit), and this RNA was used as a template in a Next Generation Sequencing (NGS) analysis. The NGS analysis was performed by the company BaseClear (https://www.baseclear.com/), and yielded approximately 98 million sequence reads, where the majority came from the host transcriptome. Approximately 28,600 of the assembled reads from the NGS analysis were found to have no significant nucleotide match in the National Center for Biotechnology Information (NCBI) GenBank. From these reads, a novel virus sequence - herein termed Cyclopterus lumpus Coronavirus or CLuCV - was identified and characterized by the inventors. The DNA sequences obtained from the NGS analysis are provided in Figures 2 to 5 (see also SEQ ID Nos: 1 to 6). However, it will be appreciated that the corresponding RNA sequences are present in the virus CLuCV.

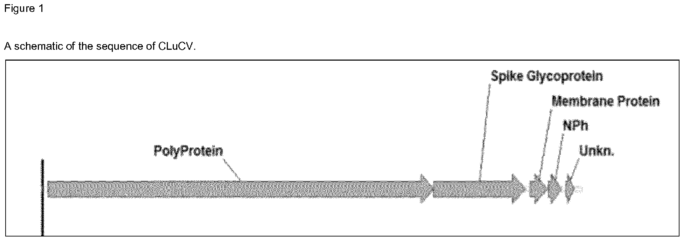

The sequence of CLuCV was identified to be 24,613 nucleotides long, and it contains five possible open reading frames (ORFs). A schematic is shown in Figure 1. A relationship between CLuCV and other known viruses could only be determined by comparison of translated amino acid sequences. The length and organization of the genome, together with downstream sequence analyses, indicate that it is a novel Torovirus, in the Coronaviridae family, with the Berne virus (isolated from horses) as its closest relative.

Preliminary results indicate that, at least in Norway, CLuCV has a significant presence in farmed lumpsucker.

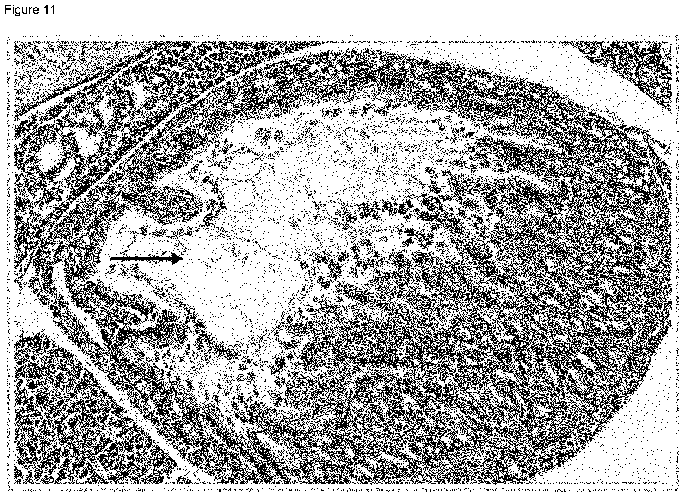

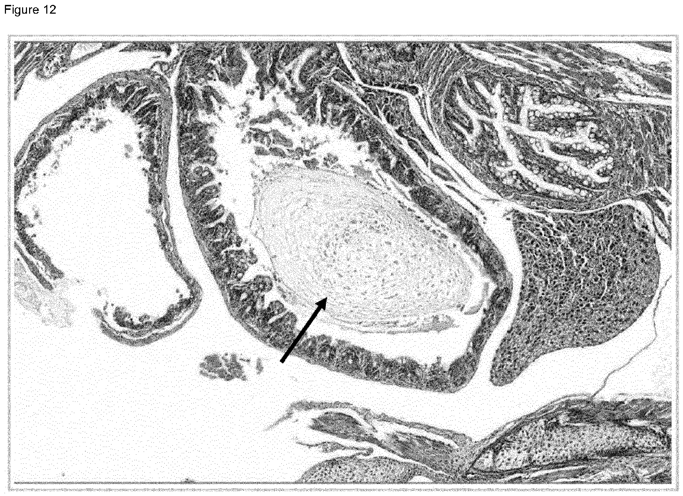

The pathology associated with CLuTV in lumpsucker is shown by Figures 9 to 12. Lumpsucker sections were stained using a Haematoxylin and Eosin (H&E) histological stain. Figure 9 is a whole section of affected lumpsucker, showing accumulation of fluid in the stomach (arrow). Figure 10 is a section of lumpsucker intestine showing accumulation of mucus, and cellular discharge (arrows). Figure 11 is a section of lumpsucker intestine showing accumulation of mucus (arrow), and cellular discharge. Figure 12 is a section of lumpsucker intestine showing accumulation of mucus (arrow).

### Example 2: Nucleotide sequence analysis of CLuCV

When performing a standard nucleotide BLAST search using the CLuCV sequence, no match was found in the NCBI GeneBank. If parameters of the search were altered to include more dissimilar sequences in the search, some sequence regions from other known viruses were identified. The percentage of nucleotide sequence identity ("Seq. Id.") between these CLuCV regions and regions from other known viruses is shown in Table 2.

**Table 2. Best nucleotide match found between CLuCV and known viruses in the NCBI GeneBank**

| ***CL_VirusB Region*** | | ***Best sequence region match (NCBI GeneBank)*** | | | | | |
|---|---|---|---|---|---|---|---|
| Start | Stop | Start | Stop | bp | Seq. Id. (%) | Acc. No. | Description |
| 11266 | 12315 | 15788 | 16837 | 1069 | 64 % | MG996765 | Berne virus, isolate P138/72 |
| 11266 | 12315 | 2314 | 3363 | 1069 | 64 % | X52374 | Berne virus mRNA, for RdRp |
| 11366 | 11893 | 15040 | 15931 | 535 | 67 % | KM403390 | Porcine torovirus, strain PToV/NPL/2014 |
| 11366 | 11893 | 15400 | 15874 | 536 | 66 % | JQ860350 | Porcine torovirus, strain SH1 |
| 11256 | 11849 | 15443 | 16036 | 601 | 65 % | AY427798 | Breda virus strain Breda 1 |

### Example 3: Amino acid sequence analysis of CLuCV

Translation of the ORFs of CLuCV yields a total of five potential proteins. The amino acid identity between the CLuCV ORFs and other known proteins from other viruses is shown in Table 3. Only the best match for each ORF is provided.

**Table 3. Best amino acid sequence match found between CLuCV and known viruses in the NCBI GeneBank**

| ***CL_VirusB*** | | ***Best αα sequence match (NCBI GeneBank)*** | | | | | |
|---|---|---|---|---|---|---|---|
| ORF | Amino acids | Total Score | Query cover. | E-value | Id. (%) | Acc. No. | Description |
| ORF01 | 5867 | 2017 | 64 % | 0.0 | 39 % | AWV66923 | Polyprotein1ab, Berne virus |
| ORF02 | 1391 | 435 | 54 % | 8,00E-125 | 34 % | CAE01338 | Spike glycoprotein, Porcine Torovirus |
| ORF03 | 260 | 42.4 | 71 % | 0.007 | 26 % | YP337908 | Membrane glycoprotein, Breda virus |
| ORF04 | 201 | NA | NA | NA | NA | NA | No match |
| ORF05 | 113 | NA | NA | NA | NA | NA | No match |

ORF-1 corresponds to a polyprotein. ORF-2 corresponds to a spike glycoprotein. ORF-3 corresponds to a membrane glycoprotein. ORF-4 corresponds to a Nucleocapsid protein. ORF-5 corresponds to an unknown protein.

### Example 4: Evaluating the presence of CLuCV in farmed lumpfish populations

Three separate real-time RT-PCR assays (TCL-ORF-1, TCL-M and TCL-S) were designed using the primers according to SEQ ID NOs: 12 to 14, 15 to 17 and 18 to 20, respectively. TCL-ORF-1 targeted ORF-1, TCL-M targeted ORF-3 (membrane protein), and TCL-S targeted ORF-2 (spike glycoprotein). The original fish sample material was confirmed to be positive for CLuCV using all three of these assays.

For more extensive investigation, assay TCL-ORF-1 was selected to evaluate the presence of CLuCV in existing farmed lumpfish populations. The results from screening different lumpfish populations in Norway are shown in Table 4.

**Table 4. Results from testing of NGS material and field material**

| ***Screening of lumpfish populations (Norway)*** | | | |
|---|---|---|---|
| County | Samples (N) | Positive (N) | Prevalence (%) |
| Finnmark | 60 | 5 | 8,3 |
| Troms | 28 | 0 | 0,0 |
| Møre | 67 | 4 | 6,0 |
| Trøndelag | 64 | 19 | 29,7 |
| Rogaland | 20 | 11 | 55,0 |
| Vest-Agder | 60 | 3 | 5,0 |

As the table shows, certain lumpfish populations, in particular those from the Trøndelag and Rogaland counties, have a high CLuCV prevalence.

In view of the disclosure provided herein, it will be appreciated that the present invention also encompasses the following **items:**
1. A nucleic acid, wherein the sequence of said nucleic acid comprises
   (a) at least one open reading frame (ORF) sequence selected from the group consisting of ORF-1, ORF-2, ORF-3, ORF-4 and ORF-5, or
   (b) a sequence complementary thereto;
   wherein
   ORF-1 is at least 80% identical to the nucleic acid sequence of SEQ ID NO:1,
   ORF-2 is at least 80% identical to the nucleic acid sequence of SEQ ID NO:2,
   ORF-3 is at least 80% identical to the nucleic acid sequence of SEQ ID NO:3,
   ORF-4 is at least 80% identical to the nucleic acid sequence of SEQ ID NO:4, and
   ORF-5 is at least 80% identical to the nucleic acid sequence of SEQ ID NO:5.
2. The nucleic acid of item 1, wherein
   ORF-1 is at least 85% identical to the nucleic acid sequence of SEQ ID NO:1,
   ORF-2 is at least 85% identical to the nucleic acid sequence of SEQ ID NO:2,
   ORF-3 is at least 85% identical to the nucleic acid sequence of SEQ ID NO:3,
   ORF-4 is at least 85% identical to the nucleic acid sequence of SEQ ID NO:4, and
   ORF-5 is at least 85% identical to the nucleic acid sequence of SEQ ID NO:5.
3. The nucleic acid of item 1 or 2, wherein
   ORF-1 is at least 90% identical to the nucleic acid sequence of SEQ ID NO:1,
   ORF-2 is at least 90% identical to the nucleic acid sequence of SEQ ID NO:2,
   ORF-3 is at least 90% identical to the nucleic acid sequence of SEQ ID NO:3,
   ORF-4 is at least 90% identical to the nucleic acid sequence of SEQ ID NO:4, and
   ORF-5 is at least 90% identical to the nucleic acid sequence of SEQ ID NO:5.
4. The nucleic acid of any one of items 1-3, wherein
   ORF-1 is at least 95% identical to the nucleic acid sequence of SEQ ID NO:1,
   ORF-2 is at least 95% identical to the nucleic acid sequence of SEQ ID NO:2,
   ORF-3 is at least 95% identical to the nucleic acid sequence of SEQ ID NO:3,
   ORF-4 is at least 95% identical to the nucleic acid sequence of SEQ ID NO:4, and
   ORF-5 is at least 95% identical to the nucleic acid sequence of SEQ ID NO:5.
5. The nucleic acid of any one of items 1-4, wherein
   ORF-1 is at least 98% identical to the nucleic acid sequence of SEQ ID NO:1,
   ORF-2 is at least 98% identical to the nucleic acid sequence of SEQ ID NO:2,
   ORF-3 is at least 98% identical to the nucleic acid sequence of SEQ ID NO:3,
   ORF-4 is at least 98% identical to the nucleic acid sequence of SEQ ID NO:4, and
   ORF-5 is at least 98% identical to the nucleic acid sequence of SEQ ID NO:5.
6. The nucleic acid of any one of items 1-5, wherein
   ORF-1 is at least 99% identical to the nucleic acid sequence of SEQ ID NO:1,
   ORF-2 is at least 99% identical to the nucleic acid sequence of SEQ ID NO:2,
   ORF-3 is at least 99% identical to the nucleic acid sequence of SEQ ID NO:3,
   ORF-4 is at least 99% identical to the nucleic acid sequence of SEQ ID NO:4, and
   ORF-5 is at least 99% identical to the nucleic acid sequence of SEQ ID NO:5.
7. The nucleic acid of any one of items 1-6, wherein
   ORF-1 is 100% identical to the nucleic acid sequence of SEQ ID NO:1,
   ORF-2 is 100% identical to the nucleic acid sequence of SEQ ID NO:2,
   ORF-3 is 100% identical to the nucleic acid sequence of SEQ ID NO:3,
   ORF-4 is 100% identical to the nucleic acid sequence of SEQ ID NO:4, and
   ORF-5 is 100% identical to the nucleic acid sequence of SEQ ID NO:5.
8. A nucleic acid, wherein the sequence of said nucleic acid is at least 80% identical to a corresponding sequence present within SEQ ID NO:6, preferably at least 85% identical, more preferably at least 90% identical, yet more preferably at least 95% identical, yet more preferably at least 98% identical, yet even more preferably at least 99% identical, and particularly preferably 100% identical.
9. A nucleic acid, wherein the sequence of said nucleic acid is at least 80% identical to a corresponding sequence present within a sequence that is complementary to SEQ ID NO:6, preferably at least 85% identical, more preferably at least 90% identical, yet more preferably at least 95% identical, yet more preferably at least 98% identical, yet even more preferably at least 99% identical, and particularly preferably 100% identical.
10. The nucleic acid of items 8 or 9, wherein the sequence of said nucleic acid comprises 200 nucleotides or less.
11. The nucleic acid of any one of items 8 to 10, wherein the sequence of said nucleic acid comprises at least 60 nucleotides.
12. The nucleic acid of item 11, wherein the sequence of said nucleic acid comprises at least 100 nucleotides, preferably at least 150 nucleotides.
13. The nucleic acid of item 8 or 9, wherein the sequence of said nucleic acid comprises at least 200 nucleotides.
14. Use of the nucleic acid of any one of items 1-13
   (a) as a hybridization probe;
   (b) for detecting the virus disclosed herein in a biological sample of a fish, particularly lumpfish;
   (c) in a method for detecting a virus that infects and is capable of killing fish, particularly lumpfish;
   (d) in a method for detecting a virus that infects and is capable of killing fish, particularly lumpfish, according to any of the corresponding methods disclosed herein;
   (e) for preparing a vaccine for protecting fish, particularly lumpsucker fish, against disease caused by Coronavirus, specifically Torovirus, infection; or
   (f) for preparing a vaccine for protecting fish, particularly lumpsucker fish, against disease caused by a virus disclosed herein, such as the virus of items 18-21 below.
15. The nucleic acid of item 1, wherein the sequence of said nucleic acid comprises at least ORF-1, ORF-2, ORF-3 and ORF-4, as defined in any of items 1 to 7, or a sequence that is complementary thereto.
16. The nucleic acid of item 1, wherein the sequence of said nucleic acid is at least 80% identical to the virus genome according to SEQ ID NO:6, preferably at least 85% identical, more preferably at least 90% identical, yet more preferably at least 95% identical, yet more preferably at least 98% identical, yet even more preferably at least 99% identical, and particularly preferably 100% identical.
17. The nucleic acid of item 1, wherein the sequence of said nucleic acid is at least 80% identical a sequence that is complementary to SEQ ID NO:6, preferably at least 85% identical, more preferably at least 90% identical, yet more preferably at least 95% identical, yet more preferably at least 98% identical, yet even more preferably at least 99% identical, and particularly preferably 100% identical.
18. A virus, in particular a virus that infects and is capable of killing lumpsucker fish (such as *Cyclopterus lumpus*), wherein the virus genome comprises the nucleic acid sequence of the nucleic acid of any one of items 1 to 13 and 15 to 17, wherein said nucleic acid sequence comprised in the virus genome contains the base uracil (U) instead of the base thymine (T).
19. The virus of item 18, wherein the infection of lumpsucker fish by the virus causes the following symptoms in the fish:
   (i) tissue damage in the intestine, and/or
   (ii) diarrhoea.
20. The virus of item 18 or item 19, wherein the virus is an enveloped virus.
21. The virus of any one of items 18 to 20, wherein the virus is a Coronavirus, preferably a Torovirus.
22. An oligonucleotide primer which comprises a sequence of at least 9 nucleotides, preferably of at least 12 nucleotides, more preferably of at least 15 nucleotides, and particularly preferably of at least 18 nucleotides, wherein said sequence is comprised within the genome of the virus of any one of items 18 to 21.
23. An oligonucleotide primer which comprises a sequence of at least 9 nucleotides, preferably of at least 12 nucleotides, more preferably of at least 15 nucleotides, and particularly preferably of at least 18 nucleotides, wherein said sequence is complementary to a nucleic acid sequence comprised within the genome of the virus of any one of items 18 to 21.
24. An oligonucleotide primer which comprises a sequence of at least 9 nucleotides, preferably of at least 12 nucleotides, more preferably of at least 15 nucleotides, and particularly preferably of at least 18 nucleotides, wherein said sequence is at least 80% identical to a sequence comprised within SEQ ID NO:6.
25. An oligonucleotide primer which comprises a sequence of at least 9 nucleotides, preferably of at least 12 nucleotides, more preferably of at least 15 nucleotides, and particularly preferably of at least 18 nucleotides, wherein said sequence is at least 80% identical to a sequence comprised within a sequence complementary to SEQ ID NO:6.
26. The oligonucleotide primer of item 24 or 25, wherein said percentage sequence identity is at least 85%, preferably at least 90%, more preferably at least 95%, yet more preferably at least 98%, yet even more preferably at least 99%, and particularly preferably 100%.
27. The oligonucleotide primer of any one of items 22 to 26, wherein said oligonucleotide primer is 9 to 60 nucleotides in length, preferably 12 to 40 nucleotides in length, more preferably 15 to 30 nucleotides in length, particularly preferably 18 to 25 nucleotides in length.
28. The oligonucleotide primer of any one of items 22 to 24, wherein said sequence comprised therein represents, or is complementary to, a portion of a reference nucleic acid sequence selected from the group consisting of SEQ ID NO:1, SEQ ID NO:2, SEQ ID NO:3, SEQ ID NO:4, SEQ ID NO:5 or SEQ ID NO:6.
29. An oligonucleotide primer which comprises at least 9 nucleotides, preferably at least 12 nucleotides, more preferably at least 15 nucleotides, and particularly preferably at least 18 nucleotides, of a sequence which is at least 80% identical to a sequence which is, or which is complementary to, a sequence selected from the group consisting of SEQ ID NO:12, SEQ ID NO:13, SEQ ID NO:14, SEQ ID NO:15, SEQ ID NO:16, SEQ ID NO:17, SEQ ID NO:18, SEQ ID NO:19, and SEQ ID NO: 20.
30. Use of at least one oligonucleotide primer in a method of detecting the virus of any one of items 18 to 21, wherein the at least one primer comprises a sequence of at least 9 nucleotides, preferably of at least 12 nucleotides, more preferably of at least 15 nucleotides, and particularly preferably of at least 18 nucleotides, and wherein said sequence is complementary to a nucleic acid sequence which is comprised within the genome of said virus.
31. Use of at least one nucleic acid of any one of items 1 to 13 or 15 to 17, or of at least one oligonucleotide primer of any one of items 22 to 30 in a method of detecting a fish virus, optionally wherein the virus infects and is capable of killing lumpsucker fish.
32. The use of item 31, wherein said nucleic acid, or said oligonucleotide primer is used in a method of detecting the virus of any one of items 18 to 21.
33. A method for detecting a virus that infects and is capable of killing fish, in particular lumpsucker fish, comprising the steps of:
   (a) contacting a nucleic acid extracted from a biological sample of a fish with at least one oligonucleotide primer to form a mixture, wherein the at least one oligonucleotide primer is complementary to a nucleic acid sequence which is comprised within the genome of the virus of any one of items 18 to 21,
   (b) determining whether upon subjecting the mixture of a) to amplification an amplification product is present, wherein the presence of amplification product indicates the presence of RNA associated with the virus, and hence the presence of the virus in the biological sample.
34. The method of item 33, wherein the oligonucleotide primer is an oligonucleotide primer of any one of items 22 to 30.
35. The method of items 33 or 34, wherein the nucleic acid in step (a) of the method, for example RNA, is extracted from biological samples by using solid-phase extraction, e.g., on-column purification using a solid phase of silica gel membrane.
36. The method of any one of items 33 to 34, wherein the nucleic acid in step (a) of the method, for example RNA, is extracted from biological samples by using phenol/chloroform extraction.
37. A method for detecting a virus that infects and is capable of killing fish, in particular lumpsucker fish, comprising the steps of:
   (a) sequencing a nucleic acid extracted from a biological sample of a fish, and
   (b) comparing the resulting nucleic acid sequence with a nucleic acid sequence which is, or which is complementary to, a reference sequence selected from the group consisting of SEQ ID NO:1, SEQ ID NO:2, SEQ ID NO:3, SEQ ID NO:4, SEQ ID NO:5 and SEQ ID NO:6, wherein an at least 80% sequence identity between the two sequences indicates the presence of the virus in the biological sample.
38. The method of item 37, wherein the percentage sequence identity between the two sequences that indicates the presence of the virus in the biological sample is at least 85%, preferably at least 90%, more preferably at least 95%, yet more preferably at least 98%, yet even more preferably at least 99%, and particularly preferably 100%.
39. An antibody that binds a polypeptide, wherein the polypeptide is encoded by a nucleic acid sequence which is comprised within the genome of the virus of any one of items 18 to 21, or is a polypeptide encoded by a nucleic acid of any one of items 1-13 and 15-17.
40. An antibody that binds a polypeptide selected from the group consisting of:
   (i) a polypeptide comprising an amino acid sequence which is at least 80%, preferably at least 85%, more preferably at least 90%, yet more preferably at least 95%, yet even more preferably at least 98%, particularly preferably at least 99%, or even 100% identical to, SEQ ID NO:7;
   (ii) a polypeptide comprising an amino acid sequence which is at least 80%, preferably at least 85%, more preferably at least 90%, yet more preferably at least 95%, yet even more preferably at least 98%, particularly preferably at least 99%, or even 100% identical to, SEQ ID NO:8;
   (iii) a polypeptide comprising an amino acid sequence which is at least 80%, preferably at least 85%, more preferably at least 90%, yet more preferably at least 95%, yet even more preferably at least 98%, particularly preferably at least 99%, or even 100% identical to, SEQ ID NO:9;
   (iv) a polypeptide comprising an amino acid sequence which is at least 80%, preferably at least 85%, more preferably at least 90%, yet more preferably at least 95%, yet even more preferably at least 98%, particularly preferably at least 99%, or even 100% identical to, SEQ ID NO:10; and
   (v) a polypeptide comprising an amino acid sequence which is at least 80%, preferably at least 85%, more preferably at least 90%, yet more preferably at least 95%, yet even more preferably at least 98%, particularly preferably at least 99%, or even 100% identical to, SEQ ID NO:11.
41. A kit for detecting a virus in a biological sample from fish, wherein the kit comprises a nucleic acid of any one of items 1-13 or 15 to 17, an oligonucleotide primer of any one of items 22 to 30 and/or an antibody of item 39 or item 40.
42. The kit of item 42, wherein the kit is suitable to conduct, or is for use for conducting, a real-time RT-PCR assay.
43. The antibody of item 39 or item 40 for use in treating fish infected with a virus, in particular lumpsucker fish.
44. The antibody of item 43, wherein the virus is the virus of any one of items 18 to 21.
45. Use of the virus of any one of items 18 to 21 for producing a vaccine.
46. A vaccine comprising:
   (i) a nucleic acid of a sequence which is comprised within the genome of the virus of any one of items 18 to 21;
   (ii) a nucleic acid of any one of items 1 to 13 or 15 to 17;
   (iii) a viral polypeptide encoded by a nucleic acid sequence comprised within the genome of the virus of any one of items 18 to 21;
   (iv) a viral polypeptide encoded by a nucleic acid of any one of items 1 to 13 or 15 to 17; or
   (v) a virus of any one of items 18 to 21.
47. The vaccine of item 46, wherein the sequence of the nucleic acid is the sequence of the nucleic acid referred to in any one of items 1 to 13 or 17, wherein said nucleic acid sequence contains the base uracil (U) instead of the base thymine (T).
48. An interfering RNA (iRNA) molecule for use in treating fish infected with a virus, particularly lumpsucker fish, wherein the iRNA molecule comprises at least 12 (preferably contiguous) nucleotides of, or complementary to, a nucleic acid sequence comprised within the genome of the virus of any one of items 18 to 21.
49. An interfering RNA (iRNA) molecule for use in treating fish infected with a virus, particularly lumpsucker fish, wherein the iRNA molecule comprises at least 12 (preferably contiguous) nucleotides of, or complimentary to, a nucleic acid sequence which is at least 80%, preferably at least 85%, more preferably at least 90%, even more preferably at least 95%, yet even more preferably 98%, particularly preferably 99%, or even 100% identical to the sequence of the virus genome according to SEQ ID NO:6 (CLuCV).
50. The interfering RNA of item 48 or item 49, wherein said iRNA molecule comprises at least 15 and more preferably at least 18 of said (preferably contiguous) nucleotides.

Those skilled in the art will recognize, or be able to ascertain using no more than routine experimentation and/or common general knowledge, numerous equivalents to the specific aspects, items and embodiments disclosed herein both in the Examples and in the body of the entire patent description. Such equivalents are considered to be within the scope of this invention and are intended to be encompassed by the following claims, or any claims that may be pursued based on the present disclosure.

## Claims

1. A nucleic acid, wherein the sequence of said nucleic acid comprises at least one open reading frame (ORF) sequence selected from the group consisting of ORF-1, ORF-2, ORF-3, ORF-4 and ORF-5; wherein
ORF-1 is at least 80% identical to the nucleic acid sequence of SEQ ID NO:1,
ORF-2 is at least 80% identical to the nucleic acid sequence of SEQ ID NO:2,
ORF-3 is at least 80% identical to the nucleic acid sequence of SEQ ID NO:3,
ORF-4 is at least 80% identical to the nucleic acid sequence of SEQ ID NO:4, and
ORF-5 is at least 80% identical to the nucleic acid sequence of SEQ ID NO:5.

2. The nucleic acid of claim 1, wherein
(a) the sequence of said nucleic acid comprises at least ORF-1, ORF-2, ORF-3 and ORF-4, as defined in claim 1; and/or
(b) the sequence of said nucleic acid is at least 80% identical to the virus genome according to SEQ ID NO:6.

3. A nucleic acid, wherein the sequence of said nucleic acid is complementary to the sequence of the nucleic acid claimed in claim 1 or 2.

4. A virus that infects and is capable of killing lumpsucker fish *(Cyclopterus lumpus),* wherein the virus genome comprises the sequence of the nucleic acid of any one of claims 1 to 3, wherein said nucleic acid sequence contains the base uracil (U) instead of the base thymine (T).

5. The virus of claim 4, wherein the infection of lumpsucker fish by the virus causes the following symptoms in the fish:
(i) tissue damage in the intestine, and/or
(ii) diarrhoea.

6. The virus of claim 4 or claim 5, wherein
(a) the virus is an enveloped virus; and/or
(b) the virus is a Coronavirus, preferably a Torovirus.

7. An oligonucleotide primer which
(a) comprises a sequence of at least 9 nucleotides, wherein said sequence is complementary to a nucleic acid sequence which is comprised within the genome of the virus of any one of claims 4 to 6;
(b) comprises at least 9 consecutive nucleotides of a sequence which is, or which is complementary to, a portion of a reference nucleic acid sequence selected from the group consisting of SEQ ID NO:1, SEQ ID NO:2, SEQ ID NO:3, SEQ ID NO:4, SEQ ID NO:5 or SEQ ID NO:6; or
(c) comprises at least 9 nucleotides of a sequence which is at least 80% identical to a sequence which is, or which is complementary to, a sequence selected from the group consisting of SEQ ID NO:12, SEQ ID NO:13, SEQ ID NO:14, SEQ ID NO:15, SEQ ID NO:16, SEQ ID NO:17, SEQ ID NO:18, SEQ ID NO:19, and SEQ ID NO: 20.

8. Use of at least one oligonucleotide primer in a method of detecting the virus of any one of claims 4 to 6, wherein the at least one primer comprises a sequence of at least 9 nucleotides and wherein said sequence is complementary to a nucleic acid sequence which is comprised within the genome of said virus.

9. A method for detecting a virus that infects and is capable of killing fish, comprising the steps of:
(a) contacting a nucleic acid extracted from a biological sample of a fish with at least one oligonucleotide primer to form a mixture, wherein the at least one oligonucleotide primer is complementary to a nucleic acid sequence which is comprised within the genome of the virus of any one of claims 4 to 6,
(b) determining whether upon subjecting the mixture of a) to amplification an amplification product is present, wherein the presence of amplification product indicates the presence of RNA associated with the virus, and hence the presence of the virus in the biological sample,
wherein optionally the oligonucleotide primer is an oligonucleotide primer of claim 7.

10. A method for detecting a virus that infects and is capable of killing fish, comprising the steps of:
(a) sequencing a nucleic acid extracted from a biological sample of a fish, and
(b) comparing the resulting nucleic acid sequence with a nucleic acid sequence which is, or which is complementary to, a reference sequence selected from the group consisting of SEQ ID NO:1, SEQ ID NO:2, SEQ ID NO:3, SEQ ID NO:4, SEQ ID NO:5 and SEQ ID NO:6, wherein an at least 80% sequence identity between the two sequences indicates the presence of the virus in the biological sample.

11. An antibody that binds a polypeptide, wherein the polypeptide is encoded by a nucleic acid sequence which is comprised within the genome of the virus of any one of claims 4 to 6, optionally wherein the polypeptide is selected from the group consisting of:
(i) a polypeptide comprising an amino acid sequence which is at least 80% identical to SEQ ID NO:7;
(ii) a polypeptide comprising an amino acid sequence which is at least 80% identical to SEQ ID NO:8;
(iii) a polypeptide comprising an amino acid sequence which is at least 80% identical to SEQ ID NO:9;
(iv) a polypeptide comprising an amino acid sequence which is at least 80% identical to SEQ ID NO:10; and
(v) a polypeptide comprising an amino acid sequence which is at least 80% identical to SEQ ID NO:11.

12. A kit for detecting a virus in a biological sample from fish, wherein the kit comprises an oligonucleotide primer of claim 7 and/or an antibody of claim 11,
optionally wherein the kit is a real-time RT-PCR assay.

13. The antibody of claim 11 for use in treating fish infected with a virus of any one of claims 4 to 6.

14. Use of the virus of any one of claims 4 to 6 for producing a vaccine.

15. A vaccine comprising:
(i) a nucleic acid of a sequence which is comprised within the genome of the virus of any one of claims 4 to 6;
(ii) a nucleic acid of a sequence which is the sequence of the nucleic acid claimed in any one of claims 1 to 3;
(iii) a polypeptide encoded by a nucleic acid sequence which is comprised within the genome of the virus of any one of claims 4 to 6; or
(vi) a virus of any one of claims 4 to 6.

16. The vaccine of claim 15, wherein the sequence of the nucleic acid is the sequence of the nucleic acid claimed in any one of claims 1 to 3, wherein said nucleic acid sequence contains the base uracil (U) instead of the base thymine (T).

17. An interfering RNA (iRNA) molecule for use in treating fish infected with a virus, wherein the iRNA molecule comprises at least 12 nucleotides of, or complimentary to, a nucleic acid sequence comprised within the genome of the virus of any one of claims 4 to 6.


SEQUENCE LISTING

<110> Pharmaq Analytiq

<120> Novel Fish Coronavirus

<130> ZOE20235EP

<160> 20

<170> BiSSAP 1.3

<210> 1
<211> 17601
<212> DNA
<213> Viruses

<220>
<223> ORF-1 nucleotide sequence ("n" at positions 2490 to 2503, 2523,
      2524 and 9771, means any base)

<400> 1
atgaaaaaaa ttgaagatgc tttaggaaca cttaaacctg cctttaaggc acacatagac      60

tctctacctc ctttcatggg aaaactagcc acaatgctag ctgagacaag acgaggaaaa     120

actccccctc ttttgatcta tgtcatatca acaattttgg aaacgaatat tacagttcat     180

tatgtcagcc acacaataac ttcataccat tcttcaaacg cacacactca cactcatgaa     240

ttcgactcag aagattacac tcctgacaat cagattttaa ctaaagtcaa ccgacatggt     300

aatgacctta atcattcata tatccatggc gccaccaata tgtacaaccc tgtctaccag     360

agacacccac caaacatgtg ctacatgctt acgggactgt acatgttgtc aggcttacag     420

gaattgtatg ccatggctga agacaacttg acaacatgcc aaatcaacct tctcagatgt     480

ctgtttgatt tgaatcaaga tgaatttgat gtagattaca catttgtaat atacactcct     540

agcaaatctc aggagtgtgc cttcaaatat cttcaggaga tagtccacca ctgtgaactc     600

accattttta gacatacaac aacaagtgtc ttcagttgca acaaatgcaa ccatgtggaa     660

actgtcattt ctagttgttc tttgaatctt gtatatataa ctgattctat tgaaaaagca     720

tttcaaccca ctgtagaagc taataccgac tacatgtgtg aaaactgcgg tctacgcgac     780

cataaactta aaacaacagt aacaaatcca gacctccgtt tggcacaact aaactatcca     840

acggattcaa aatacactat ctttcttgat gaacaagctc cttttgtctt ccattccatt     900

gcaaaacacg ttggaactgc taattccgga cattggagtg cattgaatgt aaattcagat     960

atgttgtcag actctaacga gcgacaacat tattatacaa cacctagtat tgttttgctt    1020

gcatttcttc ctgaggaaga gctacagaac attagaaatt catcacctct tcaggaccat    1080

caaccagatg atgttgaaga cgttgaatct cccctaccag gttctatatt ttatactaca    1140

gatgacattt tttctactaa gagtctatcc atagctcact gtgtagcccg cgactttcac    1200

atgtccggcg gcatagcaaa aattttctca gataaattcg gctctaaaac tttcttgaaa    1260

tcacaaaacc ccgttatagg cggtttttcc attttactta gagagtgtcg tgacatgtac 1320

tacctcgtca caaaagagaa aacttcagat aagccgacat accaagacct taaaaactcc 1380

ttgggttcta tgacagagaa tttggttcgc aagaatcata atactctttc aattccatat 1440

ataggctgtg ggattgatgg tttacaatgg gcaaccgttg aaaaacaagt caaagaaatt 1500

gtctgcgctc gaggtattga tgtaacagtc caccacctcg aaaatgaagt taaacacact 1560

ccagaacaac aaacagcttc tgacaattca gtcaaattag ttcaaaaact ctttacagaa 1620

acacctcaag ctatacctat agttgttcct tcagatgact cagacagcga catagatgag 1680

tcagccgatg tctttttacc aggacctgaa tctgaatctg attcaaaatc agaatctggg 1740

tcagattacg actttaaatc ggcttctgaa ccagaagatg aattggagcc cactccaatc 1800

tctgaacttg aactaacacc agcgtcaagt ttaactgtag agtccgatga caatccagac 1860

acaagccagg aaacattacc agaatcaaac tctgaggaaa ccaaacctga gcaaacacct 1920

gacacaacat caaaggtgtc ctccgattcg aagcttgatc cacaatcaga attagaagaa 1980

gaactggcca acaaaccaga atcggcttct gaaccacaat ctgagactga atcgagttct 2040

gaatctgaag aggagcttga gccacaatca gaatcagagg aagaaccggc caacaaaccg 2100

gaatctcctt ctgaatcaca atctgaaaat ggatcgagtt ctgaacccga agaggagtct 2160

gaaaaaccat cggagtctgc tgaaacagca acagaggata gcccggaaac aacaccggaa 2220

acaaccttag agttaaccac acaactcaaa cctgcttcag aatctgacga caaaccggac 2280

acaccagcac catcaccttc accaattcaa ccagagaaaa acttggacac cacccctgaa 2340

caaacttcac aaccaaccac acaactggaa ttgacgttag aaacacagga acaaccagac 2400

actacaccag aagtgccatc tgtctcagaa gataaaccag acacacttga agaatcttct 2460

gaatcaacac cagaactctc agaattggan nnnnnnnnnn nnnccaccag taccaagacc 2520

tanngaacca gacctccaac acctagacca agatctgcac gtggagctag gactagatcc 2580

tgtgctggca cacctatacc agttattttt gatattatag ataatacaag ccagcctcaa 2640

gttccccttg acttcccaga agcactgcaa gaactgaaca aacctagtga agtaatccca 2700

gcggctagtg aaaaacctgt ggaaaaacaa ataatccata gttttgtaag tgtcgaaaca 2760

ccttgcaaac ccaaagccac taaagttacg aattatgtag ctgcacaatc taatgcaatt 2820

ctaaattgta ttaaggcttt cattcctagc aacccgcttt cactattcaa tagaaaacca 2880

gcttttagaa aaataatatt cactgaagac acttcagaac cagatagcga tgatgatgat 2940

tgtgaataca ctccaccaac atcaccattc cctgaacttc tggcattggt agatgaagac 3000

attgaagtag aacaaactca atctgtaatt ccaaaaacag actctgcttc aattgtggag 3060

gatcttaaaa aacaagaatc ctctactttg tcattggaca ccaacacatc gaaacctaca 3120

agctctccgc gaagacagcc tagggaagta gaaagtgttg atgaatccag tgatgactca 3180

tctaaaccaa aaacaatttc aacattagac aaacctgcta tgaatagtga cacgaaacct 3240

acagactctt cgcgaaaaga gcctctggaa gtaccagtta ctacatcttt aagcacccct 3300

gctaaaaacc aagataaaaa atcttcaaaa tctgcaaaag taataaaaga ctattctttg 3360

acccctaaca cagtcaaaca gcaagtctat tcactctacg gtgaatcagt agatgcagtt 3420

aaataccttg ttcaaacata cccagacagg gctaaacaaa cagctggtat tgcttatttc 3480

cttataacta cttatttaat atggaccatc ggtctcatag gagtaccaat ggcctttaaa 3540

ataccaatgt ttttatgtct tttataccaa gttaatggat taaatatagc accatttgtt 3600

actaaccaaa agttacaata tgttgcattt ccactttggt ataagctcta tgaagtaata 3660

tcagtccgtt ttgtggcgaa tatagcacaa tttattgtta aaacaccacc tatagatgtt 3720

ttaaacaagc taattcgttc taataaagac aagccagtca aattaacacc aaataaacat 3780

actttgatgt taattcatga cttagcttta gagtctgtcg acggaaaaga aaaccgctat 3840

tataatactg atgttacaac tttcacaaaa aggcatagca cttctaatat ttcatatgtt 3900

ctaaagtcta ctttaatcaa atatgtcatg gaccattgtt atgtaaatat tgcagttttt 3960

actttagtta gatacttaac tttattagtg tttattcaac atttctctaa tccttatgtt 4020

cttgaagcaa atagccaatc acataccgtt ttacagtatc tgttttcaca cttgagacca 4080

tttggaaggc ctttgtgccc aaccctcaat gactacatga cgacagcaac accacgcgat 4140

gcacatgtac aagcaggttc tcacttcagt gaattttgtg ttcctattca ttatacaaca 4200

ccaatcatta aaagcacaat ggcagaacca tcactttttc tactttttaa cccagtctta 4260

tggcctttgg ttatggttgt atatttttat cctccaatga tgtttatagc aaatgcagtc 4320

gcttattcat gccttccttt agtggtcttg ttacaatggc tttatgccat gtggttttct 4380

tgtacatgct atggcaccaa aagatgtgcc aagcatttgc ataaaaatga agtggttaaa 4440

ccaatggaat ccacttcaac taagaaccgc atgacattta ctccatcaac gaccttttgt 4500

agtaaacata acttcttctg tccagatgca ccacatataa tgactcttgc aatggctagg 4560

caacttacaa actactacaa tttgacagat acagtaatac ctgacatcca ggaatactcc 4620

cacgagaacc ctactgtaca atttattcac tttgatccac ttaaacacgg tgccgacaca 4680

attttggaac caattacaag cgctagcgcc agttcaattg ttgcatggta ctctctcctc 4740

tttaatcaaa agtttgtcct ttcacattat agctacagaa ccccagtagc cgtagttgac 4800

aaaccagagg aaacagatgg tgatgataca aaatcattag catctgacac ttctgataac 4860

tttgagtcta ttagaaagac caaccataag aatcagagca aacaacagtt taggccaaac 4920

ggtcaccaaa gaccaagtaa gactttcaaa cgccattcaa gaataatgac atctgaacag 4980

aagaacagct taattgaaac ttttaagggt ttaacaaatg gcacagcagc catcccacag 5040

cctttaatca tttttatttg ggttatcctt atggtaatac caacactctt tttagtcgcc 5100

agttccagca gaacagctgc aacaatgcct ttaaaccgct actcaggcgt caaccccact 5160

ggaattatgt ttcaccaagc acctccttac atccattcgg aaccaccaaa ggaaacttac 5220

tacaaactca gttatcctta tccgtcagca acagttgtga gaaccttgaa aggccatctc 5280

tattaccata gcgatgatac cgttcaacaa aattgtacca tgcaatattc acttatagct 5340

gcttctacaa agcacgtgtg tggcaaggta gtttacacta taccagccca tgtctcaatt 5400

ggctcactta aactgttgct tgtccacccg gatcaaacaa atttaccatt tgaactacca 5460

gtttcagatg aagtccgtct ttgctacctc acaaccttga acgcaccaag atgcatgccc 5520

tctcaactag ccatgtcaaa taaccaattt gccgctgtaa gccttgtttt gttaataaca 5580

ttagtttctt taattaaagt ttatataatg ttttttactg tttttaaaca ctacacaaca 5640

actgttttta tacttgtagc tgtgactact atcacaatgt tggtgtcctt cttagctcct 5700

ccacttctca tagtcgttct tctttcacta gcatggctat ggtacggcaa tacaattgta 5760

ttgtgccata tcatgctttt gatagtctta gtcgtctcat ggaaagtggc tgctgtctgt 5820

ttcatcttcg ccttattgta ctttggaaaa tgtgctatgc ttagcaagaa cattaaatac 5880

gtacagggtg gagttaaatt ttcaggaacc tttgaagaaa tagctcagtc aaccttcttc 5940

attaactacg gagtagcttg tcagcttctg gaacatactg gacagacaat tgaggatata 6000

atgcaactta gaaccgcggg tggagcccca gcaaggcttg cgcgctcaat atacgattgc 6060

ttttccacaa atgcctctgt cttgtacagt cccaggtcat tttcaccaca gtcacttata 6120

acaaaatatt tatacccagg ttcgatccct gtcggcagag cccctgtctt attaggcaaa 6180

atctccggca tgacttgctt agggcgtgaa cagtccacct gtttccaatc atcagctaca 6240

accattacta cgtgtaccca tgctgtaaat actgctggaa cattcatgtc tcaaattaaa 6300

tgtgttatag ataataaaat atatacagtt caacccgaga atataaccat aaccggaatg 6360

aaagctacat ttgaagttga aggactacct ccattcacca acgatgtaac agtggcccca 6420

aagccgctga agcattacat ggatggaaag agacaccttg ttctctacac taaaagtgag 6480

agcatagtct actcttcaat aatgtggccg actgaaaacg gtttattctc gtcatcagtt 6540

tctgacccag gagattcagg tgcaccctac ttttcagaca atgtcatagt aggaatacac 6600

caaggtcgca acgaagcaac caacaatcct gccattttag caagtggtat ggatggtgag 6660

tctccctgtg taggttacga tgaccaatca tatggccttc cacttcaaga atatttcact 6720

cacattgtct tatcaaataa gccaagtgac tttggtgctc catctaacgt ggcgccaaat 6780

aaatactaca acaaaaaatc atttgaacaa ttagctgacg aagataagac ttatttaaat 6840

agtttatcat atcccctgtc ctcatctaat tattgttact ttaatagctt caaaacccaa 6900

tcaagcacaa caatgctcga caacgctgaa gttattaaat atgtagtttt gcttctcatg 6960

atcttggatt atttcttttc aatcatttgc gaagatgctt taaacccagc atcttacgct 7020

atgttagtta tcgttttggt tcaggctttt attacaaaaa ttacagtttt cagaacaggt 7080

atctatatcc aggcagccgt ttttcaagca tttattgtac ctatagtcag tcaaattaca 7140

ttgatactgg ctgcagatac tgcaagaagt tttttaacgt tccacttttt tgtacttgct 7200

gttttgacat atttcgttct ttgccgtatt gctgtagatt tttggcgttc catgtttttg 7260

ctatttttga caagcgtctt tgcaaccatc atatggacta caaagaatga cttcaatatt 7320

ttacatgaaa ctggcgtggt tctaacaccc acggcagaat tagctcttat agtagctttt 7380

acttatataa tttatgcttc atgtatgtta acacctgtac cactgtatac tatttgtgtt 7440

ttcttttcat ttttatcaaa tgctccactc taccttgccg tcctctcatt cggcattcta 7500

gtttctttca aaacaaacca agattttgga cgtctagtgg ataaagtgtt ttctttaaat 7560

atgctctatg aataccatgc ttaccaaaac tatgttattc aaaactcagg tcaacaccca 7620

ggattttaca ggtcactctt tgcttttttt atcaatttga ccacccaacc aaaaacaaca 7680

tacaaatgtt tcaaacccca gacagcaagt ggttacagag taatatatca aactcccact 7740

acagagttca ataaatctct gcaacatgcc agtatcacaa aagatgacaa ctccaaccat 7800

ataattatgt ttgctgacgg ctcatctgat aatctcaatt gggcaaaaga aatggtcgca 7860

accattcatc taaccaaccc aaatttgcag ccactcatca ttggatacta ccacaactcc 7920

atggacgtca taaccaaggg aacttacatg caacatgaat tcataaaaat gccagctgtt 7980

atcttaactc aagatcctct aactgaacca atcagtcatt tagcagcagc agcatttact 8040

tcaatttctg gaaaacctca ggcacagaaa aacaacgttg tttcaaactc caaagcgcgc 8100

ataaacacag ccgttcacga cgctgtcgaa agcgtttatt caggagaaac atacgttgcc 8160

cccaaaccta tagtctcagg aaaaactgtt gtagagaaac cattctctac aaccgaaacc 8220

accatgtaca taatgcgtgg tttacccggt tctggaaaat ctttcaaagt tagtcaatta 8280

gttgctaaag atccaaattt agtcgtagct tccgcagacc actttagata ttcaaatgac 8340

aaaactggaa aagccgtata cacctacatt ccagaagcaa ctagttctgt acatttacaa 8400

tgtcagaata gagcccgcaa agctctagaa aacggccaat ctgtgtgcat tgataataca 8460

aatctaacac tcttagaaat gagaccttac gtcttattag cccgttcttt taactataac 8520

attgaattca tacactcaga ctctccctgg gccttaaacc ttgacctgtt acatgctaaa 8580

ggtgtacata atgttcctag agcaaagctc gtaatcatgt atgatagatt ctttgaccgt 8640

gataatcaaa tcgatgcaga cagtcttata cagtatgtta ttgaagcaat tgatccaaaa 8700

cttgttgctc caatcatgaa ccgtttccct gccgactgtg atcttatcct tcaatctgcc 8760

ctaacaccag accttgaagt attaaagcaa aactacgaca gagcaaacgc aacataccaa 8820

gatgtttctt tagatgatcc tccggcttta aaggcagcac gtcgtgctat gaatatagct 8880

aaatctgaat atgaggcagg cgaagcaggc cagcgtcgca ttgagaaatt tttagaaaga 8940

caggatgtag cagcactcaa ccaaacgctc acaactgtca atcaatctaa attcatagca 9000

gcgatccgtt ccatctacct aagcaccatt agcaatttga gactaaaaac ccgtcatatg 9060

ggtgaaggat catatgcagt tacatcaggt actaatacta ccgataaagt tttagttaac 9120

acgccacaac gtatgactag aattgaagat ggcatttata agcttgttgc aaacggtttt 9180

gaaatcacaa tgtgcgacgg cagcaactta gccggtgtta cttttgaaca ggatataaat 9240

cctagcatgt acccttttgt ttttacatta atgtcaaata tagctgtacc tgttttaacc 9300

cgccaagcaa atgttggcta tcttgatatg tcaaataaat tcatctgtaa agatggcact 9360

gttcaatttc aaggtgtcat ctatgcctac cacactccat caaatgagag tgctgacttc 9420

aaagtaggca ataccagttg gaccctccag aaaaacatca atttgactgc ttttattcct 9480

gcaattcata aaactgcaac cttcgcagca caatcagtgt tcttaggagg actacccatg 9540

gaagagcacc aagccttttc cgacacaccc acagcctcaa acaaatttaa agtttttgtt 9600

tcatccacag tctgcgcctc aaccgtgtgc aaagtaaatc ataaaactta tgtacagata 9660

ccagatgaca ttcaagatcc ttttacatat atgcatcaca gcgtttgttc acacaacaaa 9720

tttttatcaa accatgaaac cagatgtcaa atctgtcctt taaactgtta nagcgcaaat 9780

ccgtgtgtgt ctacggcttg cgctctattt gataatggca cattacctcg gtcaacacat 9840

tatattaatg ttagcaccac ttcaaatgtt ggcttgttta aggcagtaaa gaagtctact 9900

cgtcaactaa acattgacgg ttttccttac atgctaaagc aggttaaaga cgactcagaa 9960

cttgtaagtt ctcttaaaat aggtctacct aatatcctcc cacatcacat ggtggaaact 10020

aagtcaaaaa cataccttct taggggcccc acaacggctt actcacttgg cgatttatgc 10080

tacgcactct ttaatggcga ctttgattat attcgcgaaa atataaactc tgatttcgtt 10140

ttggaccgtg aagccggaat gcctgataca gaaacacgta cgtggctgtt cagcatttta 10200

aactttgcag tacctagagt gtgtgctata attgaccaga tgatttctga gaacgtcttc 10260

tataaactga ctttggataa cttagatcta tacggatcac tctatgattt tgacgactat 10320

cctactgaag gctttaacag gcctgatgat gtgatacgta tgttaaagga gatatggtcc 10380

ttctgtagac gtccactacc tgccgacctt cttaaatacc atgaagacat cggtgcagca 10440

gccactcaag aaatattgct gcatgcaccc ttcattgata aagtttgtgc tctaaatgac 10500

agattagctg ttgttgataa tagagcaagt caatactttt tctgtgaaga agaaggtgtc 10560

tttacccata tttacaatcc agtctacgga actttagcat tcgataacaa gttgatccaa 10620

tcaaaggatc cttcatgtac attacagcgc ctcattacta tacaaggccc tttgtctacg 10680

aatgctagtc ccgtgatctc tatttctgat tccactcata ttgccaacaa tattaatcca 10740

tctaaccaaa agacaacacc gttgtactac gatttggaac ttgcgcaaga attcattgac 10800

gcaggtttaa atattgatgg cgtttccaac tacttcttct atggaccgtc tagagcgggt 10860

gtagtgtctg atttcttact atatgaattc caaggaactc aatggtttga caataacatg 10920

ctgcgctctc tttattcttt catattgaag aattcagagt gttacagaac aacagatcaa 10980

ctggacttta gaggtggaaa accccgtaaa tcctcaatgg gacatggtgt tactggcttt 11040

aagcaagacg tcgtgtacgc tgctttaggc cctgatatga ttgaaacctt gtatgaaacg 11100

gcaaaacaaa caccattgcc gttttgtaca aaaataactg ccaagtatgc attaacagca 11160

aagcctagag ctcgtacagt tgcagcatgc tcctttgtag cctcaactat ttttaggtac 11220

gctcacaagc ctctaactaa taatatggtc tcaaaagcac agcagggttt gggttattgt 11280

ttaattggaa tttctaaatt ccacggtcga tttaataaat ttgttaagtc tagggtaggc 11340

actgtcgaag actttaatgt tttcggtagt gactacacta aatgtgaccg tacatttccc 11400

ttagctttgc gtgctctttc agctgccctt attttcgatc ttggcggcca tgacccagac 11460

aactgtcttt ttattaacga gcttaatgca tacatgctag acattgtttc agtcgaagac 11520

tcctttgcaa ataaaccagg aggtacttca tcaggagatg ccactacagc atactccaac 11580

actctgtata actttgcagt ccactatatt atcatgtgga aaacattctt gacagtcaat 11640

gacccttcta ccaaggtcat acgcagtgca gctcatcacg ccctaacaag tggtgacttc 11700

tctatgtaca atgacatgat acaagacatg ttggatgtag actatacact caacttcctc 11760

tctgacgatt catacatctg ttcaaaacca agcgcttttc cgatctttac gctcgagaac 11820

tatccttcta aactgcagtc tatactccac acagcagtag atagcaaaaa atcctgggaa 11880

gcaaagggtg agattaaaga attctgttcc tctcacatag tcaacgttga cggcgactac 11940

cactttaaac cagaaaagga tagaatattg gcttcattgc tgatattatc gaaaatcgct 12000

gacatggaca tcttctttat gaggttcgtt gcgttattgg ctgaatccgc cgtatatata 12060

cgcatcgatc ctacattttg gctggccctg tttggtgttt tcgaaaaccg cgtaacagcg 12120

tttaaatctg aaacattgct ctcacctgtt cctgaacaac tcatgaaggt ggctttttat 12180

gaatcgcttg tctttgccga cgtggatgct acagccttat atggtttcct tgatggtttt 12240

aaaatgcaaa gtcaaactct ccacccagac ggtgttgagg gttttgacaa gcaaagtgac 12300

cgagtaaaac actgttttgc ttgtgacaat atatcagttg gacactgttc gatttgtccc 12360

gttccccttc ctttgtgctc tttttgcttc tatgagcatg ctctgctcaa tgaacattat 12420

gaagcttctg gaattgcgtg tgaatgcgga gacgctgaca ttagacaact tcacttaaaa 12480

ataaccaatc aaccatcctc gcacaatttt atctgtgctg aatgtcccac tgtagctatg 12540

aagctgccaa tcttcaactc tttccaagga aaagtactgc ttccaatgtt ccgtatgaat 12600

acgccattgc cttcctcagt ctctgtaatt gttgatgtac gttccaaccc aaaagcacct 12660

aagatgctgt gggacgacgt ccagaatttc agagaaaatt gtactaggat agcatacgaa 12720

tccgtttcgt gtgctgaact agctagggag gtggtttact atccatatga agtgattgaa 12780

tccaaagcag gtcaagcacg acttagaata cagaacttta aatgttcacc aactacttat 12840

gttcagttct acaaagtccg tcaaaatgga aagtattgtc tagtagccaa agcaactcta 12900

acgccggctt ttgaaaacca aacagacatt ttctccgttt ttcaaccaaa caacttttca 12960

ccttggaata catcatcagt gtttgcagta gaacaatacg ctgcaatata ccctcccata 13020

ccaaaggaac cagtcaatgc tacgttcgtc ttaggacctc caggctgtgg taaaacatac 13080

tacatagcca aaacgtactt ttcacaggct tctgagacat gtccggtcgt atactgcgca 13140

cctactcaca gattagtttt agatatggac gcagaatata gtggtgtagt ttcaaaatct 13200

ctctacaata atagagtgta caaaaatcca gcctacaaaa caggcgaacc attcaaatta 13260

tgtttcacca cacacaacac gatgccagtt caaaagaaag cgatcctcat tatagatgaa 13320

gtgtctttaa ttacacccca ctctctattt tcgatcattg gtaaagggtt ctatgagata 13380

gtactcgtag gagacccttt tcagctctcg gctgtttttc caggttttgt tgtcaatcac 13440

acatatgacg ggttttacat ccgccggcta gtaaataagg tcaaacacct aacagtttgt 13500

taccgttgtc cacaagaaat cttggacata ttttctaagc cctatcatga tgttgggatt 13560

gacctcacaa ccggaaatac caatccagga aaggcatcca tttatacact aaattggctt 13620

caagcagatg taggtactaa aaatccggac aaactcagac aactctttgc gcaatatcca 13680

ggctttaaaa ttatcaccaa ctacagatgt gttgttgatg cagctaaaag ttacggtatt 13740

aacgtcgaaa ccatcgactc atcccaagga accaccggag ataggcatct ggtggtaatt 13800

tgcggcagta ccaacttttc taaactttta aacaggttta tagtagcagc ctctcgttca 13860

acaactgaac tagttatagt catgttgcca gagctttaca actatttaac agagacgttt 13920

aacttcaaac cgttacaatt gcaaaatgtg catgtaccga tcgcagtatc ttctacagca 13980

ttctgcgata tagaatttta tcactttcaa aagaagttct atgttggtga aataagcgta 14040

agcacaagta ccactatgac atgtcagttg ggttgttata ttaatggctc ctacatgctc 14100

ccacctgtgc ttgaaaactc tgaagaccgt ctctacgttc cttctagatg gagacgtatg 14160

ataagaaaat accctactga atctatgcac atttccttac tggacagact tctgaggcac 14220

attttattaa caactactgg agaaattcat ttcgtaatgt tctctgcaga caatgatctc 14280

attgcactgg atccgtactt tataccaccc actctatgtg agtgtggtag tgcgggtcta 14340

gtggaagtag acatcactgt tttctgccgc aattgtttgc ctaaagatgg taaagccact 14400

cgtttggtaa aaccgtctac actagatgtc cagactgaaa aactcagact tgcaaaagtt 14460

catgctaagg tttgtaaaat caagcatggc agtgctcaca acgctgatgt tgatgctatt 14520

atgactcaat gtatatatgc taatagctta acattcacac caacaaccca actagttgtt 14580

aacactgatg agttcacctt ttacatgcta cctaggccgt caaaccgtca tttgagaatc 14640

attcataaga acgacaaacg tttctatgct atcactcatg aagaagaaga tctcttcttt 14700

actaacatct cagcagtggt agacccaatt cctgcaaaat tcaacattgc acactctaca 14760

agcttcctca ccatcaaaag tggttgcgca ggtaataaga cttgtaccag atgctattat 14820

ttacacttag catacacgga atttgtttct caacacaagt atgaaccatt cacttgtgtg 14880

tcttttaaga tacggtttga cttttcacaa ttcactgact cagtagatac tttcctccga 14940

caaggcttaa taacctttca tccggagatg aattcactgc aaaaatcact tttattagca 15000

gtggataagg tctattgtga taacttcacc tcaaacggta gaaggtttag actttacgac 15060

aacaatttgg ttaaatccat aatcaaaggt tcagtggctc aaaactccat catcatgcca 15120

ctcgactcag ttttacacgg gttgaacatt gatttcacag tcggatgtgc cgtagataac 15180

ttttcctgca aagaagcagc gagtgttagg tactcagaag tagtactttc catcaccaag 15240

ttgcccccag gcacttgcca gttatactac gtcatatctt acggcctgaa ctctcccaag 15300

accacttatg ctggtcacca attgttcgac ggctttgaga ctgttattgt tgttaatcgt 15360

aaagataaac ccccttacgt cctcacacag tatattaatg atgttgtagt tgcaatgcca 15420

gagtccctct tttcaacagg tcgattctac agagaaaaac catatcccgt ccttatgaac 15480

gaggatttaa gtggcttaaa ccatcacatt ttctctggtg actatacaga cgaatctctt 15540

acattaggag gtgtccatca tatagtaact ttaaacacct atgaccacaa gctcaactat 15600

atccaaacga aagctacatg tgccgcctca gtttcaactg gcggacgtgg tcataaaatt 15660

actacactgt ttgacgttca tgcaaatcaa cttgctgatg aaattaccag agttacatct 15720

gttgttacaa cacagtctaa agttattaat ttgacaatag attatcagca agttccttgt 15780

atgtactggt cttcaccgac cggcataaga accttctacc ctcaggctgt tagactggac 15840

gcaaagttta taccatacta cgtagaatat cccaatattc taccggcagt tgttgaagac 15900

caggtgtacg atttgtctaa ttacaatcaa ccacctttag gccaaaactg ccctgtaaac 15960

tttcacaagt acgtccagct aactcacttt attttagatc atgtgaaaat ccccgaaaag 16020

ggtttgatat atcatatcgg tgcagcaggt actaagcaat gttcacctgg agacttaata 16080

ttggaacaat ttttcaataa atccatcata tactcaagtg accttcttcc ttaccaatca 16140

cctgctgtgc aggttgcatt ggatgtaagg ttttcggctt cactcatcat ttcagactgc 16200

tattcgaaag aaccgcagcc tgatttgttg agtaagttga ttaacaaact agtgtatggt 16260

ggaactctca tttttaagac caccgagact ttcacatgtg acccagcctt ttatgttgct 16320

cattttaact gtataaagtt ttttactgcc gctgttaatc actcatcatc agaagtttat 16380

attgcgttca tcggaaaact ccctaaacca aacaacaact ttttagcctc agactatttc 16440

cagagattaa ctcaacatag aaataaagta gttaaacagc cttacgctca cacatgggac 16500

acatctttta cgtacccata cccctcaaat gttcttcaag ttagtcgtaa aaacctttta 16560

tatctatttg aaaccagagg agctgcagta ggtactttga tttttgaaga accatcaaaa 16620

cctgctgtaa agatacctac aaagtgtcaa accacacaac cctcgtgtgt cattgaggtt 16680

ggtaaccaat acgattgttg cattcaagac atcattaccc tcctcaatgg aaaatccttc 16740

acagtgaagg tgcccaactc agaatcctta ctgcgcgata tctgcacact tgcgcttagc 16800

cagagttatt ccatcaatat tcgcggaaaa acactctaca cccttagttc cctacttaga 16860

attaggcaac aatccttact gttttacgga gagaaggtca aaaaccctcg accccgtaat 16920

gtcttgaaca aatataccaa ctacctcaag gcaaaagtga ttaggcatta caccaagcct 16980

caatcaacag ttttggacat tggtacagga aaaggacaag atttgagaaa atactcgtta 17040

gcaggggtta aatccctcac ttgtgtcgag cctagtcccg agtctgtgac tgaactttca 17100

ataatagcta gtccccttga tatggagaca cacacagtta tgagttctgc ccagaaattc 17160

gagacctcgc tgacgtttga cttggctttc tctttctttg ccttgcacta tgcattggat 17220

gacgtttgta tgtctgaaac actcaacaat gttttttgca aacttaacag taattcacag 17280

ttgatcttag tagttccaaa tgctggcagg atgcaatcca taccttccct tggtttaaca 17340

gtcactcatc tagatgatga taaagtttgg tttaaatact cagactatat agactgcgaa 17400

gaaccgttag tagacaaaga aaaactactt acgtgtttag ctacatatgg aacaattgtt 17460

actgactcac cattctatga cggtgcaaac aaaatcctag accaaaaatg ctcatccatg 17520

tatagagcat cgacagccca tctaaatccc gatgaaattc aatatattaa tatgtatgat 17580

ttaattgttg tcattaagaa t 17601

<210> 2
<211> 4173
<212> DNA
<213> Viruses

<220>
<223> ORF-2 nucleotide sequence

<400> 2
   atgttcgcgc tcgttctaac cctcacaata gcttcggcta ttgcccaaga tttccccgca 60
   tatgacccgt gtcctacttg ctcaaccccc ggtaataaaa taccggctcc gagcacagtt 120
   gcccagtatt caacaaacta cggtgcgaac ttctttaccg tagtctttga tggtattatc 180
   ttcaaccaat ttagggagag ttattaccac caatgtagac caacacctga atactgccca 240
   gatgcaatca attgcgcctt aaacagaaca ggcgcatcct gcaaaccttt cgcaactggc 300
   ccgaattcac aatgtcagaa cagtttcgag ggcaacatcg acatatgtgc aacatgtagc 360
   cctctaaaac aagaaactcc attcatctgc tacaatagat acgggataat tatatacccg 420
   acagcagata tcgttctctc cgctaggttt aagataggct ctttttcacc caaggcttgt 480
   gataactacc taaacgactt aaattgtgat tcaaaaacgg caaggtcata tgtcatttcc 540
   cgaccgcagt ctttttcact gcaatatcct aactcattag gcccctatca gctgaaacga 600
   ttttctcttg caaaggagat cgttgactta cgtgctggcg ttttaacctc actcccaaac 660
   cgaggttata agggtagaac aacatactct tatcccgtca ctgcactctc actcttggct 720
   cgttccaaag tggctgaagc cgacaaattc ttttatatcg aggctaaaat tctactgtac 780
   gcttggtcac agaaacctca aatccgcttt ctaggtgcat actgtcccac agacgtgtca 840
   tgccctgatt caactgccct cggctgttgt ttttccggaa gtggatctga gttttactac 900
   gcctttcgcc agtggtacta cgcaagcctg ggtatggaag acctagtcga ctttgataat 960
   tcaacagtct taagtctctc gcctgatact cctcaaatta cacccgttgt gtcttatttt 1020
   ctagaaaaag ttttaccttt gtttaaatca catgtacccg gacgtgtttt ttactgccat 1080
   tcacttatgt ctaacggtgt atgtactttt gaccatgttg ttgtaaatat taatgccgag 1140
   gccgtctttt ttgacctcga agtagacata ggcagcataa ttgctgacgc atatcgcgtt 1200
   gaaaggccta atactttatg ttatgataca aactgtactc ttgccacaag caggaccact 1260
   gagtataatt acgctgctta tgttgtttat atactcttca atttgtattc tagtaatcgc 1320
   attgcgatag atttcaacac acactcaatc ttgcaaggat tactacaaca caatagcaac 1380
   taccagactg ctaatttaga ctatctgttt gttggagcac tttttacagg tacttttaaa 1440
   catattacaa gcaatcaagc ttacccagta cctttaactt atccaattgt taagacatat 1500
   gtagggccgt caaaccaata ctcaatgtca aataaactgt tttcatatac tcacaatttg 1560
   acggctcaag cccattcagg catatgtaac tctttttact gttataaacc acgttttgta 1620
   ccaattgatg tttttattca tagtgcttta acccctgaca gcttgatgga aacagaatct 1680
   tttgtttgtg tctctttgcg ttcaccatct gcaggatcaa catccgcagg tagtttttat 1740
   ttgcaatgtc tcaattcttc catcgatttg catccaggtt catttgtacc cgtttcctca 1800
   agtccagagt cttccagccg cgtaacagct gagctggctt ttaatactag aaatggtata 1860
   ttttctcctt gtcttaacgg tacatgtgta ctcgcaccta ctgacccaat tgtttttatg 1920
   cgtcagggtg cctggtttac aaaatcttta cactttgatg tttcaccatg caaacctatg 1980
   cattttccag acatagatat acagccccca acatacaatg tctcctctat caagatggac 2040
   gacaatgctg tattggttca agaccttact tcgggtttag taattgacca caatttaggc 2100
   tccatactca gaccgaaagg tagagctttg gaagtttcgt attatgctca ctccatttta 2160
   cgttaccttg aaccggattc ttgtctacct gacaactttc ttaactttgt cacttgttta 2220
   gactatatct gttcagactc gtcaccttgc cgtgctgccg caagccagta ctgtcaggca 2280
   ggcatttatt ttgagtctgc atttaataag tctaggtatt ctttgcttaa cgcttacacg 2340
   ctttttaaca caagtcttca aaccttattg cctgagactt ttcttgagat agaagatgat 2400
   gaaccccata gcagatcaaa gagatcaatt gatactacaa gcaatattcg ccctagtcaa 2460
   ttgcttgtta atggacgtat tccgtctaca agttcagctt ttgctgttaa cgtcgctcgt 2520
   ggtcgaggaa cgattatgcc tcgtcctgga actggtggca tgggttcgtc cttttctgct 2580
   gtttctaggt cgggtagtat ttcttcctta tcctcggttg gctcctcaac acctttgatc 2640
   tctaattgga gaacatcttc atctcaactc aaaactctca acctcaacat taacactaaa 2700
   attcctaaga tttcaacaaa gtcaggtttt gccagtatta catctttgtt tgcttcaggt 2760
   ttaggagtcg tcgatctagg tctatctatt ttcaacatga tagaacagcg tagagttgct 2820
   gagatcactc agatgcaaat tagccaactg gctgactcta tagtgtatct tgctgatgtg 2880
   acatttgaag ctatcaagaa tttggaactc tcggttaact ccttgggtac gttcttatcg 2940
   gaattttcca ctcagatgtc gatcaccata agccaaatac aatcatcatt tgaagagcag 3000
   caagatgcta caaatgatgc gttgtactac actaacgctg ctgcgtcata ccaagcctcc 3060
   atggcgtatg tcatttcaga gttaaacgca atatctctgt ctgtcactag atcctacgac 3120
   tcttacacca gttgcatcac ttctggcatt aatgggctca ttacaccatc atgcttgcca 3180
   gcccaccagt tgttacagtt actcgacacc gttatcaatt ccacagcagg aacaggatgc 3240
   cgtcccatct acggcagaga agaagtggtg aaatactaca ctttacctct aatcaatcaa 3300
   ggttattcct ttaacgggtc gattttcttc gtctttaaca ttcccatcac ttgccaggga 3360
   attgccggag atgtatatga agtagaacca cctatacttg tagatgtacc atcaaagact 3420
   gctttacgca tgattacacc atcaaacgta gtcgcaacac aagcaggatt agctgaatta 3480
   gatttgcgtc attgcgaaag gtaccataac gagttcctat gcgattcttc agcattcctt 3540
   tctacacctt caaaatacat agactgttta acaaacgcaa ctgactgttc tttgcaattc 3600
   atcacacaac acgttccaga tccttgcgtt tacacatcgc cagcttcttt atattgttat 3660
   tattcaccca tatgtgatca atgtcacata gtagccggtt gtaatgaatc tcagcagtac 3720
   aacttcactt ctgctgatgg cggcgtagtc ttttattcca tacaagacag agactgtggc 3780
   cacttccccc acatcactgt tactacgcct gcagccatac aagaagactt cactgtcgga 3840
   ccgtatttac catcgctgcc aattcacacc gcctacgtca atgttacctg gaatgtaaca 3900
   ctaccaggaa attggacctg ggaaaatatc accctaacag ccaattggac ccaacacttc 3960
   attgagatga aaaaaaacat cacaatgatg gctgaagaaa tagataacct taccaacttc 4020
   ggtaaggttt tagttggcca gctaaatagc tttttatcat ctttgtttaa cataccatta 4080
   ggtttgatga cgttttgctt ttctgtagcc gctttaggcc tgtccattat tgctttactt 4140
   gtgttatgtt ttccacagaa gccacataaa tta 4173

<210> 3
<211> 780
<212> DNA
<213> Viruses

<220>
<223> ORF-3 nucleotide sequence

<400> 3
atgatgttta ccctagtagt gctttttacc ctcctcggcc tttccatggc ctccacagag 60

ctgaatttcg atcctactct acccctcccc tctcctataa atgccctcgt cgacattttc 120

ggaaacaaca gcttgtttct caaagagtcc ctgctcggca aatccaccgg agccgtctac 180

gcatacttgt acagcagtgc catctctctc ctgctgctac tttgggtaac tgtatggagt 240

attgctactt cacactttaa cgtaactcgc attccaacca tcgcggttct cactaatgcg 300

agtatgtttt tgctgttggc atcggctact gttacaacct ggtttctccc aactgtgacg 360

aacgtcttct tttatacact cactgcgctg ttcaccttct tttcctttgt gttcttactg 420

tggttggttt actatatgtt tactaccatt agggcatatc gaagggtcgg ttcatggcgc 480

gttgtgttta acggaaaata ttctctactt gctggaactc aggctgtttg cctttgcaga 540

cccgccatac atctggttct aaccaaaacg aacacagata catactggtg tctagatgga 600

acccccatct acaatgttga cttactacaa ttagttggcc ccaaaggatt atatccttac 660

aaaagaatga ctacaatcac tgcaccaaaa ggcacaaaaa catctgctgc cgtttacacc 720

cttcaaaaag aagaagtttg tgctctctca gaaatcacag tacataatga tactgatttt 780

<210> 4
<211> 603
<212> DNA
<213> Viruses

<220>
<223> ORF-4 nucleotide sequence

<400> 4
atgtcttacc cggtttacta cgaacagcgt cgttattccc cccgccaatt caacaatggc 60

ggagggtata atcctacacc tcaacctaga gtagttcgta ctaatcctgg taaccaagct 120

tacaaccccc ggcgtaaccg aaacgccact ccgaaccaac aacaaatggt tccttaccag 180

cctcagtatc aagcacctcc tcagccaagg gtggtctatg tagatcgccc tcaagaacct 240

gtagtaattt acagagctcc tccacaagga aaaaaacaat caggcaaacg ccacacagca 300

gaagaacgct ggtatcaagg cgaaaaacct gtgcagaaga aacaggcacc caaaggaaaa 360

tcaaagaaag cagcaacacc tgctaatcct aaaaagcagc ctacacaatc tgacaaagtt 420

cccatcgcct acccagacaa tcatcccttc catgacctcg caccagctga catccgcgct 480

ttcaaaaagc agctgatcca aaatctggac cttggacatg gtgaaatgaa tcaactgcgg 540

ctttcaatcg atctgttgcc catcaagaaa ccagcaccaa caccagcggt gccagctcct 600

ctg 603

<210> 5
<211> 339
<212> DNA
<213> Viruses

<220>
<223> ORF-5 nucleotide sequence

<400> 5
atgtttaccc ttgtgcttat tatcctgctt agtttttcta tggcttttaa tgcttttaca 60

tttctgctgt tattattttt tacttttaag tgcattataa cccgcacttt agtcgtagtt 120

cccattgact acccagaaaa tcatcctttc aatggcctct caccagagga aatcatcagc 180

tacaaatcac agctgatcca aaatctcgat cttggacatg gtgaagtaat taaacatcga 240

ttctcaattg atttacttcc cctcaaaaca acaagcactc ctaccaccag tgctatttta 300

tggaaaaggt tcaaaacctc ccataaagaa aacaaccac 339

<210> 6
<211> 24613
<212> DNA
<213> Viruses

<220>
<223> Genome nucleotide sequence ("n" at positions 2748 to 2761, 2781,
      2782 and 10029, means any base)

<400> 6
   gcagtccacc aacacaacgt ggctctctgc ttacctgtaa gggcacgccc ttatgctgaa 60
   ataatcattc aggaacacct ttttgtttca agaaatagta ggagttttaa accatccttc 120
   tatcttgttc caacaaagga gccgcaatca aacactctaa cccttcatgg gggtgatttt 180
   gatgcaaagc gacacttggg taagaaacca gtaaagtgca atattcttga cattgttgac 240
   aaaaaccaca caatagcaat gaaaaaaatt gaagatgctt taggaacact taaacctgcc 300
   tttaaggcac acatagactc tctacctcct ttcatgggaa aactagccac aatgctagct 360
   gagacaagac gaggaaaaac tccccctctt ttgatctatg tcatatcaac aattttggaa 420
   acgaatatta cagttcatta tgtcagccac acaataactt cataccattc ttcaaacgca 480
   cacactcaca ctcatgaatt cgactcagaa gattacactc ctgacaatca gattttaact 540
   aaagtcaacc gacatggtaa tgaccttaat cattcatata tccatggcgc caccaatatg 600
   tacaaccctg tctaccagag acacccacca aacatgtgct acatgcttac gggactgtac 660
   atgttgtcag gcttacagga attgtatgcc atggctgaag acaacttgac aacatgccaa 720
   atcaaccttc tcagatgtct gtttgatttg aatcaagatg aatttgatgt agattacaca 780
   tttgtaatat acactcctag caaatctcag gagtgtgcct tcaaatatct tcaggagata 840
   gtccaccact gtgaactcac catttttaga catacaacaa caagtgtctt cagttgcaac 900
   aaatgcaacc atgtggaaac tgtcatttct agttgttctt tgaatcttgt atatataact 960
   gattctattg aaaaagcatt tcaacccact gtagaagcta ataccgacta catgtgtgaa 1020
   aactgcggtc tacgcgacca taaacttaaa acaacagtaa caaatccaga cctccgtttg 1080
   gcacaactaa actatccaac ggattcaaaa tacactatct ttcttgatga acaagctcct 1140
   tttgtcttcc attccattgc aaaacacgtt ggaactgcta attccggaca ttggagtgca 1200
   ttgaatgtaa attcagatat gttgtcagac tctaacgagc gacaacatta ttatacaaca 1260
   cctagtattg ttttgcttgc atttcttcct gaggaagagc tacagaacat tagaaattca 1320
   tcacctcttc aggaccatca accagatgat gttgaagacg ttgaatctcc cctaccaggt 1380
   tctatatttt atactacaga tgacattttt tctactaaga gtctatccat agctcactgt 1440
   gtagcccgcg actttcacat gtccggcggc atagcaaaaa ttttctcaga taaattcggc 1500
   tctaaaactt tcttgaaatc acaaaacccc gttataggcg gtttttccat tttacttaga 1560
   gagtgtcgtg acatgtacta cctcgtcaca aaagagaaaa cttcagataa gccgacatac 1620
   caagacctta aaaactcctt gggttctatg acagagaatt tggttcgcaa gaatcataat 1680
   actctttcaa ttccatatat aggctgtggg attgatggtt tacaatgggc aaccgttgaa 1740
   aaacaagtca aagaaattgt ctgcgctcga ggtattgatg taacagtcca ccacctcgaa 1800
   aatgaagtta aacacactcc agaacaacaa acagcttctg acaattcagt caaattagtt 1860
   caaaaactct ttacagaaac acctcaagct atacctatag ttgttccttc agatgactca 1920
   gacagcgaca tagatgagtc agccgatgtc tttttaccag gacctgaatc tgaatctgat 1980
   tcaaaatcag aatctgggtc agattacgac tttaaatcgg cttctgaacc agaagatgaa 2040
   ttggagccca ctccaatctc tgaacttgaa ctaacaccag cgtcaagttt aactgtagag 2100
   tccgatgaca atccagacac aagccaggaa acattaccag aatcaaactc tgaggaaacc 2160
   aaacctgagc aaacacctga cacaacatca aaggtgtcct ccgattcgaa gcttgatcca 2220
   caatcagaat tagaagaaga actggccaac aaaccagaat cggcttctga accacaatct 2280
   gagactgaat cgagttctga atctgaagag gagcttgagc cacaatcaga atcagaggaa 2340
   gaaccggcca acaaaccgga atctccttct gaatcacaat ctgaaaatgg atcgagttct 2400
   gaacccgaag aggagtctga aaaaccatcg gagtctgctg aaacagcaac agaggatagc 2460
   ccggaaacaa caccggaaac aaccttagag ttaaccacac aactcaaacc tgcttcagaa 2520
   tctgacgaca aaccggacac accagcacca tcaccttcac caattcaacc agagaaaaac 2580
   ttggacacca cccctgaaca aacttcacaa ccaaccacac aactggaatt gacgttagaa 2640
   acacaggaac aaccagacac tacaccagaa gtgccatctg tctcagaaga taaaccagac 2700
   acacttgaag aatcttctga atcaacacca gaactctcag aattggannn nnnnnnnnnn 2760
   nccaccagta ccaagaccta nngaaccaga cctccaacac ctagaccaag atctgcacgt 2820
   ggagctagga ctagatcctg tgctggcaca cctataccag ttatttttga tattatagat 2880
   aatacaagcc agcctcaagt tccccttgac ttcccagaag cactgcaaga actgaacaaa 2940
   cctagtgaag taatcccagc ggctagtgaa aaacctgtgg aaaaacaaat aatccatagt 3000
   tttgtaagtg tcgaaacacc ttgcaaaccc aaagccacta aagttacgaa ttatgtagct 3060
   gcacaatcta atgcaattct aaattgtatt aaggctttca ttcctagcaa cccgctttca 3120
   ctattcaata gaaaaccagc ttttagaaaa ataatattca ctgaagacac ttcagaacca 3180
   gatagcgatg atgatgattg tgaatacact ccaccaacat caccattccc tgaacttctg 3240
   gcattggtag atgaagacat tgaagtagaa caaactcaat ctgtaattcc aaaaacagac 3300
   tctgcttcaa ttgtggagga tcttaaaaaa caagaatcct ctactttgtc attggacacc 3360
   aacacatcga aacctacaag ctctccgcga agacagccta gggaagtaga aagtgttgat 3420
   gaatccagtg atgactcatc taaaccaaaa acaatttcaa cattagacaa acctgctatg 3480
   aatagtgaca cgaaacctac agactcttcg cgaaaagagc ctctggaagt accagttact 3540
   acatctttaa gcacccctgc taaaaaccaa gataaaaaat cttcaaaatc tgcaaaagta 3600
   ataaaagact attctttgac ccctaacaca gtcaaacagc aagtctattc actctacggt 3660
   gaatcagtag atgcagttaa ataccttgtt caaacatacc cagacagggc taaacaaaca 3720
   gctggtattg cttatttcct tataactact tatttaatat ggaccatcgg tctcatagga 3780
   gtaccaatgg cctttaaaat accaatgttt ttatgtcttt tataccaagt taatggatta 3840
   aatatagcac catttgttac taaccaaaag ttacaatatg ttgcatttcc actttggtat 3900
   aagctctatg aagtaatatc agtccgtttt gtggcgaata tagcacaatt tattgttaaa 3960
   acaccaccta tagatgtttt aaacaagcta attcgttcta ataaagacaa gccagtcaaa 4020
   ttaacaccaa ataaacatac tttgatgtta attcatgact tagctttaga gtctgtcgac 4080
   ggaaaagaaa accgctatta taatactgat gttacaactt tcacaaaaag gcatagcact 4140
   tctaatattt catatgttct aaagtctact ttaatcaaat atgtcatgga ccattgttat 4200
   gtaaatattg cagtttttac tttagttaga tacttaactt tattagtgtt tattcaacat 4260
   ttctctaatc cttatgttct tgaagcaaat agccaatcac ataccgtttt acagtatctg 4320
   ttttcacact tgagaccatt tggaaggcct ttgtgcccaa ccctcaatga ctacatgacg 4380
   acagcaacac cacgcgatgc acatgtacaa gcaggttctc acttcagtga attttgtgtt 4440
   cctattcatt atacaacacc aatcattaaa agcacaatgg cagaaccatc acttttctca 4500
   ctttttaacc cagtcttatg gcctttggtt atggttgtat atttttatcc tccaatgatg 4560
   tttatagcaa atgcagtcgc ttattcatgc cttcctttag tggtcttgtt acaatggctt 4620
   tatgccatgt ggttttcttg tacatgctat ggcaccaaaa gatgtgccaa gcatttgcat 4680
   aaaaatgaag tggttaaacc aatggaatcc acttcaacta agaaccgcat gacatttact 4740
   ccatcaacga ccttttgtag taaacataac ttcttctgtc cagatgcacc acatataatg 4800
   actcttgcaa tggctaggca acttacaaac tactacaatt tgacagatac agtaatacct 4860
   gacatccagg aatactccca cgagaaccct actgtacaat ttattcactt tgatccactt 4920
   aaacacggtg ccgacacaat tttggaacca attacaagcg ctagcgccag ttcaattgtt 4980
   gcatggtact ctctcctctt taatcaaaag tttgtccttt cacattatag ctacagaacc 5040
   ccagtagccg tagttgacaa accagaggaa acagatggtg atgatacaaa atcattagca 5100
   tctgacactt ctgataactt tgagtctatt agaaagacca accataagaa tcagagcaaa 5160
   caacagttta ggccaaacgg tcaccaaaga ccaagtaaga ctttcaaacg ccattcaaga 5220
   ataatgacat ctgaacagaa gaacagctta attgaaactt ttaagggttt aacaaatggc 5280
   acagcagcca tcccacagcc tttaatcatt tttatttggg ttatccttat ggtaatacca 5340
   acactctttt tagtcgccag ttccagcaga acagctgcaa caatgccttt aaaccgctac 5400
   tcaggcgtca accccactgg aattatgttt caccaagcac ctccttacat ccattcggaa 5460
   ccaccaaagg aaacttacta caaactcagt tatccttatc cgtcagcaac agttgtgaga 5520
   accttgaaag gccatctcta ttaccatagc gatgataccg ttcaacaaaa ttgtaccatg 5580
   caatattcac ttatagctgc ttctacaaag cacgtgtgtg gcaaggtagt ttacactata 5640
   ccagcccatg tctcaattgg ctcacttaaa ctgttgcttg tccacccgga tcaaacaaat 5700
   ttaccatttg aactaccagt ttcagatgaa gtccgtcttt gctacctcac aaccttgaac 5760
   gcaccaagat gcatgccctc tcaactagcc atgtcaaata accaatttgc cgctgtaagc 5820
   cttgttttgt taataacatt agtttcttta attaaagttt atataatgtt ttttactgtt 5880
   tttaaacact acacaacaac tgtttttata cttgtagctg tgactactat cacaatgttg 5940
   gtgtccttct tagctcctcc acttctcata gtcgttcttc tttcactagc atggctatgg 6000
   tacggcaata caattgtatt gtgccatatc atgcttttga tagtcttagt cgtctcatgg 6060
   aaagtggctg ctgtctgttt catcttcgcc ttattgtact ttggaaaatg tgctatgctt 6120
   agcaagaaca ttaaatacgt acagggtgga gttaaatttt caggaacctt tgaagaaata 6180
   gctcagtcaa ccttcttcat taactacgga gtagcttgtc agcttctgga acatactgga 6240
   cagacaattg aggatataat gcaacttaga accgcgggtg gagccccagc aaggcttgcg 6300
   cgctcaatat acgattgctt ttccacaaat gcctctgtct tgtacagtcc caggtcattt 6360
   tcaccacagt cacttataac aaaatattta tacccaggtt cgatccctgt cggcagagcc 6420
   cctgtcttat taggcaaaat ctccggcatg acttgcttag ggcgtgaaca gtccacctgt 6480
   ttccaatcat cagctacaac cattactacg tgtacccatg ctgtaaatac tgctggaaca 6540
   ttcatgtctc aaattaaatg tgttatagat aataaaatat atacagttca acccgagaat 6600
   ataaccataa ccggaatgaa agctacattt gaagttgaag gactacctcc attcaccaac 6660
   gatgtaacag tggccccaaa gccgctgaag cattacatgg atggaaagag acaccttgtt 6720
   ctctacacta aaagtgagag catagtctac tcttcaataa tgtggccgac tgaaaacggt 6780
   ttattctcgt catcagtttc tgacccagga gattcaggtg caccctactt ttcagacaat 6840
   gtcatagtag gaatacacca aggtcgcaac gaagcaacca acaatcctgc cattttagca 6900
   agtggtatgg atggtgagtc tccctgtgta ggttacgatg accaatcata tggccttcca 6960
   cttcaagaat atttcactca cattgtctta tcaaataagc caagtgactt tggtgctcca 7020
   tctaacgtgg cgccaaataa atactacaac aaaaaatcat ttgaacaatt agctgacgaa 7080
   gataagactt atttaaatag tttatcatat cccctgtcct catctaatta ttgttacttt 7140
   aatagcttca aaacccaatc aagcacaaca atgctcgaca acgctgaagt tattaaatat 7200
   gtagttttgc ttctcatgat cttggattat ttcttttcaa tcatttgcga agatgcttta 7260
   aacccagcat cttacgctat gttagttatc gttttggttc aggcttttat tacaaaaatt 7320
   acagttttca gaacaggtat ctatatccag gcagccgttt ttcaagcatt tattgtacct 7380
   atagtcagtc aaattacatt gatactggct gcagatactg caagaagttt tttaacgttc 7440
   cacttttttg tacttgctgt tttgacatat ttcgttcttt gccgtattgc tgtagatttt 7500
   tggcgttcca tgtttttgct atttttgaca agcgtctttg caaccatcat atggactaca 7560
   aagaatgact tcaatatttt acatgaaact ggcgtggttc taacacccac ggcagaatta 7620
   gctcttatag tagcttttac ttatataatt tatgcttcat gtatgttaac acctgtacca 7680
   ctgtatacta tttgtgtttt cttttcattt ttatcaaatg ctccactcta ccttgccgtc 7740
   ctctcattcg gcattctagt ttctttcaaa acaaaccaag attttggacg tctagtggat 7800
   aaagtgtttt ctttaaatat gctctatgaa taccatgctt accaaaacta tgttattcaa 7860
   aactcaggtc aacacccagg attttacagg tcactctttg ctttttttat caatttgacc 7920
   acccaaccaa aaacaacata caaatgtttc aaaccccaga cagcaagtgg ttacagagta 7980
   atatatcaaa ctcccactac agagttcaat aaatctctgc aacatgccag tatcacaaaa 8040
   gatgacaact ccaaccatat aattatgttt gctgacggct catctgataa tctcaattgg 8100
   gcaaaagaaa tggtcgcaac cattcatcta accaacccaa atttgcagcc actcatcatt 8160
   ggatactacc acaactccat ggacgtcata accaagggaa cttacatgca acatgaattc 8220
   ataaaaatgc cagctgttat cttaactcaa gatcctctaa ctgaaccaat cagtcattta 8280
   gcagcagcag catttacttc aatttctgga aaacctcagg cacagaaaaa caacgttgtt 8340
   tcaaactcca aagcgcgcat aaacacagcc gttcacgacg ctgtcgaaag cgtttattca 8400
   ggagaaacat acgttgcccc caaacctata gtctcaggaa aaactgttgt agagaaacca 8460
   ttctctacaa ccgaaaccac catgtacata atgcgtggtt tacccggttc tggaaaatct 8520
   ttcaaagtta gtcaattagt tgctaaagat ccaaatttag tcgtagcttc cgcagaccac 8580
   tttagatatt caaatgacaa aactggaaaa gccgtataca cctacattcc agaagcaact 8640
   agttctgtac atttacaatg tcagaataga gcccgcaaag ctctagaaaa cggccaatct 8700
   gtgtgcattg ataatacaaa tctaacactc ttagaaatga gaccttacgt cttattagcc 8760
   cgttctttta actataacat tgaattcata cactcagact ctccctgggc cttaaacctt 8820
   gacctgttac atgctaaagg tgtacataat gttcctagag caaagctcgt aatcatgtat 8880
   gatagattct ttgaccgtga taatcaaatc gatgcagaca gtcttataca gtatgttatt 8940
   gaagcaattg atccaaaact tgttgctcca atcatgaacc gtttccctgc cgactgtgat 9000
   cttatccttc aatctgccct aacaccagac cttgaagtat taaagcaaaa ctacgacaga 9060
   gcaaacgcaa cataccaaga tgtttcttta gatgatcctc cggctttaaa ggcagcacgt 9120
   cgtgctatga atatagctaa atctgaatat gaggcaggcg aagcaggcca gcgtcgcatt 9180
   gagaaatttt tagaaagaca ggatgtagca gcactcaacc aaacgctcac aactgtcaat 9240
   caatctaaat tcatagcagc gatccgttcc atctacctaa gcaccattag caatttgaga 9300
   ctaaaaaccc gtcatatggg tgaaggatca tatgcagtta catcaggtac taatactacc 9360
   gataaagttt tagttaacac gccacaacgt atgactagaa ttgaagatgg catttataag 9420
   cttgttgcaa acggttttga aatcacaatg tgcgacggca gcaacttagc cggtgttact 9480
   tttgaacagg atataaatcc tagcatgtac ccttttgttt ttacattaat gtcaaatata 9540
   gctgtacctg ttttaacccg ccaagcaaat gttggctatc ttgatatgtc aaataaattc 9600
   atctgtaaag atggcactgt tcaatttcaa ggtgtcatct atgcctacca cactccatca 9660
   aatgagagtg ctgacttcaa agtaggcaat accagttgga ccctccagaa aaacatcaat 9720
   ttgactgctt ttattcctgc aattcataaa actgcaacct tcgcagcaca atcagtgttc 9780
   ttaggaggac tacccatgga agagcaccaa gccttttccg acacacccac agcctcaaac 9840
   aaatttaaag tttttgtttc atccacagtc tgcgcctcaa ccgtgtgcaa agtaaatcat 9900
   aaaacttatg tacagatacc agatgacatt caagatcctt ttacatatat gcatcacagc 9960
   gtttgttcac acaacaaatt tttatcaaac catgaaccca gatgtcaaat ctgtccttta 10020
   aactgttana gcgcaaatcc gtgtgtgtct acggcttgcg ctctatttga taatggcaca 10080
   ttacctcggt caacacatta tattaatgtt agcaccactt caaatgttgg cttgtttaag 10140
   gcagtaaaga agtctactcg tcaactaaac attgacggtt ttccttacat gctaaagcag 10200
   gttaaagacg actcagaact tgtaagttct cttaaaatag gtctacctaa tatcctccca 10260
   catcacatgg tggaaactaa gtcaaaaaca taccttctta ggggccccac aacggcttac 10320
   tcacttggcg atttatgcta cgcactcttt aatggcgact ttgattatat tcgcgaaaat 10380
   ataaactctg atttcgtttt ggaccgtgaa gccggaatgc ctgatacaga aacacgtacg 10440
   tggctgttca gcattttaaa ctttgcagta cctagagtgt gtgctataat tgaccagatg 10500
   atttctgaga acgtcttcta taaactgact ttggataact tagatctata cggatcactc 10560
   tatgattttg acgactatcc tactgaaggc tttaacaggc ctgatgatgt gatacgtatg 10620
   ttaaaggaga tatggtcctt ctgtagacgt ccactacctg ccgaccttct taaataccat 10680
   gaagacatcg gtgcagcagc cactcaagaa atattgctgc atgcaccctt cattgataaa 10740
   gtttgtgctc taaatgacag attagctgtt gttgataata gagcaagtca atactttttc 10800
   tgtgaagaag aaggtgtctt tacccatatt tacaatccag tctacggaac tttagcattc 10860
   gataacaagt tgatccaatc aaaggatcct tcatgtacat tacagcgcct cattactata 10920
   caaggccctt tgtctacgaa tgctagtccc gtgatctcta tttctgattc cactcatatt 10980
   gccaacaata ttaatccatc taaccaaaag acaacaccgt tgtactacga tttggaactt 11040
   gcgcaagaat tcattgacgc aggtttaaat attgatggcg tttccaacta cttcttctat 11100
   ggaccgtcta gagcgggtgt agtgtctgat ttcttactat atgaattcca aggaactcaa 11160
   tggtttgaca ataacatgct gcgctctctt tattctttca tattgaagaa ttcagagtgt 11220
   tacagaacaa cagatcaact ggactttaga ggtggaaaac cccgtaaatc ctcaatggga 11280
   catggtgtta ctggctttaa gcaagacgtc gtgtacgctg ctttaggccc tgatatgatt 11340
   gaaaccttgt atgaaacggc aaaacaaaca ccattgccgt tttgtacaaa aataactgcc 11400
   aagtatgcat taacagcaaa gcctagagct cgtacagttg cagcatgctc ctttgtagcc 11460
   tcaactattt ttaggtacgc tcacaagcct ctaactaata atatggtctc aaaagcacag 11520
   cagggtttgg gttattgttt aattggaatt tctaaattcc acggtcgatt taataaattt 11580
   gttaagtcta gggtaggcac tgtcgaagac tttaatgttt tcggtagtga ctacactaaa 11640
   tgtgaccgta catttccctt agctttgcgt gctctttcag ctgcccttat tttcgatctt 11700
   ggcggccatg acccagacaa ctgtcttttt attaacgagc ttaatgcata catgctagac 11760
   attgtttcag tcgaagactc ctttgcaaat aaaccaggag gtacttcatc aggagatgcc 11820
   actacagcat actccaacac tctgtataac tttgcagtcc actatattat catgtggaaa 11880
   acattcttga cagtcaatga cccttctacc aaggtcatac gcagtgcagc tcatcacgcc 11940
   ctaacaagtg gtgacttctc tatgtacaat gacatgatac aagacatgtt ggatgtagac 12000
   tatacactca acttcctctc tgacgattca tacatctgtt caaaaccaag cgcttttccg 12060
   atctttacgc tcgagaacta tccttctaaa ctgcagtcta tactccacac agcagtagat 12120
   agcaaaaaat cctgggaagc aaagggtgag attaaagaat tctgttcctc tcacatagtc 12180
   aacgttgacg gcgactacca ctttaaacca gaaaaggata gaatattggc ttcattgctg 12240
   atattatcga aaatcgctga catggacatc ttctttatga ggttcgttgc gttattggct 12300
   gaatccgccg tatatatacg catcgatcct acattttggc tggccctgtt tggtgttttc 12360
   gaaaaccgcg taacagcgtt taaatctgaa acattgctct cacctgttcc tgaacaactc 12420
   atgaaggtgg ctttttatga atcgcttgtc tttgccgacg tggatgctac agccttatat 12480
   ggtttccttg atggttttaa aatgcaaagt caaactctcc acccagacgg tgttgagggt 12540
   tttgacaagc aaagtgaccg agtaaaacac tgttttgctt gtgacaatat atcagttgga 12600
   cactgttcga tttgtcccgt tccccttcct ttgtgctctt tttgcttcta tgagcatgct 12660
   ctgctcaatg aacattatga agcttctgga attgcgtgtg aatgcggaga cgctgacatt 12720
   agacaacttc acttaaaaat aaccaatcaa ccatcctcgc acaattttat ctgtgctgaa 12780
   tgtcccactg tagctatgaa gctgccaatc ttcaactctt tccaaggaaa agtactgctt 12840
   ccaatgttcc gtatgaatac gccattgcct tcctcagtct ctgtaattgt tgatgtacgt 12900
   tccaacccaa aagcacctaa gatgctgtgg gacgacgtcc agaatttcag agaaaattgt 12960
   actaggatag catacgaatc cgtttcgtgt gctgaactag ctagggaggt ggtttactat 13020
   ccatatgaag tgattgaatc caaagcaggt caagcacgac ttagaataca gaactttaaa 13080
   tgttcaccaa ctacttatgt tcagttctac aaagtccgtc aaaatggaaa gtattgtcta 13140
   gtagccaaag caactctaac gccggctttt gaaaaccaaa cagacatttt ctccgttttt 13200
   caaccaaaca acttttcacc ttggaataca tcatcagtgt ttgcagtaga acaatacgct 13260
   gcaatatacc ctcccatacc aaaggaacca gtcaatgcta cgttcgtctt aggacctcca 13320
   ggctgtggta aaacatacta catagccaaa acgtactttt cacaggcttc tgagacatgt 13380
   ccggtcgtat actgcgcacc tactcacaga ttagttttag atatggacgc agaatatagt 13440
   ggtgtagttt caaaatctct ctacaataat agagtgtaca aaaatccagc ctacaaaaca 13500
   ggcgaaccat tcaaattatg tttcaccaca cacaacacga tgccagttca aaagaaagcg 13560
   atcctcatta tagatgaagt gtctttaatt acaccccact ctctattttc gatcattggt 13620
   aaagggttct atgagatagt actcgtagga gacccttttc agctctcggc tgtttttcca 13680
   ggttttgttg tcaatcacac atatgacggg ttttacatcc gccggctagt aaataaggtc 13740
   aaacacctaa cagtttgtta ccgttgtcca caagaaatct tggacatatt ttctaagccc 13800
   tatcatgatg ttgggattga cctcacaacc ggaaatacca atccaggaaa ggcatccatt 13860
   tatacactaa attggcttca agcagatgta ggtactaaaa atccggacaa actcagacaa 13920
   ctctttgcgc aatatccagg ctttaaaatt atcaccaact acagatgtgt tgttgatgca 13980
   gctaaaagtt acggtattaa cgtcgaaacc atcgactcat cccaaggaac caccggagat 14040
   aggcatctgg tggtaatttg cggcagtacc aacttttcta aacttttaaa caggtttata 14100
   gtagcagcct ctcgttcaac aactgaacta gttatagtca tgttgccaga gctttacaac 14160
   tatttaacag agacgtttaa cttcaaaccg ttacaattgc aaaatgtgca tgtaccgatc 14220
   gcagtatctt ctacagcatt ctgcgatata gaattttatc actttcaaaa gaagttctat 14280
   gttggtgaaa taagcgtaag cacaagtacc actatgacat gtcagttggg ttgttatatt 14340
   aatggctcct acatgctccc acctgtgctt gaaaactctg aagaccgtct ctacgttcct 14400
   tctagatgga gacgtatgat aagaaaatac cctactgaat ctatgcacat ttccttactg 14460
   gacagacttc tgaggcacat tttattaaca actactggag aaattcattt cgtaatgttc 14520
   tctgcagaca atgatctcat tgcactggat ccgtacttta taccacccac tctatgtgag 14580
   tgtggtagtg cgggtctagt ggaagtagac atcactgttt tctgccgcaa ttgtttgcct 14640
   aaagatggta aagccactcg tttggtaaaa ccgtctacac tagatgtcca gactgaaaaa 14700
   ctcagacttg caaaagttca tgctaaggtt tgtaaaatca agcatggcag tgctcacaac 14760
   gctgatgttg atgctattat gactcaatgt atatatgcta atagcttaac attcacacca 14820
   acaacccaac tagttgttaa cactgatgag ttcacctttt acatgctacc taggccgtca 14880
   aaccgtcatt tgagaatcat tcataagaac gacaaacgtt tctatgctat cactcatgaa 14940
   gaagaagatc tcttctttac taacatctca gcagtggtag acccaattcc tgcaaaattc 15000
   aacattgcac actctacaag cttcctcacc atcaaaagtg gttgcgcagg taataagact 15060
   tgtaccagat gctattattt acacttagca tacacggaat ttgtttctca acacaagtat 15120
   gaaccattca cttgtgtgtc ttttaagata cggtttgact tttcacaatt cactgactca 15180
   gtagatactt tcctccgaca aggcttaata acctttcatc cggagatgaa ttcactgcaa 15240
   aaatcacttt tattagcagt ggataaggtc tattgtgata acttcacctc aaacggtaga 15300
   aggtttagac tttacgacaa caatttggtt aaatccataa tcaaaggttc agtggctcaa 15360
   aactccatca tcatgccact cgactcagtt ttacacgggt tgaacattga tttcacagtc 15420
   ggatgtgccg tagataactt ttcctgcaaa gaagcagcga gtgttaggta ctcagaagta 15480
   gtactttcca tcaccaagtt gcccccaggc acttgccagt tatactacgt catatcttac 15540
   ggcctgaact ctcccaagac cacttatgct ggtcaccaat tgttcgacgg ctttgagact 15600
   gttattgttg ttaatcgtaa agataaaccc ccttacgtcc tcacacagta tattaatgat 15660
   gttgtagttg caatgccaga gtccctcttt tcaacaggtc gattctacag agaaaaacca 15720
   tatcccgtcc ttatgaacga ggatttaagt ggcttaaacc atcacatttt ctctggtgac 15780
   tatacagacg aatctcttac attaggaggt gtccatcata tagtaacttt aaacacctat 15840
   gaccacaagc tcaactatat ccaaacgaaa gctacatgtg ccgcctcagt ttcaactggc 15900
   ggacgtggtc ataaaattac tacactgttt gacgttcatg caaatcaact tgctgatgaa 15960
   attaccagag ttacatctgt tgttacaaca cagtctaaag ttattaattt gacaatagat 16020
   tatcagcaag ttccttgtat gtactggtct tcaccgaccg gcataagaac cttctaccct 16080
   caggctgtta gactggacgc aaagtttata ccatactacg tagaatatcc caatattcta 16140
   ccggcagttg ttgaagacca ggtgtacgat ttgtctaatt acaatcaacc acctttaggc 16200
   caaaactgcc ctgtaaactt tcacaagtac gtccagctaa ctcactttat tttagatcat 16260
   gtgaaaatcc ccgaaaaggg tttgatatat catatcggtg cagcaggtac taagcaatgt 16320
   tcacctggag acttaatatt ggaacaattt ttcaataaat ccatcatata ctcaagtgac 16380
   cttcttcctt accaatcacc tgctgtgcag gttgcattgg atgtaaggtt ttcggcttca 16440
   ctcatcattt cagactgcta ttcgaaagaa ccgcagcctg atttgttgag taagttgatt 16500
   aacaaactag tgtatggtgg aactctcatt tttaagacca ccgagacttt cacatgtgac 16560
   ccagcctttt atgttgctca ttttaactgt ataaagtttt ttactgccgc tgttaatcac 16620
   tcatcatcag aagtttatat tgcgttcatc ggaaaactcc ctaaaccaaa caacaacttt 16680
   ttagcctcag actatttcca gagattaact caacatagaa ataaagtagt taaacagcct 16740
   tacgctcaca catgggacac atcttttacg tacccatacc cctcaaatgt tcttcaagtt 16800
   agtcgtaaaa accttttata tctatttgaa accagaggag ctgcagtagg tactttgatt 16860
   tttgaagaac catcaaaacc tgctgtaaag atacctacaa agtgtcaaac cacacaaccc 16920
   tcgtgtgtca ttgaggttgg taaccaatac gattgttgca ttcaagacat cattaccctc 16980
   ctcaatggaa aatccttcac agtgaaggtg cccaactcag aatccttact gcgcgatatc 17040
   tgcacacttg cgcttagcca gagttattcc atcaatattc gcggaaaaac actctacacc 17100
   cttagttccc tacttagaat taggcaacaa tccttactgt tttacggaga gaaggtcaaa 17160
   aaccctcgac cccgtaatgt cttgaacaaa tataccaact acctcaaggc aaaagtgatt 17220
   aggcattaca ccaagcctca atcaacagtt ttggacattg gtacaggaaa aggacaagat 17280
   ttgagaaaat actcgttagc aggggttaaa tccctcactt gtgtcgagcc tagtcccgag 17340
   tctgtgactg aactttcaat aatagctagt ccccttgata tggagacaca cacagttatg 17400
   agttctgccc agaaattcga gacctcgctg acgtttgact tggctttctc tttctttgcc 17460
   ttgcactatg cattggatga cgtttgtatg tctgaaacac tcaacaatgt tttttgcaaa 17520
   cttaacagta attcacagtt gatcttagta gttccaaatg ctggcaggat gcaatccata 17580
   ccttcccttg gtttaacagt cactcatcta gatgatgata aagtttggtt taaatactca 17640
   gactatatag actgcgaaga accgttagta gacaaagaaa aactacttac gtgtttagct 17700
   acatatggaa caattgttac tgactcacca ttctatgacg gtgcaaacaa aatcctagac 17760
   caaaaatgct catccatgta tagagcatcg acagcccatc taaatcccga tgaaattcaa 17820
   tatattaata tgtatgattt aattgttgtc attaagaatt agcacaacaa aatgttcgcg 17880
   ctcgttctaa ccctcacaat agcttcggct attgcccaag atttccccgc atatgacccg 17940
   tgtcctactt gctcaacccc cggtaataaa ataccggctc cgagcacagt tgcccagtat 18000
   tcaacaaact acggtgcgaa cttctttacc gtagtctttg atggtattat cttcaaccaa 18060
   tttagggaga gttattacca ccaatgtaga ccaacacctg aatactgccc agatgcaatc 18120
   aattgcgcct taaacagaac aggcgcatcc tgcaaacctt tcgcaactgg cccgaattca 18180
   caatgtcaga acagtttcga gggcaacatc gacatatgtg caacatgtag ccctctaaaa 18240
   caagaaactc cattcatctg ctacaataga tacgggataa ttatataccc gacagcagat 18300
   atcgttctct ccgctaggtt taagataggc tctttttcac ccaaggcttg tgataactac 18360
   ctaaacgact taaattgtga ttcaaaaacg gcaaggtcat atgtcatttc ccgaccgcag 18420
   tctttttcac tgcaatatcc taactcatta ggcccctatc agctgaaacg atttctcttt 18480
   gcaaaggaga tcgttgactt acgtgctggc gttttaacct cactcccaaa ccgaggttat 18540
   aagggtagaa caacatactc ttatcccgtc actgcactct cactcttggc tcgttccaaa 18600
   gtggctgaag ccgacaaatt cttttatatc gaggctaaaa ttctactgta cgcttggtca 18660
   cagaaacctc aaatccgctt tctaggtgca tactgtccca cagacgtgtc atgccctgat 18720
   tcaactgccc tcggctgttg tttttccgga agtggatctg agttttacta cgcctttcgc 18780
   cagtggtact acgcaagcct gggtatggaa gacctagtcg actttgataa ttcaacagtc 18840
   ttaagtctct cgcctgatac tcctcaaatt acacccgttg tgtcttattt tctagaaaaa 18900
   gttttacctt tgtttaaatc acatgtaccc ggacgtgttt tttactgcca ttcacttatg 18960
   tctaacggtg tatgtacttt tgaccatgtt gttgtaaata ttaatgccga ggccgtcttt 19020
   tttgacctcg aagtagacat aggcagcata attgctgacg catatcgcgt tgaaaggcct 19080
   aatactttat gttatgatac aaactgtact cttgccacaa gcaggaccac tgagtataat 19140
   tacgctgctt atgttgttta tatactcttc aatttgtatt ctagtaatcg cattgcgata 19200
   gatttcaaca cacactcaat cttgcaagga ttactacaac acaatagcaa ctaccagact 19260
   gctaatttag actatctgtt tgttggagca ctttttacag gtacttttaa acatattaca 19320
   agcaatcaag cttacccagt acctttaact tatccaattg ttaagacata tgtagggccg 19380
   tcaaaccaat actcaatgtc aaataaactg ttttcatata ctcacaattt gacggctcaa 19440
   gcccattcag gcatatgtaa ctctttttac tgttataaac cacgttttgt accaattgat 19500
   gtttttattc atagtgcttt aacccctgac agcttgatgg aaacagaatc ttttgtttgt 19560
   gtctctttgc gttcaccatc tgcaggatca acatccgcag gtagttttta tttgcaatgt 19620
   ctcaattctt ccatcgattt gcatccaggt tcatttgtac ccgtttcctc aagtccagag 19680
   tcttccagcc gcgtaacagc tgagctggct tttaatacta gaaatggtat attttctcct 19740
   tgtcttaacg gtacatgtgt actcgcacct actgacccaa ttgtttttat gcgtcagggt 19800
   gcctggttta caaaatcttt acactttgat gtttcaccat gcaaacctat gcattttcca 19860
   gacatagata tacagccccc aacatacaat gtctcctcta tcaagatgga cgacaatgct 19920
   gtattggttc aagaccttac ttcgggttta gtaattgacc acaatttagg ctccatactc 19980
   agaccgaaag gtagagcttt ggaagtttcg tattatgctc actccatttt acgttacctt 20040
   gaaccggatt cttgtctacc tgacaacttt cttaactttg tcacttgttt agactatatc 20100
   tgttcagact cgtcaccttg ccgtgctgcc gcaagccagt actgtcaggc aggcatttat 20160
   tttgagtctg catttaataa gtctaggtat tctttgctta acgcttacac gctttttaac 20220
   acaagtcttc aaaccttatt gcctgagact tttcttgaga tagaagatga tgaaccccat 20280
   agcagatcaa agagatcaat tgatactaca agcaatattc gccctagtca attgcttgtt 20340
   aatggacgta ttccgtctac aagttcagct tttgctgtta acgtcgctcg tggtcgagga 20400
   acgattatgc ctcgtcctgg aactggtggc atgggttcgt ccttttctgc tgtttctagg 20460
   tcgggtagta tttcttcctt atcctcggtt ggctcctcaa cacctttgat ctctaattgg 20520
   agaacatctt catctcaact caaaactctc aacctcaaca ttaacactaa aattcctaag 20580
   atttcaacaa agtcaggttt tgccagtatt acatctttgt ttgcttcagg tttaggagtc 20640
   gtcgatctag gtctatctat tttcaacatg atagaacagc gtagagttgc tgagatcact 20700
   cagatgcaaa ttagccaact ggctgactct atagtgtatc ttgctgatgt gacatttgaa 20760
   gctatcaaga atttggaact ctcggttaac tccttgggta cgttcttatc ggaattttcc 20820
   actcagatgt cgatcaccat aagccaaata caatcatcat ttgaagagca gcaagatgct 20880
   acaaatgatg cgttgtacta cactaacgct gctgcgtcat accaagcctc catggcgtat 20940
   gtcatttcag agttaaacgc aatatctctg tctgtcacta gatcctacga ctcttacacc 21000
   agttgcatca cttctggcat taatgggctc attacaccat catgcttgcc agcccaccag 21060
   ttgttacagt tactcgacac cgttatcaat tccacagcag gaacaggatg ccgtcccatc 21120
   tacggcagag aagaagtggt gaaatactac actttacctc taatcaatca aggttattcc 21180
   tttaacgggt cgattttctt cgtctttaac attcccatca cttgccaggg aattgccgga 21240
   gatgtatatg aagtagaacc acctatactt gtagatgtac catcaaagac tgctttacgc 21300
   atgattacac catcaaacgt agtcgcaaca caagcaggat tagctgaatt agatttgcgt 21360
   cattgcgaaa ggtaccataa cgagttccta tgcgattctt cagcattcct ttctacacct 21420
   tcaaaataca tagactgttt aacaaacgca actgactgtt ctttgcaatt catcacacaa 21480
   cacgttccag atccttgcgt ttacacatcg ccagcttctt tatattgtta ttattcaccc 21540
   atatgtgatc aatgtcacat agtagccggt tgtaatgaat ctcagcagta caacttcact 21600
   tctgctgatg gcggcgtagt cttttattcc atacaagaca gagactgtgg ccacttcccc 21660
   cacatcactg ttactacgcc tgcagccata caagaagact tcactgtcgg accgtattta 21720
   ccatcgctgc caattcacac cgcctacgtc aatgttacct ggaatgtaac actaccagga 21780
   aattggacct gggaaaatat caccctaaca gccaattgga cccaacactt cattgagatg 21840
   aaaaaaaaca tcacaatgat ggctgaagaa atagataacc ttaccaactt cggtaaggtt 21900
   ttagttggcc agctaaatag ctttttatca tctttgttta acataccatt aggtttgatg 21960
   acgttttgct tttctgtagc cgctttaggc ctgtccatta ttgctttact tgtgttatgt 22020
   tttccacaga agccacataa attataatcg tggtttcgct tgtaaatatt gatcaattga 22080
   ggttttttac actttagtgt ttttcctcaa ccaattagac cagaggtttt tttacaccaa 22140
   agtgtttttc ctctacaaag aattgaggtt ttttacactc tagtgttttt cctcaaactt 22200
   atatatataa aatttcatta gtttgacatt tcattataaa tagcacaaca aatacattca 22260
   ggcgacttgc atgatgttta ccctagtagt gctttttacc ctcctcggcc tttccatggc 22320
   ctccacagag ctgaatttcg atcctactct acccctcccc tctcctataa atgccctcgt 22380
   cgacattttc ggaaacaaca gcttgtttct caaagagtcc ctgctcggca aatccaccgg 22440
   agccgtctac gcatacttgt acagcagtgc catctctctc ctgctgctac tttgggtaac 22500
   tgtatggagt attgctactt cacactttaa cgtaactcgc attccaacca tcgcggttct 22560
   cactaatgcg agtatgtttt tgctgttggc atcggctact gttacaacct ggtttctccc 22620
   aactgtgacg aacgtcttct tttatacact cactgcgctg ttcaccttct tttcctttgt 22680
   gttcttactg tggttggttt actatatgtt tactaccatt agggcatatc gaagggtcgg 22740
   ttcatggcgc gttgtgttta acggaaaata ttctctactt gctggaactc aggctgtttg 22800
   cctttgcaga cccgccatac atctggttct aaccaaaacg aacacagata catactggtg 22860
   tctagatgga acccccatct acaatgttga cttactacaa ttagttggcc ccaaaggatt 22920
   atatccttac aaaagaatga ctacaatcac tgcaccaaaa ggcacaaaaa catctgctgc 22980
   cgtttacacc cttcaaaaag aagaagtttg tgctctctca gaaatcacag tacataatga 23040
   tactgatttt taggtcatat aaaaaagcta acacatctaa aaaatgtctt acccggttta 23100
   ctacgaacag cgtcgttatt ccccccgcca attcaacaat ggcggagggt ataatcctac 23160
   acctcaacct agagtagttc gtactaatcc tggtaaccaa gcttacaacc cccggcgtaa 23220
   ccgaaacgcc actccgaacc aacaacaaat ggttccttac cagcctcagt atcaagcacc 23280
   tcctcagcca agggtggtct atgtagatcg ccctcaagaa cctgtagtaa tttacagagc 23340
   tcctccacaa ggaaaaaaac aatcaggcaa acgccacaca gcagaagaac gctggtatca 23400
   aggcgaaaaa cctgtgcaga agaaacaggc acccaaagga aaatcaaaga aagcagcaac 23460
   acctgctaat cctaaaaagc agcctacaca atctgacaaa gttcccatcg cctacccaga 23520
   caatcatccc ttccatgacc tcgcaccagc tgacatccgc gctttcaaaa agcagctgat 23580
   ccaaaatctg gaccttggac atggtgaaat gaatcaactg cggctttcaa tcgatctgtt 23640
   gcccatcaag aaaccagcac caacaccagc ggtgccagct cctctgtaat ttatggaaaa 23700
   ggtgcaagac ctcccataaa taagtagata tgttcattac cctcattatg atcttcgcca 23760
   tcttggcttt cccttcaaca tctgaaggag cagcccaaga actactcaaa gctgtaaaat 23820
   ctgctgctat catggaaaag gtgcaagacc tcccatgaaa atagtagccg gtttcaaaag 23880
   ttgataatta ttgcattatg tttacccttg tgcttattat cctgcttagt ttttctatgg 23940
   cttttaatgc ttttacattt ctgctgttat tattttttac ttttaagtgc attataaccc 24000
   gcactttagt cgtagttccc attgactacc cagaaaatca tcctttcaat ggcctctcac 24060
   cagaggaaat catcagctac aaatcacagc tgatccaaaa tctcgatctt ggacatggtg 24120
   aagtaattaa acatcgattc tcaattgatt tacttcccct caaaacaaca agcactccta 24180
   ccaccagtgc tattttatgg aaaaggttca aaacctccca taaagaaaac aaccactaaa 24240
   caaccatgga aaaggtgcaa gacctcccat gaaattagtg gttgctttca aaaattaata 24300
   aatattgtga taaatgtcta tttctaacca cccttaaaaa taggtacccc cactatatct 24360
   agccgacgtt aactcctgga tatgttatag tgttctctcc ccaatcgttc atctgctcgc 24420
   ttttagaact gctaggctgt atctgttaaa tgtttaatct ttagactcaa tttacgttct 24480
   tttttacata aaatcctcct attttgctat cccttatttt aattaaaccc ctttagtatc 24540
   accagtatcc ctaatcactc ccctagcctc ccttgttctg acctgtatga aatgtcaaaa 24600
   aactaaatga aaa 24613

<210> 7
<211> 5867
<212> PRT
<213> Viruses

<220>
<223> ORF-1 amino acid sequence ("X" at positions 830 to 835, 841, 842
      and 3257, means any amino acid)

<400> 7
Met Lys Lys Ile Glu Asp Ala Leu Gly Thr Leu Lys Pro Ala Phe Lys
1               5                   10                  15
Ala His Ile Asp Ser Leu Pro Pro Phe Met Gly Lys Leu Ala Thr Met
            20                  25                  30
Leu Ala Glu Thr Arg Arg Gly Lys Thr Pro Pro Leu Leu Ile Tyr Val
        35                  40                  45
Ile Ser Thr Ile Leu Glu Thr Asn Ile Thr Val His Tyr Val Ser His

50                  55                  60
Thr Ile Thr Ser Tyr His Ser Ser Asn Ala His Thr His Thr His Glu
65                  70                  75                  80
Phe Asp Ser Glu Asp Tyr Thr Pro Asp Asn Gln Ile Leu Thr Lys Val
                85                  90                  95
Asn Arg His Gly Asn Asp Leu Asn His Ser Tyr Ile His Gly Ala Thr
            100                 105                 110
Asn Met Tyr Asn Pro Val Tyr Gln Arg His Pro Pro Asn Met Cys Tyr
        115                 120                 125
Met Leu Thr Gly Leu Tyr Met Leu Ser Gly Leu Gln Glu Leu Tyr Ala
    130                 135                 140
Met Ala Glu Asp Asn Leu Thr Thr Cys Gln Ile Asn Leu Leu Arg Cys
145                 150                 155                 160
Leu Phe Asp Leu Asn Gln Asp Glu Phe Asp Val Asp Tyr Thr Phe Val
                165                 170                 175
Ile Tyr Thr Pro Ser Lys Ser Gln Glu Cys Ala Phe Lys Tyr Leu Gln
            180                 185                 190
Glu Ile Val His His Cys Glu Leu Thr Ile Phe Arg His Thr Thr Thr
        195                 200                 205
Ser Val Phe Ser Cys Asn Lys Cys Asn His Val Glu Thr Val Ile Ser
    210                 215                 220
Ser Cys Ser Leu Asn Leu Val Tyr Ile Thr Asp Ser Ile Glu Lys Ala
225                 230                 235                 240
Phe Gln Pro Thr Val Glu Ala Asn Thr Asp Tyr Met Cys Glu Asn Cys
                245                 250                 255
Gly Leu Arg Asp His Lys Leu Lys Thr Thr Val Thr Asn Pro Asp Leu
            260                 265                 270
Arg Leu Ala Gln Leu Asn Tyr Pro Thr Asp Ser Lys Tyr Thr Ile Phe
        275                 280                 285
Leu Asp Glu Gln Ala Pro Phe Val Phe His Ser Ile Ala Lys His Val
    290                 295                 300
Gly Thr Ala Asn Ser Gly His Trp Ser Ala Leu Asn Val Asn Ser Asp
305                 310                 315                 320
Met Leu Ser Asp Ser Asn Glu Arg Gln His Tyr Tyr Thr Thr Pro Ser
                325                 330                 335
Ile Val Leu Leu Ala Phe Leu Pro Glu Glu Glu Leu Gln Asn Ile Arg
            340                 345                 350
Asn Ser Ser Pro Leu Gln Asp His Gln Pro Asp Asp Val Glu Asp Val
        355                 360                 365
Glu Ser Pro Leu Pro Gly Ser Ile Phe Tyr Thr Thr Asp Asp Ile Phe
    370                 375                 380
Ser Thr Lys Ser Leu Ser Ile Ala His Cys Val Ala Arg Asp Phe His
385                 390                 395                 400
Met Ser Gly Gly Ile Ala Lys Ile Phe Ser Asp Lys Phe Gly Ser Lys
                405                 410                 415
Thr Phe Leu Lys Ser Gln Asn Pro Val Ile Gly Gly Phe Ser Ile Leu
            420                 425                 430
Leu Arg Glu Cys Arg Asp Met Tyr Tyr Leu Val Thr Lys Glu Lys Thr
        435                 440                 445
Ser Asp Lys Pro Thr Tyr Gln Asp Leu Lys Asn Ser Leu Gly Ser Met
    450                 455                 460
Thr Glu Asn Leu Val Arg Lys Asn His Asn Thr Leu Ser Ile Pro Tyr
465                 470                 475                 480
Ile Gly Cys Gly Ile Asp Gly Leu Gln Trp Ala Thr Val Glu Lys Gln
                485                 490                 495
Val Lys Glu Ile Val Cys Ala Arg Gly Ile Asp Val Thr Val His His
            500                 505                 510
Leu Glu Asn Glu Val Lys His Thr Pro Glu Gln Gln Thr Ala Ser Asp
        515                 520                 525
Asn Ser Val Lys Leu Val Gln Lys Leu Phe Thr Glu Thr Pro Gln Ala
    530                 535                 540
Ile Pro Ile Val Val Pro Ser Asp Asp Ser Asp Ser Asp Ile Asp Glu
545                 550                 555                 560

Ser Ala Asp Val Phe Leu Pro Gly Pro Glu Ser Glu Ser Asp Ser Lys
                565                 570                 575
Ser Glu Ser Gly Ser Asp Tyr Asp Phe Lys Ser Ala Ser Glu Pro Glu
            580                 585                 590
Asp Glu Leu Glu Pro Thr Pro Ile Ser Glu Leu Glu Leu Thr Pro Ala
        595                 600                 605
Ser Ser Leu Thr Val Glu Ser Asp Asp Asn Pro Asp Thr Ser Gln Glu
    610                 615                 620
Thr Leu Pro Glu Ser Asn Ser Glu Glu Thr Lys Pro Glu Gln Thr Pro
625                 630                 635                 640
Asp Thr Thr Ser Lys Val Ser Ser Asp Ser Lys Leu Asp Pro Gln Ser
                645                 650                 655
Glu Leu Glu Glu Glu Leu Ala Asn Lys Pro Glu Ser Ala Ser Glu Pro
            660                 665                 670
Gln Ser Glu Thr Glu Ser Ser Ser Glu Ser Glu Glu Glu Leu Glu Pro
        675                 680                 685
Gln Ser Glu Ser Glu Glu Glu Pro Ala Asn Lys Pro Glu Ser Pro Ser
    690                 695                 700
Glu Ser Gln Ser Glu Asn Gly Ser Ser Ser Glu Pro Glu Glu Glu Ser
705                 710                 715                 720
Glu Lys Pro Ser Glu Ser Ala Glu Thr Ala Thr Glu Asp Ser Pro Glu
                725                 730                 735
Thr Thr Pro Glu Thr Thr Leu Glu Leu Thr Thr Gln Leu Lys Pro Ala
            740                 745                 750
Ser Glu Ser Asp Asp Lys Pro Asp Thr Pro Ala Pro Ser Pro Ser Pro
        755                 760                 765
Ile Gln Pro Glu Lys Asn Leu Asp Thr Thr Pro Glu Gln Thr Ser Gln
    770                 775                 780
Pro Thr Thr Gln Leu Glu Leu Thr Leu Glu Thr Gln Glu Gln Pro Asp
785                 790                 795                 800
Thr Thr Pro Glu Val Pro Ser Val Ser Glu Asp Lys Pro Asp Thr Leu
                805                 810                 815
Glu Glu Ser Ser Glu Ser Thr Pro Glu Leu Ser Glu Leu Xaa Xaa Xaa
            820                 825                 830
Xaa Xaa Xaa Thr Ser Thr Lys Thr Xaa Xaa Thr Arg Pro Pro Thr Pro
        835                 840                 845
Arg Pro Arg Ser Ala Arg Gly Ala Arg Thr Arg Ser Cys Ala Gly Thr
    850                 855                 860
Pro Ile Pro Val Ile Phe Asp Ile Ile Asp Asn Thr Ser Gln Pro Gln
865                 870                 875                 880
Val Pro Leu Asp Phe Pro Glu Ala Leu Gln Glu Leu Asn Lys Pro Ser
                885                 890                 895
Glu Val Ile Pro Ala Ala Ser Glu Lys Pro Val Glu Lys Gln Ile Ile
            900                 905                 910
His Ser Phe Val Ser Val Glu Thr Pro Cys Lys Pro Lys Ala Thr Lys
        915                 920                 925
Val Thr Asn Tyr Val Ala Ala Gln Ser Asn Ala Ile Leu Asn Cys Ile
    930                 935                 940
Lys Ala Phe Ile Pro Ser Asn Pro Leu Ser Leu Phe Asn Arg Lys Pro
945                 950                 955                 960
Ala Phe Arg Lys Ile Ile Phe Thr Glu Asp Thr Ser Glu Pro Asp Ser
                965                 970                 975
Asp Asp Asp Asp Cys Glu Tyr Thr Pro Pro Thr Ser Pro Phe Pro Glu
            980                 985                 990
Leu Leu Ala Leu Val Asp Glu Asp Ile Glu Val Glu Gln Thr Gln Ser
        995                 1000                1005
Val Ile Pro Lys Thr Asp Ser Ala Ser Ile Val Glu Asp Leu Lys Lys
    1010                1015                1020
Gln Glu Ser Ser Thr Leu Ser Leu Asp Thr Asn Thr Ser Lys Pro Thr
1025                1030                1035                1040
Ser Ser Pro Arg Arg Gln Pro Arg Glu Val Glu Ser Val Asp Glu Ser
                1045                1050                1055
Ser Asp Asp Ser Ser Lys Pro Lys Thr Ile Ser Thr Leu Asp Lys Pro

1060                1065                1070
Ala Met Asn Ser Asp Thr Lys Pro Thr Asp Ser Ser Arg Lys Glu Pro
        1075                1080                1085
Leu Glu Val Pro Val Thr Thr Ser Leu Ser Thr Pro Ala Lys Asn Gln
    1090                1095                1100
Asp Lys Lys Ser Ser Lys Ser Ala Lys Val Ile Lys Asp Tyr Ser Leu
1105                1110                1115                1120
Thr Pro Asn Thr Val Lys Gln Gln Val Tyr Ser Leu Tyr Gly Glu Ser
                1125                1130                1135
Val Asp Ala Val Lys Tyr Leu Val Gln Thr Tyr Pro Asp Arg Ala Lys
            1140                1145                1150
Gln Thr Ala Gly Ile Ala Tyr Phe Leu Ile Thr Thr Tyr Leu Ile Trp
        1155                1160                1165
Thr Ile Gly Leu Ile Gly Val Pro Met Ala Phe Lys Ile Pro Met Phe
    1170                1175                1180
Leu Cys Leu Leu Tyr Gln Val Asn Gly Leu Asn Ile Ala Pro Phe Val
1185                1190                1195                1200
Thr Asn Gln Lys Leu Gln Tyr Val Ala Phe Pro Leu Trp Tyr Lys Leu
                1205                1210                1215
Tyr Glu Val Ile Ser Val Arg Phe Val Ala Asn Ile Ala Gln Phe Ile
            1220                1225                1230
Val Lys Thr Pro Pro Ile Asp Val Leu Asn Lys Leu Ile Arg Ser Asn
        1235                1240                1245
Lys Asp Lys Pro Val Lys Leu Thr Pro Asn Lys His Thr Leu Met Leu
    1250                1255                1260
Ile His Asp Leu Ala Leu Glu Ser Val Asp Gly Lys Glu Asn Arg Tyr
1265                1270                1275                1280
Tyr Asn Thr Asp Val Thr Thr Phe Thr Lys Arg His Ser Thr Ser Asn
                1285                1290                1295
Ile Ser Tyr Val Leu Lys Ser Thr Leu Ile Lys Tyr Val Met Asp His
            1300                1305                1310
Cys Tyr Val Asn Ile Ala Val Phe Thr Leu Val Arg Tyr Leu Thr Leu
        1315                1320                1325
Leu Val Phe Ile Gln His Phe Ser Asn Pro Tyr Val Leu Glu Ala Asn
    1330                1335                1340
Ser Gln Ser His Thr Val Leu Gln Tyr Leu Phe Ser His Leu Arg Pro
1345                1350                1355                1360
Phe Gly Arg Pro Leu Cys Pro Thr Leu Asn Asp Tyr Met Thr Thr Ala
                1365                1370                1375
Thr Pro Arg Asp Ala His Val Gln Ala Gly Ser His Phe Ser Glu Phe
            1380                1385                1390
Cys Val Pro Ile His Tyr Thr Thr Pro Ile Ile Lys Ser Thr Met Ala
        1395                1400                1405
Glu Pro Ser Leu Phe Leu Leu Phe Asn Pro Val Leu Trp Pro Leu Val
    1410                1415                1420
Met Val Val Tyr Phe Tyr Pro Pro Met Met Phe Ile Ala Asn Ala Val
1425                1430                1435                1440
Ala Tyr Ser Cys Leu Pro Leu Val Val Leu Leu Gln Trp Leu Tyr Ala
                1445                1450                1455
Met Trp Phe Ser Cys Thr Cys Tyr Gly Thr Lys Arg Cys Ala Lys His
            1460                1465                1470
Leu His Lys Asn Glu Val Val Lys Pro Met Glu Ser Thr Ser Thr Lys
        1475                1480                1485
Asn Arg Met Thr Phe Thr Pro Ser Thr Thr Phe Cys Ser Lys His Asn
    1490                1495                1500
Phe Phe Cys Pro Asp Ala Pro His Ile Met Thr Leu Ala Met Ala Arg
1505                1510                1515                1520
Gln Leu Thr Asn Tyr Tyr Asn Leu Thr Asp Thr Val Ile Pro Asp Ile
                1525                1530                1535
Gln Glu Tyr Ser His Glu Asn Pro Thr Val Gln Phe Ile His Phe Asp
            1540                1545                1550
Pro Leu Lys His Gly Ala Asp Thr Ile Leu Glu Pro Ile Thr Ser Ala
        1555                1560                1565

Ser Ala Ser Ser Ile Val Ala Trp Tyr Ser Leu Leu Phe Asn Gln Lys
    1570                1575                1580
Phe Val Leu Ser His Tyr Ser Tyr Arg Thr Pro Val Ala Val Val Asp
1585                1590                1595                1600
Lys Pro Glu Glu Thr Asp Gly Asp Asp Thr Lys Ser Leu Ala Ser Asp
                1605                1610                1615
Thr Ser Asp Asn Phe Glu Ser Ile Arg Lys Thr Asn His Lys Asn Gln
            1620                1625                1630
Ser Lys Gln Gln Phe Arg Pro Asn Gly His Gln Arg Pro Ser Lys Thr
        1635                1640                1645
Phe Lys Arg His Ser Arg Ile Met Thr Ser Glu Gln Lys Asn Ser Leu
    1650                1655                1660
Ile Glu Thr Phe Lys Gly Leu Thr Asn Gly Thr Ala Ala Ile Pro Gln
1665                1670                1675                1680
Pro Leu Ile Ile Phe Ile Trp Val Ile Leu Met Val Ile Pro Thr Leu
                1685                1690                1695
Phe Leu Val Ala Ser Ser Ser Arg Thr Ala Ala Thr Met Pro Leu Asn
            1700                1705                1710
Arg Tyr Ser Gly Val Asn Pro Thr Gly Ile Met Phe His Gln Ala Pro
        1715                1720                1725
Pro Tyr Ile His Ser Glu Pro Pro Lys Glu Thr Tyr Tyr Lys Leu Ser
    1730                1735                1740
Tyr Pro Tyr Pro Ser Ala Thr Val Val Arg Thr Leu Lys Gly His Leu
1745                1750                1755                1760
Tyr Tyr His Ser Asp Asp Thr Val Gln Gln Asn Cys Thr Met Gln Tyr
                1765                1770                1775
Ser Leu Ile Ala Ala Ser Thr Lys His Val Cys Gly Lys Val Val Tyr
            1780                1785                1790
Thr Ile Pro Ala His Val Ser Ile Gly Ser Leu Lys Leu Leu Leu Val
        1795                1800                1805
His Pro Asp Gln Thr Asn Leu Pro Phe Glu Leu Pro Val Ser Asp Glu
    1810                1815                1820
Val Arg Leu Cys Tyr Leu Thr Thr Leu Asn Ala Pro Arg Cys Met Pro
1825                1830                1835                1840
Ser Gln Leu Ala Met Ser Asn Asn Gln Phe Ala Ala Val Ser Leu Val
                1845                1850                1855
Leu Leu Ile Thr Leu Val Ser Leu Ile Lys Val Tyr Ile Met Phe Phe
            1860                1865                1870
Thr Val Phe Lys His Tyr Thr Thr Thr Val Phe Ile Leu Val Ala Val
        1875                1880                1885
Thr Thr Ile Thr Met Leu Val Ser Phe Leu Ala Pro Pro Leu Leu Ile
    1890                1895                1900
Val Val Leu Leu Ser Leu Ala Trp Leu Trp Tyr Gly Asn Thr Ile Val
1905                1910                1915                1920
Leu Cys His Ile Met Leu Leu Ile Val Leu Val Val Ser Trp Lys Val
                1925                1930                1935
Ala Ala Val Cys Phe Ile Phe Ala Leu Leu Tyr Phe Gly Lys Cys Ala
            1940                1945                1950
Met Leu Ser Lys Asn Ile Lys Tyr Val Gln Gly Gly Val Lys Phe Ser
        1955                1960                1965
Gly Thr Phe Glu Glu Ile Ala Gln Ser Thr Phe Phe Ile Asn Tyr Gly
    1970                1975                1980
Val Ala Cys Gln Leu Leu Glu His Thr Gly Gln Thr Ile Glu Asp Ile
1985                1990                1995                2000
Met Gln Leu Arg Thr Ala Gly Gly Ala Pro Ala Arg Leu Ala Arg Ser
                2005                2010                2015
Ile Tyr Asp Cys Phe Ser Thr Asn Ala Ser Val Leu Tyr Ser Pro Arg
            2020                2025                2030
Ser Phe Ser Pro Gln Ser Leu Ile Thr Lys Tyr Leu Tyr Pro Gly Ser
        2035                2040                2045
Ile Pro Val Gly Arg Ala Pro Val Leu Leu Gly Lys Ile Ser Gly Met
    2050                2055                2060
Thr Cys Leu Gly Arg Glu Gln Ser Thr Cys Phe Gln Ser Ser Ala Thr

2065                    2070                    2075                    2080
Thr Ile Thr Thr Cys Thr His Ala Val Asn Thr Ala Gly Thr Phe Met
                2085                    2090                    2095
Ser Gln Ile Lys Cys Val Ile Asp Asn Lys Ile Tyr Thr Val Gln Pro
            2100                    2105                    2110
Glu Asn Ile Thr Ile Thr Gly Met Lys Ala Thr Phe Glu Val Glu Gly
        2115                    2120                    2125
Leu Pro Pro Phe Thr Asn Asp Val Thr Val Ala Pro Lys Pro Leu Lys
    2130                    2135                    2140
His Tyr Met Asp Gly Lys Arg His Leu Val Leu Tyr Thr Lys Ser Glu
2145                    2150                    2155                    2160
Ser Ile Val Tyr Ser Ser Ile Met Trp Pro Thr Glu Asn Gly Leu Phe
                2165                    2170                    2175
Ser Ser Ser Val Ser Asp Pro Gly Asp Ser Gly Ala Pro Tyr Phe Ser
            2180                    2185                    2190
Asp Asn Val Ile Val Gly Ile His Gln Gly Arg Asn Glu Ala Thr Asn
        2195                    2200                    2205
Asn Pro Ala Ile Leu Ala Ser Gly Met Asp Gly Glu Ser Pro Cys Val
    2210                    2215                    2220
Gly Tyr Asp Asp Gln Ser Tyr Gly Leu Pro Leu Gln Glu Tyr Phe Thr
2225                    2230                    2235                    2240
His Ile Val Leu Ser Asn Lys Pro Ser Asp Phe Gly Ala Pro Ser Asn
                2245                    2250                    2255
Val Ala Pro Asn Lys Tyr Tyr Asn Lys Lys Ser Phe Glu Gln Leu Ala
            2260                    2265                    2270
Asp Glu Asp Lys Thr Tyr Leu Asn Ser Leu Ser Tyr Pro Leu Ser Ser
        2275                    2280                    2285
Ser Asn Tyr Cys Tyr Phe Asn Ser Phe Lys Thr Gln Ser Ser Thr Thr
    2290                    2295                    2300
Met Leu Asp Asn Ala Glu Val Ile Lys Tyr Val Val Leu Leu Leu Met
2305                    2310                    2315                    2320
Ile Leu Asp Tyr Phe Phe Ser Ile Ile Cys Glu Asp Ala Leu Asn Pro
                2325                    2330                    2335
Ala Ser Tyr Ala Met Leu Val Ile Val Leu Val Gln Ala Phe Ile Thr
            2340                    2345                    2350
Lys Ile Thr Val Phe Arg Thr Gly Ile Tyr Ile Gln Ala Ala Val Phe
        2355                    2360                    2365
Gln Ala Phe Ile Val Pro Ile Val Ser Gln Ile Thr Leu Ile Leu Ala
    2370                    2375                    2380
Ala Asp Thr Ala Arg Ser Phe Leu Thr Phe His Phe Phe Val Leu Ala
2385                    2390                    2395                    2400
Val Leu Thr Tyr Phe Val Leu Cys Arg Ile Ala Val Asp Phe Trp Arg
                2405                    2410                    2415
Ser Met Phe Leu Leu Phe Leu Thr Ser Val Phe Ala Thr Ile Ile Trp
            2420                    2425                    2430
Thr Thr Lys Asn Asp Phe Asn Ile Leu His Glu Thr Gly Val Val Leu
        2435                    2440                    2445
Thr Pro Thr Ala Glu Leu Ala Leu Ile Val Ala Phe Thr Tyr Ile Ile
    2450                    2455                    2460
Tyr Ala Ser Cys Met Leu Thr Pro Val Pro Leu Tyr Thr Ile Cys Val
2465                    2470                    2475                    2480
Phe Phe Ser Phe Leu Ser Asn Ala Pro Leu Tyr Leu Ala Val Leu Ser
                2485                    2490                    2495
Phe Gly Ile Leu Val Ser Phe Lys Thr Asn Gln Asp Phe Gly Arg Leu
            2500                    2505                    2510
Val Asp Lys Val Phe Ser Leu Asn Met Leu Tyr Glu Tyr His Ala Tyr
        2515                    2520                    2525
Gln Asn Tyr Val Ile Gln Asn Ser Gly Gln His Pro Gly Phe Tyr Arg
    2530                    2535                    2540
Ser Leu Phe Ala Phe Phe Ile Asn Leu Thr Thr Gln Pro Lys Thr Thr
2545                    2550                    2555                    2560
Tyr Lys Cys Phe Lys Pro Gln Thr Ala Ser Gly Tyr Arg Val Ile Tyr
                2565                    2570                    2575

Gln Thr Pro Thr Thr Glu Phe Asn Lys Ser Leu Gln His Ala Ser Ile
            2580                2585                2590
Thr Lys Asp Asp Asn Ser Asn His Ile Ile Met Phe Ala Asp Gly Ser
        2595                2600                2605
Ser Asp Asn Leu Asn Trp Ala Lys Glu Met Val Ala Thr Ile His Leu
    2610                2615                2620
Thr Asn Pro Asn Leu Gln Pro Leu Ile Ile Gly Tyr Tyr His Asn Ser
2625                2630                2635                2640
Met Asp Val Ile Thr Lys Gly Thr Tyr Met Gln His Glu Phe Ile Lys
                2645                2650                2655
Met Pro Ala Val Ile Leu Thr Gln Asp Pro Leu Thr Glu Pro Ile Ser
            2660                2665                2670
His Leu Ala Ala Ala Ala Phe Thr Ser Ile Ser Gly Lys Pro Gln Ala
        2675                2680                2685
Gln Lys Asn Asn Val Val Ser Asn Ser Lys Ala Arg Ile Asn Thr Ala
    2690                2695                2700
Val His Asp Ala Val Glu Ser Val Tyr Ser Gly Glu Thr Tyr Val Ala
2705                2710                2715                2720
Pro Lys Pro Ile Val Ser Gly Lys Thr Val Val Glu Lys Pro Phe Ser
                2725                2730                2735
Thr Thr Glu Thr Thr Met Tyr Ile Met Arg Gly Leu Pro Gly Ser Gly
            2740                2745                2750
Lys Ser Phe Lys Val Ser Gln Leu Val Ala Lys Asp Pro Asn Leu Val
        2755                2760                2765
Val Ala Ser Ala Asp His Phe Arg Tyr Ser Asn Asp Lys Thr Gly Lys
    2770                2775                2780
Ala Val Tyr Thr Tyr Ile Pro Glu Ala Thr Ser Ser Val His Leu Gln
2785                2790                2795                2800
Cys Gln Asn Arg Ala Arg Lys Ala Leu Glu Asn Gly Gln Ser Val Cys
                2805                2810                2815
Ile Asp Asn Thr Asn Leu Thr Leu Leu Glu Met Arg Pro Tyr Val Leu
            2820                2825                2830
Leu Ala Arg Ser Phe Asn Tyr Asn Ile Glu Phe Ile His Ser Asp Ser
        2835                2840                2845
Pro Trp Ala Leu Asn Leu Asp Leu Leu His Ala Lys Gly Val His Asn
    2850                2855                2860
Val Pro Arg Ala Lys Leu Val Ile Met Tyr Asp Arg Phe Phe Asp Arg
2865                2870                2875                2880
Asp Asn Gln Ile Asp Ala Asp Ser Leu Ile Gln Tyr Val Ile Glu Ala
                2885                2890                2895
Ile Asp Pro Lys Leu Val Ala Pro Ile Met Asn Arg Phe Pro Ala Asp
            2900                2905                2910
Cys Asp Leu Ile Leu Gln Ser Ala Leu Thr Pro Asp Leu Glu Val Leu
        2915                2920                2925
Lys Gln Asn Tyr Asp Arg Ala Asn Ala Thr Tyr Gln Asp Val Ser Leu
    2930                2935                2940
Asp Asp Pro Pro Ala Leu Lys Ala Ala Arg Arg Ala Met Asn Ile Ala
2945                2950                2955                2960
Lys Ser Glu Tyr Glu Ala Gly Glu Ala Gly Gln Arg Arg Ile Glu Lys
                2965                2970                2975
Phe Leu Glu Arg Gln Asp Val Ala Ala Leu Asn Gln Thr Leu Thr Thr
            2980                2985                2990
Val Asn Gln Ser Lys Phe Ile Ala Ala Ile Arg Ser Ile Tyr Leu Ser
        2995                3000                3005
Thr Ile Ser Asn Leu Arg Leu Lys Thr Arg His Met Gly Glu Gly Ser
    3010                3015                3020
Tyr Ala Val Thr Ser Gly Thr Asn Thr Thr Asp Lys Val Leu Val Asn
3025                3030                3035                3040
Thr Pro Gln Arg Met Thr Arg Ile Glu Asp Gly Ile Tyr Lys Leu Val
                3045                3050                3055
Ala Asn Gly Phe Glu Ile Thr Met Cys Asp Gly Ser Asn Leu Ala Gly
            3060                3065                3070
Val Thr Phe Glu Gln Asp Ile Asn Pro Ser Met Tyr Pro Phe Val Phe

3075                3080                3085
Thr Leu Met Ser Asn Ile Ala Val Pro Val Leu Thr Arg Gln Ala Asn
    3090                3095                3100
Val Gly Tyr Leu Asp Met Ser Asn Lys Phe Ile Cys Lys Asp Gly Thr
3105                3110                3115                3120
Val Gln Phe Gln Gly Val Ile Tyr Ala Tyr His Thr Pro Ser Asn Glu
                3125                3130                3135
Ser Ala Asp Phe Lys Val Gly Asn Thr Ser Trp Thr Leu Gln Lys Asn
            3140                3145                3150
Ile Asn Leu Thr Ala Phe Ile Pro Ala Ile His Lys Thr Ala Thr Phe
        3155                3160                3165
Ala Ala Gln Ser Val Phe Leu Gly Gly Leu Pro Met Glu Glu His Gln
    3170                3175                3180
Ala Phe Ser Asp Thr Pro Thr Ala Ser Asn Lys Phe Lys Val Phe Val
3185                3190                3195                3200
Ser Ser Thr Val Cys Ala Ser Thr Val Cys Lys Val Asn His Lys Thr
                3205                3210                3215
Tyr Val Gln Ile Pro Asp Asp Ile Gln Asp Pro Phe Thr Tyr Met His
            3220                3225                3230
His Ser Val Cys Ser His Asn Lys Phe Leu Ser Asn His Glu Thr Arg
        3235                3240                3245
Cys Gln Ile Cys Pro Leu Asn Cys Xaa Ser Ala Asn Pro Cys Val Ser
    3250                3255                3260
Thr Ala Cys Ala Leu Phe Asp Asn Gly Thr Leu Pro Arg Ser Thr His
3265                3270                3275                3280
Tyr Ile Asn Val Ser Thr Thr Ser Asn Val Gly Leu Phe Lys Ala Val
                3285                3290                3295
Lys Lys Ser Thr Arg Gln Leu Asn Ile Asp Gly Phe Pro Tyr Met Leu
            3300                3305                3310
Lys Gln Val Lys Asp Asp Ser Glu Leu Val Ser Ser Leu Lys Ile Gly
        3315                3320                3325
Leu Pro Asn Ile Leu Pro His His Met Val Glu Thr Lys Ser Lys Thr
    3330                3335                3340
Tyr Leu Leu Arg Gly Pro Thr Thr Ala Tyr Ser Leu Gly Asp Leu Cys
3345                3350                3355                3360
Tyr Ala Leu Phe Asn Gly Asp Phe Asp Tyr Ile Arg Glu Asn Ile Asn
                3365                3370                3375
Ser Asp Phe Val Leu Asp Arg Glu Ala Gly Met Pro Asp Thr Glu Thr
            3380                3385                3390
Arg Thr Trp Leu Phe Ser Ile Leu Asn Phe Ala Val Pro Arg Val Cys
        3395                3400                3405
Ala Ile Ile Asp Gln Met Ile Ser Glu Asn Val Phe Tyr Lys Leu Thr
    3410                3415                3420
Leu Asp Asn Leu Asp Leu Tyr Gly Ser Leu Tyr Asp Phe Asp Asp Tyr
3425                3430                3435                3440
Pro Thr Glu Gly Phe Asn Arg Pro Asp Asp Val Ile Arg Met Leu Lys
                3445                3450                3455
Glu Ile Trp Ser Phe Cys Arg Arg Pro Leu Pro Ala Asp Leu Leu Lys
            3460                3465                3470
Tyr His Glu Asp Ile Gly Ala Ala Ala Thr Gln Glu Ile Leu Leu His
        3475                3480                3485
Ala Pro Phe Ile Asp Lys Val Cys Ala Leu Asn Asp Arg Leu Ala Val
    3490                3495                3500
Val Asp Asn Arg Ala Ser Gln Tyr Phe Phe Cys Glu Glu Glu Gly Val
3505                3510                3515                3520
Phe Thr His Ile Tyr Asn Pro Val Tyr Gly Thr Leu Ala Phe Asp Asn
                3525                3530                3535
Lys Leu Ile Gln Ser Lys Asp Pro Ser Cys Thr Leu Gln Arg Leu Ile
            3540                3545                3550
Thr Ile Gln Gly Pro Leu Ser Thr Asn Ala Ser Pro Val Ile Ser Ile
        3555                3560                3565
Ser Asp Ser Thr His Ile Ala Asn Asn Ile Asn Pro Ser Asn Gln Lys
    3570                3575                3580

Thr Thr Pro Leu Tyr Tyr Asp Leu Glu Leu Ala Gln Glu Phe Ile Asp
3585                3590                3595                3600
Ala Gly Leu Asn Ile Asp Gly Val Ser Asn Tyr Phe Phe Tyr Gly Pro
                3605                3610                3615
Ser Arg Ala Gly Val Val Ser Asp Phe Leu Leu Tyr Glu Phe Gln Gly
            3620                3625                3630
Thr Gln Trp Phe Asp Asn Asn Met Leu Arg Ser Leu Tyr Ser Phe Ile
        3635                3640                3645
Leu Lys Asn Ser Glu Cys Tyr Arg Thr Thr Asp Gln Leu Asp Phe Arg
    3650                3655                3660
Gly Gly Lys Pro Arg Lys Ser Ser Met Gly His Gly Val Thr Gly Phe
3665                3670                3675                3680
Lys Gln Asp Val Val Tyr Ala Ala Leu Gly Pro Asp Met Ile Glu Thr
                3685                3690                3695
Leu Tyr Glu Thr Ala Lys Gln Thr Pro Leu Pro Phe Cys Thr Lys Ile
            3700                3705                3710
Thr Ala Lys Tyr Ala Leu Thr Ala Lys Pro Arg Ala Arg Thr Val Ala
        3715                3720                3725
Ala Cys Ser Phe Val Ala Ser Thr Ile Phe Arg Tyr Ala His Lys Pro
    3730                3735                3740
Leu Thr Asn Asn Met Val Ser Lys Ala Gln Gln Gly Leu Gly Tyr Cys
3745                3750                3755                3760
Leu Ile Gly Ile Ser Lys Phe His Gly Arg Phe Asn Lys Phe Val Lys
                3765                3770                3775
Ser Arg Val Gly Thr Val Glu Asp Phe Asn Val Phe Gly Ser Asp Tyr
            3780                3785                3790
Thr Lys Cys Asp Arg Thr Phe Pro Leu Ala Leu Arg Ala Leu Ser Ala
        3795                3800                3805
Ala Leu Ile Phe Asp Leu Gly Gly His Asp Pro Asp Asn Cys Leu Phe
    3810                3815                3820
Ile Asn Glu Leu Asn Ala Tyr Met Leu Asp Ile Val Ser Val Glu Asp
3825                3830                3835                3840
Ser Phe Ala Asn Lys Pro Gly Gly Thr Ser Ser Gly Asp Ala Thr Thr
                3845                3850                3855
Ala Tyr Ser Asn Thr Leu Tyr Asn Phe Ala Val His Tyr Ile Ile Met
            3860                3865                3870
Trp Lys Thr Phe Leu Thr Val Asn Asp Pro Ser Thr Lys Val Ile Arg
        3875                3880                3885
Ser Ala Ala His His Ala Leu Thr Ser Gly Asp Phe Ser Met Tyr Asn
    3890                3895                3900
Asp Met Ile Gln Asp Met Leu Asp Val Asp Tyr Thr Leu Asn Phe Leu
3905                3910                3915                3920
Ser Asp Asp Ser Tyr Ile Cys Ser Lys Pro Ser Ala Phe Pro Ile Phe
                3925                3930                3935
Thr Leu Glu Asn Tyr Pro Ser Lys Leu Gln Ser Ile Leu His Thr Ala
            3940                3945                3950
Val Asp Ser Lys Lys Ser Trp Glu Ala Lys Gly Glu Ile Lys Glu Phe
        3955                3960                3965
Cys Ser Ser His Ile Val Asn Val Asp Gly Asp Tyr His Phe Lys Pro
    3970                3975                3980
Glu Lys Asp Arg Ile Leu Ala Ser Leu Leu Ile Leu Ser Lys Ile Ala
3985                3990                3995                4000
Asp Met Asp Ile Phe Phe Met Arg Phe Val Ala Leu Leu Ala Glu Ser
                4005                4010                4015
Ala Val Tyr Ile Arg Ile Asp Pro Thr Phe Trp Leu Ala Leu Phe Gly
            4020                4025                4030
Val Phe Glu Asn Arg Val Thr Ala Phe Lys Ser Glu Thr Leu Leu Ser
        4035                4040                4045
Pro Val Pro Glu Gln Leu Met Lys Val Ala Phe Tyr Glu Ser Leu Val
    4050                4055                4060
Phe Ala Asp Val Asp Ala Thr Ala Leu Tyr Gly Phe Leu Asp Gly Phe
4065                4070                4075                4080
Lys Met Gln Ser Gln Thr Leu His Pro Asp Gly Val Glu Gly Phe Asp

4085                4090                4095
Lys Gln Ser Asp Arg Val Lys His Cys Phe Ala Cys Asp Asn Ile Ser
            4100                4105                4110
Val Gly His Cys Ser Ile Cys Pro Val Pro Leu Pro Leu Cys Ser Phe
        4115                4120                4125
Cys Phe Tyr Glu His Ala Leu Leu Asn Glu His Tyr Glu Ala Ser Gly
    4130                4135                4140
Ile Ala Cys Glu Cys Gly Asp Ala Asp Ile Arg Gln Leu His Leu Lys
4145                4150                4155                4160
Ile Thr Asn Gln Pro Ser Ser His Asn Phe Ile Cys Ala Glu Cys Pro
                4165                4170                4175
Thr Val Ala Met Lys Leu Pro Ile Phe Asn Ser Phe Gln Gly Lys Val
            4180                4185                4190
Leu Leu Pro Met Phe Arg Met Asn Thr Pro Leu Pro Ser Ser Val Ser
        4195                4200                4205
Val Ile Val Asp Val Arg Ser Asn Pro Lys Ala Pro Lys Met Leu Trp
    4210                4215                4220
Asp Asp Val Gln Asn Phe Arg Glu Asn Cys Thr Arg Ile Ala Tyr Glu
4225                4230                4235                4240
Ser Val Ser Cys Ala Glu Leu Ala Arg Glu Val Val Tyr Tyr Pro Tyr
                4245                4250                4255
Glu Val Ile Glu Ser Lys Ala Gly Gln Ala Arg Leu Arg Ile Gln Asn
            4260                4265                4270
Phe Lys Cys Ser Pro Thr Thr Tyr Val Gln Phe Tyr Lys Val Arg Gln
        4275                4280                4285
Asn Gly Lys Tyr Cys Leu Val Ala Lys Ala Thr Leu Thr Pro Ala Phe
    4290                4295                4300
Glu Asn Gln Thr Asp Ile Phe Ser Val Phe Gln Pro Asn Asn Phe Ser
4305                4310                4315                4320
Pro Trp Asn Thr Ser Ser Val Phe Ala Val Glu Gln Tyr Ala Ala Ile
                4325                4330                4335
Tyr Pro Pro Ile Pro Lys Glu Pro Val Asn Ala Thr Phe Val Leu Gly
            4340                4345                4350
Pro Pro Gly Cys Gly Lys Thr Tyr Tyr Ile Ala Lys Thr Tyr Phe Ser
        4355                4360                4365
Gln Ala Ser Glu Thr Cys Pro Val Val Tyr Cys Ala Pro Thr His Arg
    4370                4375                4380
Leu Val Leu Asp Met Asp Ala Glu Tyr Ser Gly Val Val Ser Lys Ser
4385                4390                4395                4400
Leu Tyr Asn Asn Arg Val Tyr Lys Asn Pro Ala Tyr Lys Thr Gly Glu
                4405                4410                4415
Pro Phe Lys Leu Cys Phe Thr Thr His Asn Thr Met Pro Val Gln Lys
            4420                4425                4430
Lys Ala Ile Leu Ile Ile Asp Glu Val Ser Leu Ile Thr Pro His Ser
        4435                4440                4445
Leu Phe Ser Ile Ile Gly Lys Gly Phe Tyr Glu Ile Val Leu Val Gly
    4450                4455                4460
Asp Pro Phe Gln Leu Ser Ala Val Phe Pro Gly Phe Val Val Asn His
4465                4470                4475                4480
Thr Tyr Asp Gly Phe Tyr Ile Arg Arg Leu Val Asn Lys Val Lys His
                4485                4490                4495
Leu Thr Val Cys Tyr Arg Cys Pro Gln Glu Ile Leu Asp Ile Phe Ser
            4500                4505                4510
Lys Pro Tyr His Asp Val Gly Ile Asp Leu Thr Thr Gly Asn Thr Asn
        4515                4520                4525
Pro Gly Lys Ala Ser Ile Tyr Thr Leu Asn Trp Leu Gln Ala Asp Val
    4530                4535                4540
Gly Thr Lys Asn Pro Asp Lys Leu Arg Gln Leu Phe Ala Gln Tyr Pro
4545                4550                4555                4560
Gly Phe Lys Ile Ile Thr Asn Tyr Arg Cys Val Val Asp Ala Ala Lys
                4565                4570                4575
Ser Tyr Gly Ile Asn Val Glu Thr Ile Asp Ser Ser Gln Gly Thr Thr
            4580                4585                4590

Gly Asp Arg His Leu Val Val Ile Cys Gly Ser Thr Asn Phe Ser Lys
        4595                4600                4605
Leu Leu Asn Arg Phe Ile Val Ala Ala Ser Arg Ser Thr Thr Glu Leu
    4610                4615                4620
Val Ile Val Met Leu Pro Glu Leu Tyr Asn Tyr Leu Thr Glu Thr Phe
4625                4630                4635                4640
Asn Phe Lys Pro Leu Gln Leu Gln Asn Val His Val Pro Ile Ala Val
                4645                4650                4655
Ser Ser Thr Ala Phe Cys Asp Ile Glu Phe Tyr His Phe Gln Lys Lys
            4660                4665                4670
Phe Tyr Val Gly Glu Ile Ser Val Ser Thr Ser Thr Thr Met Thr Cys
        4675                4680                4685
Gln Leu Gly Cys Tyr Ile Asn Gly Ser Tyr Met Leu Pro Pro Val Leu
    4690                4695                4700
Glu Asn Ser Glu Asp Arg Leu Tyr Val Pro Ser Arg Trp Arg Arg Met
4705                4710                4715                4720
Ile Arg Lys Tyr Pro Thr Glu Ser Met His Ile Ser Leu Leu Asp Arg
                4725                4730                4735
Leu Leu Arg His Ile Leu Leu Thr Thr Thr Gly Glu Ile His Phe Val
            4740                4745                4750
Met Phe Ser Ala Asp Asn Asp Leu Ile Ala Leu Asp Pro Tyr Phe Ile
        4755                4760                4765
Pro Pro Thr Leu Cys Glu Cys Gly Ser Ala Gly Leu Val Glu Val Asp
    4770                4775                4780
Ile Thr Val Phe Cys Arg Asn Cys Leu Pro Lys Asp Gly Lys Ala Thr
4785                4790                4795                4800
Arg Leu Val Lys Pro Ser Thr Leu Asp Val Gln Thr Glu Lys Leu Arg
                4805                4810                4815
Leu Ala Lys Val His Ala Lys Val Cys Lys Ile Lys His Gly Ser Ala
            4820                4825                4830
His Asn Ala Asp Val Asp Ala Ile Met Thr Gln Cys Ile Tyr Ala Asn
        4835                4840                4845
Ser Leu Thr Phe Thr Pro Thr Thr Gln Leu Val Val Asn Thr Asp Glu
    4850                4855                4860
Phe Thr Phe Tyr Met Leu Pro Arg Pro Ser Asn Arg His Leu Arg Ile
4865                4870                4875                4880
Ile His Lys Asn Asp Lys Arg Phe Tyr Ala Ile Thr His Glu Glu Glu
                4885                4890                4895
Asp Leu Phe Phe Thr Asn Ile Ser Ala Val Val Asp Pro Ile Pro Ala
            4900                4905                4910
Lys Phe Asn Ile Ala His Ser Thr Ser Phe Leu Thr Ile Lys Ser Gly
        4915                4920                4925
Cys Ala Gly Asn Lys Thr Cys Thr Arg Cys Tyr Tyr Leu His Leu Ala
    4930                4935                4940
Tyr Thr Glu Phe Val Ser Gln His Lys Tyr Glu Pro Phe Thr Cys Val
4945                4950                4955                4960
Ser Phe Lys Ile Arg Phe Asp Phe Ser Gln Phe Thr Asp Ser Val Asp
                4965                4970                4975
Thr Phe Leu Arg Gln Gly Leu Ile Thr Phe His Pro Glu Met Asn Ser
            4980                4985                4990
Leu Gln Lys Ser Leu Leu Leu Ala Val Asp Lys Val Tyr Cys Asp Asn
        4995                5000                5005
Phe Thr Ser Asn Gly Arg Arg Phe Arg Leu Tyr Asp Asn Asn Leu Val
    5010                5015                5020
Lys Ser Ile Ile Lys Gly Ser Val Ala Gln Asn Ser Ile Ile Met Pro
5025                5030                5035                5040
Leu Asp Ser Val Leu His Gly Leu Asn Ile Asp Phe Thr Val Gly Cys
                5045                5050                5055
Ala Val Asp Asn Phe Ser Cys Lys Glu Ala Ala Ser Val Arg Tyr Ser
            5060                5065                5070
Glu Val Val Leu Ser Ile Thr Lys Leu Pro Pro Gly Thr Cys Gln Leu
        5075                5080                5085
Tyr Tyr Val Ile Ser Tyr Gly Leu Asn Ser Pro Lys Thr Thr Tyr Ala

5090                    5095                    5100
Gly His Gln Leu Phe Asp Gly Phe Glu Thr Val Ile Val Val Asn Arg
5105                    5110                    5115                    5120
Lys Asp Lys Pro Pro Tyr Val Leu Thr Gln Tyr Ile Asn Asp Val Val
                5125                    5130                    5135
Val Ala Met Pro Glu Ser Leu Phe Ser Thr Gly Arg Phe Tyr Arg Glu
            5140                    5145                    5150
Lys Pro Tyr Pro Val Leu Met Asn Glu Asp Leu Ser Gly Leu Asn His
        5155                    5160                    5165
His Ile Phe Ser Gly Asp Tyr Thr Asp Glu Ser Leu Thr Leu Gly Gly
    5170                    5175                    5180
Val His His Ile Val Thr Leu Asn Thr Tyr Asp His Lys Leu Asn Tyr
5185                    5190                    5195                    5200
Ile Gln Thr Lys Ala Thr Cys Ala Ala Ser Val Ser Thr Gly Gly Arg
                5205                    5210                    5215
Gly His Lys Ile Thr Thr Leu Phe Asp Val His Ala Asn Gln Leu Ala
            5220                    5225                    5230
Asp Glu Ile Thr Arg Val Thr Ser Val Val Thr Thr Gln Ser Lys Val
        5235                    5240                    5245
Ile Asn Leu Thr Ile Asp Tyr Gln Gln Val Pro Cys Met Tyr Trp Ser
    5250                    5255                    5260
Ser Pro Thr Gly Ile Arg Thr Phe Tyr Pro Gln Ala Val Arg Leu Asp
5265                    5270                    5275                    5280
Ala Lys Phe Ile Pro Tyr Tyr Val Glu Tyr Pro Asn Ile Leu Pro Ala
                5285                    5290                    5295
Val Val Glu Asp Gln Val Tyr Asp Leu Ser Asn Tyr Asn Gln Pro Pro
            5300                    5305                    5310
Leu Gly Gln Asn Cys Pro Val Asn Phe His Lys Tyr Val Gln Leu Thr
        5315                    5320                    5325
His Phe Ile Leu Asp His Val Lys Ile Pro Glu Lys Gly Leu Ile Tyr
    5330                    5335                    5340
His Ile Gly Ala Ala Gly Thr Lys Gln Cys Ser Pro Gly Asp Leu Ile
5345                    5350                    5355                    5360
Leu Glu Gln Phe Phe Asn Lys Ser Ile Ile Tyr Ser Ser Asp Leu Leu
                5365                    5370                    5375
Pro Tyr Gln Ser Pro Ala Val Gln Val Ala Leu Asp Val Arg Phe Ser
            5380                    5385                    5390
Ala Ser Leu Ile Ile Ser Asp Cys Tyr Ser Lys Glu Pro Gln Pro Asp
        5395                    5400                    5405
Leu Leu Ser Lys Leu Ile Asn Lys Leu Val Tyr Gly Gly Thr Leu Ile
    5410                    5415                    5420
Phe Lys Thr Thr Glu Thr Phe Thr Cys Asp Pro Ala Phe Tyr Val Ala
5425                    5430                    5435                    5440
His Phe Asn Cys Ile Lys Phe Phe Thr Ala Ala Val Asn His Ser Ser
                5445                    5450                    5455
Ser Glu Val Tyr Ile Ala Phe Ile Gly Lys Leu Pro Lys Pro Asn Asn
            5460                    5465                    5470
Asn Phe Leu Ala Ser Asp Tyr Phe Gln Arg Leu Thr Gln His Arg Asn
        5475                    5480                    5485
Lys Val Val Lys Gln Pro Tyr Ala His Thr Trp Asp Thr Ser Phe Thr
    5490                    5495                    5500
Tyr Pro Tyr Pro Ser Asn Val Leu Gln Val Ser Arg Lys Asn Leu Leu
5505                    5510                    5515                    5520
Tyr Leu Phe Glu Thr Arg Gly Ala Ala Val Gly Thr Leu Ile Phe Glu
                5525                    5530                    5535
Glu Pro Ser Lys Pro Ala Val Lys Ile Pro Thr Lys Cys Gln Thr Thr
            5540                    5545                    5550
Gln Pro Ser Cys Val Ile Glu Val Gly Asn Gln Tyr Asp Cys Cys Ile
        5555                    5560                    5565
Gln Asp Ile Ile Thr Leu Leu Asn Gly Lys Ser Phe Thr Val Lys Val
    5570                    5575                    5580
Pro Asn Ser Glu Ser Leu Leu Arg Asp Ile Cys Thr Leu Ala Leu Ser
5585                    5590                    5595                    5600

Gln Ser Tyr Ser Ile Asn Ile Arg Gly Lys Thr Leu Tyr Thr Leu Ser
                5605                5610                5615
Ser Leu Leu Arg Ile Arg Gln Gln Ser Leu Leu Phe Tyr Gly Glu Lys
            5620                5625                5630
Val Lys Asn Pro Arg Pro Arg Asn Val Leu Asn Lys Tyr Thr Asn Tyr
        5635                5640                5645
Leu Lys Ala Lys Val Ile Arg His Tyr Thr Lys Pro Gln Ser Thr Val
    5650                5655                5660
Leu Asp Ile Gly Thr Gly Lys Gly Gln Asp Leu Arg Lys Tyr Ser Leu
5665                5670                5675                5680
Ala Gly Val Lys Ser Leu Thr Cys Val Glu Pro Ser Pro Glu Ser Val
                5685                5690                5695
Thr Glu Leu Ser Ile Ile Ala Ser Pro Leu Asp Met Glu Thr His Thr
            5700                5705                5710
Val Met Ser Ser Ala Gln Lys Phe Glu Thr Ser Leu Thr Phe Asp Leu
        5715                5720                5725
Ala Phe Ser Phe Phe Ala Leu His Tyr Ala Leu Asp Asp Val Cys Met
    5730                5735                5740
Ser Glu Thr Leu Asn Asn Val Phe Cys Lys Leu Asn Ser Asn Ser Gln
5745                5750                5755                5760
Leu Ile Leu Val Val Pro Asn Ala Gly Arg Met Gln Ser Ile Pro Ser
                5765                5770                5775
Leu Gly Leu Thr Val Thr His Leu Asp Asp Asp Lys Val Trp Phe Lys
            5780                5785                5790
Tyr Ser Asp Tyr Ile Asp Cys Glu Glu Pro Leu Val Asp Lys Glu Lys
        5795                5800                5805
Leu Leu Thr Cys Leu Ala Thr Tyr Gly Thr Ile Val Thr Asp Ser Pro
    5810                5815                5820
Phe Tyr Asp Gly Ala Asn Lys Ile Leu Asp Gln Lys Cys Ser Ser Met
5825                5830                5835                5840
Tyr Arg Ala Ser Thr Ala His Leu Asn Pro Asp Glu Ile Gln Tyr Ile
                5845                5850                5855
Asn Met Tyr Asp Leu Ile Val Val Ile Lys Asn
            5860                5865

<210> 8
<211> 1391
<212> PRT
<213> Viruses

<220>
<223> ORF-2 amino acid sequence

<400> 8
Met Phe Ala Leu Val Leu Thr Leu Thr Ile Ala Ser Ala Ile Ala Gln
1               5                   10                  15
Asp Phe Pro Ala Tyr Asp Pro Cys Pro Thr Cys Ser Thr Pro Gly Asn
            20                  25                  30
Lys Ile Pro Ala Pro Ser Thr Val Ala Gln Tyr Ser Thr Asn Tyr Gly
        35                  40                  45
Ala Asn Phe Phe Thr Val Val Phe Asp Gly Ile Ile Phe Asn Gln Phe
    50                  55                  60
Arg Glu Ser Tyr Tyr His Gln Cys Arg Pro Thr Pro Glu Tyr Cys Pro
65                  70                  75                  80
Asp Ala Ile Asn Cys Ala Leu Asn Arg Thr Gly Ala Ser Cys Lys Pro
                85                  90                  95
Phe Ala Thr Gly Pro Asn Ser Gln Cys Gln Asn Ser Phe Glu Gly Asn
            100                 105                 110
Ile Asp Ile Cys Ala Thr Cys Ser Pro Leu Lys Gln Glu Thr Pro Phe
        115                 120                 125
Ile Cys Tyr Asn Arg Tyr Gly Ile Ile Ile Tyr Pro Thr Ala Asp Ile
    130                 135                 140
Val Leu Ser Ala Arg Phe Lys Ile Gly Ser Phe Ser Pro Lys Ala Cys

145                 150                 155                 160
Asp Asn Tyr Leu Asn Asp Leu Asn Cys Asp Ser Lys Thr Ala Arg Ser
                165                 170                 175
Tyr Val Ile Ser Arg Pro Gln Ser Phe Ser Leu Gln Tyr Pro Asn Ser
            180                 185                 190
Leu Gly Pro Tyr Gln Leu Lys Arg Phe Ser Leu Ala Lys Glu Ile Val
        195                 200                 205
Asp Leu Arg Ala Gly Val Leu Thr Ser Leu Pro Asn Arg Gly Tyr Lys
    210                 215                 220
Gly Arg Thr Thr Tyr Ser Tyr Pro Val Thr Ala Leu Ser Leu Leu Ala
225                 230                 235                 240
Arg Ser Lys Val Ala Glu Ala Asp Lys Phe Phe Tyr Ile Glu Ala Lys
                245                 250                 255
Ile Leu Leu Tyr Ala Trp Ser Gln Lys Pro Gln Ile Arg Phe Leu Gly
            260                 265                 270
Ala Tyr Cys Pro Thr Asp Val Ser Cys Pro Asp Ser Thr Ala Leu Gly
        275                 280                 285
Cys Cys Phe Ser Gly Ser Gly Ser Glu Phe Tyr Tyr Ala Phe Arg Gln
    290                 295                 300
Trp Tyr Tyr Ala Ser Leu Gly Met Glu Asp Leu Val Asp Phe Asp Asn
305                 310                 315                 320
Ser Thr Val Leu Ser Leu Ser Pro Asp Thr Pro Gln Ile Thr Pro Val
                325                 330                 335
Val Ser Tyr Phe Leu Glu Lys Val Leu Pro Leu Phe Lys Ser His Val
            340                 345                 350
Pro Gly Arg Val Phe Tyr Cys His Ser Leu Met Ser Asn Gly Val Cys
        355                 360                 365
Thr Phe Asp His Val Val Val Asn Ile Asn Ala Glu Ala Val Phe Phe
    370                 375                 380
Asp Leu Glu Val Asp Ile Gly Ser Ile Ile Ala Asp Ala Tyr Arg Val
385                 390                 395                 400
Glu Arg Pro Asn Thr Leu Cys Tyr Asp Thr Asn Cys Thr Leu Ala Thr
                405                 410                 415
Ser Arg Thr Thr Glu Tyr Asn Tyr Ala Ala Tyr Val Val Tyr Ile Leu
            420                 425                 430
Phe Asn Leu Tyr Ser Ser Asn Arg Ile Ala Ile Asp Phe Asn Thr His
        435                 440                 445
Ser Ile Leu Gln Gly Leu Leu Gln His Asn Ser Asn Tyr Gln Thr Ala
    450                 455                 460
Asn Leu Asp Tyr Leu Phe Val Gly Ala Leu Phe Thr Gly Thr Phe Lys
465                 470                 475                 480
His Ile Thr Ser Asn Gln Ala Tyr Pro Val Pro Leu Thr Tyr Pro Ile
                485                 490                 495
Val Lys Thr Tyr Val Gly Pro Ser Asn Gln Tyr Ser Met Ser Asn Lys
            500                 505                 510
Leu Phe Ser Tyr Thr His Asn Leu Thr Ala Gln Ala His Ser Gly Ile
        515                 520                 525
Cys Asn Ser Phe Tyr Cys Tyr Lys Pro Arg Phe Val Pro Ile Asp Val
    530                 535                 540
Phe Ile His Ser Ala Leu Thr Pro Asp Ser Leu Met Glu Thr Glu Ser
545                 550                 555                 560
Phe Val Cys Val Ser Leu Arg Ser Pro Ser Ala Gly Ser Thr Ser Ala
                565                 570                 575
Gly Ser Phe Tyr Leu Gln Cys Leu Asn Ser Ser Ile Asp Leu His Pro
            580                 585                 590
Gly Ser Phe Val Pro Val Ser Ser Ser Pro Glu Ser Ser Ser Arg Val
        595                 600                 605
Thr Ala Glu Leu Ala Phe Asn Thr Arg Asn Gly Ile Phe Ser Pro Cys
    610                 615                 620
Leu Asn Gly Thr Cys Val Leu Ala Pro Thr Asp Pro Ile Val Phe Met
625                 630                 635                 640
Arg Gln Gly Ala Trp Phe Thr Lys Ser Leu His Phe Asp Val Ser Pro
                645                 650                 655

Cys Lys Pro Met His Phe Pro Asp Ile Asp Ile Gln Pro Pro Thr Tyr
            660                 665                 670
Asn Val Ser Ser Ile Lys Met Asp Asp Asn Ala Val Leu Val Gln Asp
        675                 680                 685
Leu Thr Ser Gly Leu Val Ile Asp His Asn Leu Gly Ser Ile Leu Arg
    690                 695                 700
Pro Lys Gly Arg Ala Leu Glu Val Ser Tyr Tyr Ala His Ser Ile Leu
705                 710                 715                 720
Arg Tyr Leu Glu Pro Asp Ser Cys Leu Pro Asp Asn Phe Leu Asn Phe
                725                 730                 735
Val Thr Cys Leu Asp Tyr Ile Cys Ser Asp Ser Ser Pro Cys Arg Ala
            740                 745                 750
Ala Ala Ser Gln Tyr Cys Gln Ala Gly Ile Tyr Phe Glu Ser Ala Phe
        755                 760                 765
Asn Lys Ser Arg Tyr Ser Leu Leu Asn Ala Tyr Thr Leu Phe Asn Thr
    770                 775                 780
Ser Leu Gln Thr Leu Leu Pro Glu Thr Phe Leu Glu Ile Glu Asp Asp
785                 790                 795                 800
Glu Pro His Ser Arg Ser Lys Arg Ser Ile Asp Thr Thr Ser Asn Ile
                805                 810                 815
Arg Pro Ser Gln Leu Leu Val Asn Gly Arg Ile Pro Ser Thr Ser Ser
            820                 825                 830
Ala Phe Ala Val Asn Val Ala Arg Gly Arg Gly Thr Ile Met Pro Arg
        835                 840                 845
Pro Gly Thr Gly Gly Met Gly Ser Ser Phe Ser Ala Val Ser Arg Ser
    850                 855                 860
Gly Ser Ile Ser Ser Leu Ser Ser Val Gly Ser Ser Thr Pro Leu Ile
865                 870                 875                 880
Ser Asn Trp Arg Thr Ser Ser Ser Gln Leu Lys Thr Leu Asn Leu Asn
                885                 890                 895
Ile Asn Thr Lys Ile Pro Lys Ile Ser Thr Lys Ser Gly Phe Ala Ser
            900                 905                 910
Ile Thr Ser Leu Phe Ala Ser Gly Leu Gly Val Val Asp Leu Gly Leu
        915                 920                 925
Ser Ile Phe Asn Met Ile Glu Gln Arg Arg Val Ala Glu Ile Thr Gln
    930                 935                 940
Met Gln Ile Ser Gln Leu Ala Asp Ser Ile Val Tyr Leu Ala Asp Val
945                 950                 955                 960
Thr Phe Glu Ala Ile Lys Asn Leu Glu Leu Ser Val Asn Ser Leu Gly
                965                 970                 975
Thr Phe Leu Ser Glu Phe Ser Thr Gln Met Ser Ile Thr Ile Ser Gln
            980                 985                 990
Ile Gln Ser Ser Phe Glu Glu Gln Gln Asp Ala Thr Asn Asp Ala Leu
        995                 1000                1005
Tyr Tyr Thr Asn Ala Ala Ala Ser Tyr Gln Ala Ser Met Ala Tyr Val
    1010                1015                1020
Ile Ser Glu Leu Asn Ala Ile Ser Leu Ser Val Thr Arg Ser Tyr Asp
1025                1030                1035                1040
Ser Tyr Thr Ser Cys Ile Thr Ser Gly Ile Asn Gly Leu Ile Thr Pro
                1045                1050                1055
Ser Cys Leu Pro Ala His Gln Leu Leu Gln Leu Leu Asp Thr Val Ile
            1060                1065                1070
Asn Ser Thr Ala Gly Thr Gly Cys Arg Pro Ile Tyr Gly Arg Glu Glu
        1075                1080                1085
Val Val Lys Tyr Tyr Thr Leu Pro Leu Ile Asn Gln Gly Tyr Ser Phe
    1090                1095                1100
Asn Gly Ser Ile Phe Phe Val Phe Asn Ile Pro Ile Thr Cys Gln Gly
1105                1110                1115                1120
Ile Ala Gly Asp Val Tyr Glu Val Glu Pro Pro Ile Leu Val Asp Val
                1125                1130                1135
Pro Ser Lys Thr Ala Leu Arg Met Ile Thr Pro Ser Asn Val Val Ala
            1140                1145                1150
Thr Gln Ala Gly Leu Ala Glu Leu Asp Leu Arg His Cys Glu Arg Tyr

1155                1160                1165
His Asn Glu Phe Leu Cys Asp Ser Ser Ala Phe Leu Ser Thr Pro Ser
    1170                1175                1180
Lys Tyr Ile Asp Cys Leu Thr Asn Ala Thr Asp Cys Ser Leu Gln Phe
1185                1190                1195                1200
Ile Thr Gln His Val Pro Asp Pro Cys Val Tyr Thr Ser Pro Ala Ser
                1205                1210                1215
Leu Tyr Cys Tyr Tyr Ser Pro Ile Cys Asp Gln Cys His Ile Val Ala
            1220                1225                1230
Gly Cys Asn Glu Ser Gln Gln Tyr Asn Phe Thr Ser Ala Asp Gly Gly
        1235                1240                1245
Val Val Phe Tyr Ser Ile Gln Asp Arg Asp Cys Gly His Phe Pro His
    1250                1255                1260
Ile Thr Val Thr Thr Pro Ala Ala Ile Gln Glu Asp Phe Thr Val Gly
1265                1270                1275                1280
Pro Tyr Leu Pro Ser Leu Pro Ile His Thr Ala Tyr Val Asn Val Thr
                1285                1290                1295
Trp Asn Val Thr Leu Pro Gly Asn Trp Thr Trp Glu Asn Ile Thr Leu
            1300                1305                1310
Thr Ala Asn Trp Thr Gln His Phe Ile Glu Met Lys Lys Asn Ile Thr
        1315                1320                1325
Met Met Ala Glu Glu Ile Asp Asn Leu Thr Asn Phe Gly Lys Val Leu
    1330                1335                1340
Val Gly Gln Leu Asn Ser Phe Leu Ser Ser Leu Phe Asn Ile Pro Leu
1345                1350                1355                1360
Gly Leu Met Thr Phe Cys Phe Ser Val Ala Ala Leu Gly Leu Ser Ile
                1365                1370                1375
Ile Ala Leu Leu Val Leu Cys Phe Pro Gln Lys Pro His Lys Leu
            1380                1385                1390

<210> 9
<211> 260
<212> PRT
<213> Viruses

<220>
<223> ORF-3 amino acid sequence

<400> 9
Met Met Phe Thr Leu Val Val Leu Phe Thr Leu Leu Gly Leu Ser Met
1               5                   10                  15
Ala Ser Thr Glu Leu Asn Phe Asp Pro Thr Leu Pro Leu Pro Ser Pro
            20                  25                  30
Ile Asn Ala Leu Val Asp Ile Phe Gly Asn Asn Ser Leu Phe Leu Lys
        35                  40                  45
Glu Ser Leu Leu Gly Lys Ser Thr Gly Ala Val Tyr Ala Tyr Leu Tyr
    50                  55                  60
Ser Ser Ala Ile Ser Leu Leu Leu Leu Leu Trp Val Thr Val Trp Ser
65                  70                  75                  80
Ile Ala Thr Ser His Phe Asn Val Thr Arg Ile Pro Thr Ile Ala Val
                85                  90                  95
Leu Thr Asn Ala Ser Met Phe Leu Leu Leu Ala Ser Ala Thr Val Thr
            100                 105                 110
Thr Trp Phe Leu Pro Thr Val Thr Asn Val Phe Phe Tyr Thr Leu Thr
        115                 120                 125
Ala Leu Phe Thr Phe Phe Ser Phe Val Phe Leu Leu Trp Leu Val Tyr
    130                 135                 140
Tyr Met Phe Thr Thr Ile Arg Ala Tyr Arg Arg Val Gly Ser Trp Arg
145                 150                 155                 160
Val Val Phe Asn Gly Lys Tyr Ser Leu Leu Ala Gly Thr Gln Ala Val
                165                 170                 175
Cys Leu Cys Arg Pro Ala Ile His Leu Val Leu Thr Lys Thr Asn Thr
            180                 185                 190

Asp Thr Tyr Trp Cys Leu Asp Gly Thr Pro Ile Tyr Asn Val Asp Leu
        195                 200                 205
Leu Gln Leu Val Gly Pro Lys Gly Leu Tyr Pro Tyr Lys Arg Met Thr
    210                 215                 220
Thr Ile Thr Ala Pro Lys Gly Thr Lys Thr Ser Ala Ala Val Tyr Thr
225                 230                 235                 240
Leu Gln Lys Glu Glu Val Cys Ala Leu Ser Glu Ile Thr Val His Asn
                245                 250                 255
Asp Thr Asp Phe
            260

<210> 10
<211> 201
<212> PRT
<213> Viruses

<220>
<223> ORF-4 amino acid sequence

<400> 10
Met Ser Tyr Pro Val Tyr Tyr Glu Gln Arg Arg Tyr Ser Pro Arg Gln
1               5                   10                  15
Phe Asn Asn Gly Gly Gly Tyr Asn Pro Thr Pro Gln Pro Arg Val Val
            20                  25                  30
Arg Thr Asn Pro Gly Asn Gln Ala Tyr Asn Pro Arg Arg Asn Arg Asn
        35                  40                  45
Ala Thr Pro Asn Gln Gln Gln Met Val Pro Tyr Gln Pro Gln Tyr Gln
    50                  55                  60
Ala Pro Pro Gln Pro Arg Val Val Tyr Val Asp Arg Pro Gln Glu Pro
65                  70                  75                  80
Val Val Ile Tyr Arg Ala Pro Pro Gln Gly Lys Lys Gln Ser Gly Lys
                85                  90                  95
Arg His Thr Ala Glu Glu Arg Trp Tyr Gln Gly Glu Lys Pro Val Gln
            100                 105                 110
Lys Lys Gln Ala Pro Lys Gly Lys Ser Lys Lys Ala Ala Thr Pro Ala
        115                 120                 125
Asn Pro Lys Lys Gln Pro Thr Gln Ser Asp Lys Val Pro Ile Ala Tyr
    130                 135                 140
Pro Asp Asn His Pro Phe His Asp Leu Ala Pro Ala Asp Ile Arg Ala
145                 150                 155                 160
Phe Lys Lys Gln Leu Ile Gln Asn Leu Asp Leu Gly His Gly Glu Met
                165                 170                 175
Asn Gln Leu Arg Leu Ser Ile Asp Leu Leu Pro Ile Lys Lys Pro Ala
            180                 185                 190
Pro Thr Pro Ala Val Pro Ala Pro Leu
        195                 200

<210> 11
<211> 113
<212> PRT
<213> Viruses

<220>
<223> ORF-5 amino acid sequence

<400> 11
Met Phe Thr Leu Val Leu Ile Ile Leu Leu Ser Phe Ser Met Ala Phe
1               5                   10                  15
Asn Ala Phe Thr Phe Leu Leu Leu Leu Phe Phe Thr Phe Lys Cys Ile
            20                  25                  30
Ile Thr Arg Thr Leu Val Val Val Pro Ile Asp Tyr Pro Glu Asn His
        35                  40                  45
Pro Phe Asn Gly Leu Ser Pro Glu Glu Ile Ile Ser Tyr Lys Ser Gln

        50                  55                  60
Leu Ile Gln Asn Leu Asp Leu Gly His Gly Glu Val Ile Lys His Arg
65                  70                  75                  80
Phe Ser Ile Asp Leu Leu Pro Leu Lys Thr Thr Ser Thr Pro Thr Thr
                85                  90                  95
Ser Ala Ile Leu Trp Lys Arg Phe Lys Thr Ser His Lys Glu Asn Asn
            100                 105                 110
His


<210> 12
<211> 18
<212> DNA
<213> Artificial Sequence

<220>
<223> TCL-ORF-1 Forward primer

<400> 12
acactatacc agcccatg                                                    18


<210> 13
<211> 22
<212> DNA
<213> Artificial Sequence

<220>
<223> TCL-ORF-1 Taqman Probe

<400> 13
caagcaacag tttaagtgag cc                                               22


<210> 14
<211> 18
<212> DNA
<213> Artificial Sequence

<220>
<223> TCL-ORF-1 Reverse primer

<400> 14
tgtgaggtag caaagacg                                                    18


<210> 15
<211> 19
<212> DNA
<213> Artificial Sequence

<220>
<223> TCL-M Forward primer

<400> 15
gtgttcttac tgtggttgg                                                   19


<210> 16
<211> 22
<212> DNA
<213> Artificial Sequence

<220>
<223> TCL-M Taqman Probe

<400> 16
cattagggca tatcgaaggg tc                                                22


<210> 17
<211> 19
<212> DNA
<213> Artificial Sequence

<220>
<223> TCL-M Reverse primer

<400> 17
tgagttccag caagtagag                                                    19


<210> 18
<211> 20
<212> DNA
<213> Artificial Sequence

<220>
<223> TCL-S Forward primer

<400> 18
tcaaaactct caacctcaac                                                   20


<210> 19
<211> 24
<212> DNA
<213> Artificial Sequence

<220>
<223> TCL-S Taqman Probe

<400> 19
aagatgtaat actggcaaaa cctg                                              24


<210> 20
<211> 19
<212> DNA
<213> Artificial Sequence

<220>
<223> TCL-S Reverse primer

<400> 20
acgactccta aacctgaag                                                    19